(19) Europäisches Patentamt
European Patent Office
Office européen des brevets

(11) EP 4 058 443 B1

(12) **EUROPEAN PATENT SPECIFICATION**

(45) Date of publication and mention of the grant of the patent:
**02.04.2025 Bulletin 2025/14**

(21) Application number: **20821378.5**

(22) Date of filing: **12.11.2020**

(51) International Patent Classification (IPC):
***C07D 401/06*** *(2006.01)* ***A61K 6/00*** *(2020.01)*
***C07C 211/63*** *(2006.01)* ***C07C 211/64*** *(2006.01)*
***A61K 31/14*** *(2006.01)* ***A61K 31/4425*** *(2006.01)*
***A61P 31/04*** *(2006.01)*

(52) Cooperative Patent Classification (CPC):
(C-Sets available)
**C07D 401/06; C07C 219/08; C07C 233/44** (Cont.)

(86) International application number:
**PCT/IB2020/060638**

(87) International publication number:
**WO 2021/094960 (20.05.2021 Gazette 2021/20)**

(54) **PHOTOPOLYMERIZABLE ANTIBACTERIAL MONOMER**

FOTOPOLYMERISIERBARES ANTIBAKTERIELLES MONOMER

MONOMÈRE ANTIBACTÉRIEN PHOTOPOLYMÉRISABLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.11.2019 IT 201900020949**

(43) Date of publication of application:
**21.09.2022 Bulletin 2022/38**

(73) Proprietors:
- **Università degli Studi di Trieste**
  **34127 Trieste (IT)**
- **Alma Mater Studiorum - Università di Bologna**
  **40126 Bologna (IT)**

(72) Inventors:
- **CADENARO, Milena**
  **34139 Trieste (IT)**
- **MARSICH, Eleonora**
  **34148 Trieste (IT)**
- **TURCO, Gianluca**
  **34126 Trieste (IT)**
- **FANFONI, Lidia**
  **32030 Arsiè (IT)**
- **BRESCHI, Lorenzo**
  **40065 Pianoro (IT)**

(74) Representative: **Valenza, Silvia et al**
**Notarbartolo & Gervasi S.p.A.**
**Viale Achille Papa, 30**
**20149 Milano (IT)**

(56) References cited:
**EP-A1- 0 980 682** **JP-A- H08 157 318**
**US-B1- 6 339 113**

Remarks:
The file contains technical information submitted after the application was filed and not included in this specification

EP 4 058 443 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61K 6/30, C08L 33/10**

## Description

### FIELD OF THE INVENTION

The present invention relates to a compound having antibacterial properties suitable for use as an antibacterial monomer, a photopolymerizable composition comprising said compound, and use thereof in a dental method.

### STATE OF THE ART

Adhesive dentistry is a branch of dentistry that has developed considerably in recent years, in which the development and increasingly widespread use of materials with adhesive properties has revolutionized many aspects of dentistry, both in the restorative and preventive fields.

Dental adhesives serve the primary purpose of ensuring the preservation of fillers or composite cements used in restorative and preventive dental treatments. Moreover, said adhesives, to be used, must be able to withstand mechanical stresses, and in particular stresses from rubbing and/or compression.

To date, dental adhesives are largely resinous preparations composed of (photo)polymerizable monomers, which can have both a structural role, and in particular having the purpose of creating a rigid three-dimensional network structure, and a functional role, through which properties of interaction with the biological environment, for example with collagen fibrils or dentin, or specific properties are conferred to the adhesives.

Among the specific properties shown by the (photo)polymerizable monomers in said resinous preparations, the antibacterial properties are of fundamental importance since they are particularly useful in preventing bacterial colonization phenomena in the dental treatment sites.

EP 0 980 682 A1 discloses antibacterial binder compositions for dental use, which comprise (A) an antibacterial primer comprising a polymerizable antibacterial monomer having an unsaturated ethylene group and at least one or more cationic groups selected from the group consisting of ammonium bases, pyridinium bases and phosphonium bases, and a volatile solvent, and (B) an adhesive composition comprising a polymerizable monomer having a group acid, a polymerizable monomer and a polymerization initiator; and an adhesive composition for dental use, which comprises (P) an adhesive primer comprising a polymerizable monomer having an acid group, a hydrophilic polymerizable monomer and water, and (Q) a bonding agent comprising a polymerizable monomer and an acylphosphine oxide compound and an alpha-diketone compound which both serve as a polymerization initiator.

JP H08 157 318 A discloses an antibacterial dental adhesive composition comprising (A) 0.01-25 wt% of an antibacterial polymerizable monomer, (B) 5-40 wt% of a polymerizable monomer bearing an acid group, e.g. 2-(meth)acryloyloxyethyl dihydrogen phosphate, (C) 10-50% by weight of a polymerizable monomer bearing a hydroxyl group, 20-75% by weight of water and a polymerization catalyst.

US 6 339 113 B1 discloses a light-curing composition for dental restoration based on the mixture of a multifunctional prepolymer of 2,2-bis-(4-(2-hydroxy-3-methacryloyloxypropoxy)phenyl)propane ("Bis-GMA") and a multifunctional prepolymer formed by replacing hydrogen atoms in the hydroxyl group with methacrylate groups in said Bis-GMA molecules, and further comprising a diluent, an inorganic filler, a photoinitiation system and other additives.

Although examples of photopolymerizable monomers which have a quaternary ammonium functionality (QAMs) acting as antibacterial agents in resinous preparations for dental use are known in the literature and on the market, the Applicant has found that said monomers have a whole series of technical and performance limitations that affect its use and performance.

In particular, the Applicant has found that the hitherto known photopolymerizable monomers which have quaternary ammonium functionalities are generally poorly soluble in organic solvents, and therefore their use is limited to dental adhesives with hydrophilic characteristics, typically *primer*-type formulations.

Furthermore, the Applicant has found that the present currently known and commercially available photopolymerizable monomers bearing a quaternary ammonium functionalities very often shown a structure characterized by a single methacrylic type group which can limit their use in hydrophobic highly cross-linked dental adhesives, such as *bonding*-type formulations.

The Applicant has also noted that, if on the one hand the achievement of a relevant antibacterial activity is desirable, it can be accompanied by an undesirable cytotoxic effect, which can compromise the possibility of contact between the compounds having antibacterial properties and the biological substrates, such as the oral mucosa. In particular, the Applicant has noted that the ISO 10093-5 standard indicates that a reduction in cell viability greater than 30% is to be considered due to a cytotoxic effect. The Applicant has found that in order to be able to use a compound in a dental adhesive it is therefore necessary to balance these two different and divergent needs.

At last, the Applicant has noted that depending on the structure of the compound, the antibacterial properties deriving from the presence of the quaternary ammonium functionality can vary and be more or less significant and that the structure of the compound can also influence its ability to enter the chain during the (photo)polymerization and,

consequently, influencing the mechanical properties of the obtained resin.

## SUMMARY OF THE INVENTION

Therefore, the aim of the present invention is to develop a new compound having antibacterial properties, capable of being easily and effectively used as an antibacterial monomer in a wide range of adhesive resins for dental use, without causing cytotoxic effect at the concentrations of use and without compromising the mechanical properties of the resins themselves after photopolymerization.

The Applicant has surprisingly observed that it is possible to achieve this and other desirable purposes by suitably identifying some specific structural characteristics of a compound which has quaternary ammonium functionality.

In a first aspect, therefore, the present invention relates to a compound of formula (I):

$A\text{-}R_1\text{-}B$ (I)

wherein:

$R_1$ is selected from the group consisting of:
$-(CH_2)_{12}-$, and

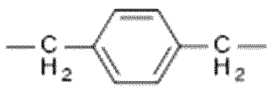

;

and

A and B are independently selected from the group consisting of:

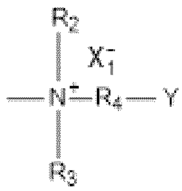

, and

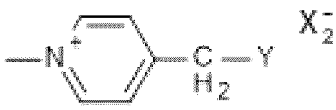

;

wherein:

$R_2$ and $R_3$ are independently selected from the group consisting of: methyl, ethyl, and n-propyl;

$R_4$ is selected from the group consisting of methylene, ethylene, n-propylene, and 1,4-phenylene;

Y is selected from the group consisting of:

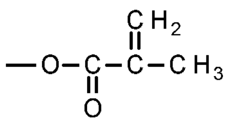

, and

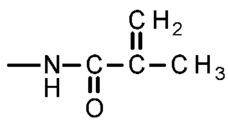

;

and

$X_1^-$ and $X_2^-$ are independently selected from the group consisting of: $F^-$, $Cl^-$, and $Br^-$.

Thanks to its specific structural characteristics, the compound according to the present invention in fact shows high antibacterial properties and the absence of unwanted cytotoxic effects, which allow it to be easily and effectively used in a wide range of dental adhesives, without compromising their mechanical properties.

In an additional aspect, the present invention further relates to a photopolymerizable composition comprising at

least one compound of formula (I) according to the present invention, and at least one photopolymerization activator.

In fact the structural and antibacterial properties of the compound according to the present invention allow the compound according to the invention and the photopolymerizable compositions containing it to be used in dental treatments, for example in methods of restorative dentistry in order to prevent bacterial colonization phenomena, such as for example caries, in the sites of said treatments.

Therefore, in a further aspect, the present invention also relates to a compound of formula (I) according to the present invention or a photopolymerizable composition according to the present invention, for use in a method of dental treatment.

Thanks to the antibacterial properties deriving from the presence of the quaternary ammonium functionality, the compound of formula (I) and the photopolymerizable composition according to the invention prevent bacterial colonization phenomena in the sites of said dental treatments, especially in restorative dental treatment methods.

In a preferred fulfilment, said dental treatment is a restorative method.

Finally, in a further aspect, the present invention also relates to a compound of formula (I) according to the present invention or a photopolymerizable composition according to the present invention for use as a medicament.

The antibacterial properties deriving from the presence of the quaternary ammonium functionality make the photopolymerizable compound of formula (I) and the photopolymerizable composition according to the invention suitable for use in the medical field.

Finally, in a still further aspect, the present invention relates to the use of at least one compound of formula (I) according to the present invention as an antibacterial monomer in polymeric compositions.

## BRIEF DESCRIPTION OF THE FIGURES

Figure 1 shows the 1H-NMR spectrum of the compound according to Example 1;
Figure 2 shows the 13C-NMR spectrum of the compound according to Example 1;
Figure 3 shows the HH-Cosy spectrum of the compound according to Example 1;
Figure 4 shows the gHSQC spectrum of the compound according to Example 1;
Figure 5 shows the FTIR-ATR spectrum of the compound according to Example 1;
Figure 6 shows the 1H-NMR spectrum of the compound according to Example 2;
Figure 7 shows the 13C-NMR spectrum of the compound according to Example 2;
Figure 8 shows the 19F-NMR spectrum of the compound according to Example 2;
Figure 9 shows the HH-Cosy spectrum of the compound according to Example 2;
Figure 10 shows the gHSQC spectrum of the compound according to Example 2;
Figure 11 shows the 1H-NMR spectrum of the compound according to Example 3;
Figure 12 shows the 13C-NMR spectrum of the compound according to Example 3;
Figure 13 shows the HH-Cosy spectrum of the compound according to Example 3;
Figure 14 shows the gHSQC spectrum of the compound according to Example 3;
Figure 15 shows the **FTIR-ATR** spectrum of the compound according to Example 3;
Figure 16 shows the 1H-NMR spectrum of the compound according to Example 4;
Figure 17 shows the 19F-NMR spectrum of the compound according to Example 4;
Figure 18 shows the HH-Cosy spectrum of the compound according to Example 4;
Figure 19 shows the 1H-NMR spectrum of the compound according to Example 5;
Figure 20 shows the 13C-NMR spectrum of the compound according to Example 5;
Figure 21 shows the HH-Cosy spectrum of the compound according to Example 5;
Figure 22 shows the gHSQC spectrum of the compound according to Example 5;
Figure 23 shows the FTIR-ATR spectrum of the compound according to Example 5;
Figure 24 shows the 1H-NMR spectrum of the compound according to Example 6;
Figure 25 shows the 13C-NMR spectrum of the compound according to Example 6;
Figure 26 shows the HH-Cosy spectrum of the compound according to Example 6;
Figure 27 shows the gHSQC spectrum of the compound according to Example 6;
Figure 28 shows the FTIR-ATR spectrum of the compound according to Example 6;
Figure 29 shows the 1H-NMR spectrum of the compound according to Example 7;
Figure 30 shows the 13C-NMR spectrum of the compound according to Example 7;
Figure 31 shows the HH-Cosy spectrum of the compound according to Example 7;
Figure 32 shows the gHSQC spectrum of the compound according to Example 7;
Figure 33 shows the FTIR-ATR spectrum of the compound according to Example 7;
Figure 34 shows the 1H-NMR spectrum of the compound according to Example 8;

Figure 35 shows the 13C-NMR spectrum of the compound according to Example 8;
Figure 36 shows the HH-Cosy spectrum of the compound according to Example 8;
Figure 37 shows the gHSQC spectrum of the compound according to Example 8;
Figure 38 shows the FTIR-ATR spectrum of the compound according to Example 8;
Figure 39 shows the optical density curves recorded during the tests conducted according to Example 12 with the compound according to Example 1;
Figure 40 shows the optical density curves recorded during the tests conducted according to Example 12 with the compound according to Example 5;
Figure 41 shows the optical density curves recorded during the tests conducted according to Example 12 with the compound according to Example 6;
Figure 42 shows the optical density curves recorded during the tests conducted according to Example 12 with the compound according to Example 7;
Figure 43 shows the comparison according to Example 13 between the polymerization kinetics of the RT3 resin as such and with 1% by weight of the compounds according to Examples 5, 6, and 7; and
Figure 44 shows the result of the tensile strength characterization tests carried out according to Example 14.

**DETAILED DESCRIPTION OF THE INVENTION**

In a first aspect, therefore, the present invention relates to a compound of formula (I):

$A\text{-}R_1\text{-}B$ (I)

wherein:

$R_1$ is selected from the group consisting of:
$-(CH_2)_{12}-$, and

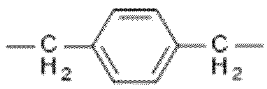
;

and
A and B are independently selected from the group consisting of:

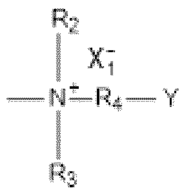
, and
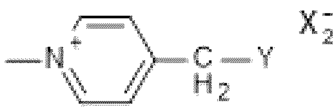
;

wherein:

$R_2$ and $R_3$ are independently selected from the group consisting of: methyl, ethyl, and n-propyl;
$R_4$ is selected from the group consisting of methylene, ethylene, n-propylene, and 1,4-phenylene;
Y is selected from the group consisting of:

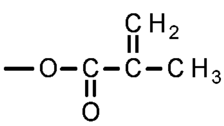
, and
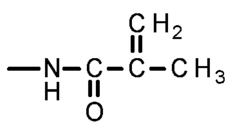
;

and
$X_1^-$ and $X_2^-$ are independently selected from the group consisting of: $F^-$, $Cl^-$, and $Br^-$.

Thanks to its specific structural characteristics, the compound according to the present invention in fact shows

high antibacterial properties and the absence of unwanted cytotoxic effects, which allow it to be easily and effectively used in a wide range of dental adhesives, without compromising their mechanical properties.

Preferably, in the compound of formula (I) according to the present invention, $R_1$ is the group -(CH2)12-.

Preferably, in the compound of formula (I) according to the present invention, $R_4$ is selected from the group consisting of ethylene, n-propylene, and 1,4-phenylene.

Preferably, in the compound of formula (I) according to the present invention, $R_2$ and $R_3$ are independently selected from the group consisting of: methyl, and ethyl.

Preferably, in the compound of formula (I) according to the present invention, A and B are the same.

Examples of compounds of formula (I) according to the present invention are the following:

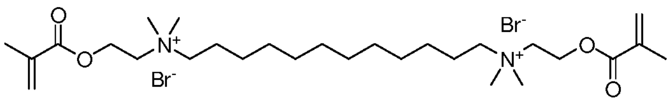

,

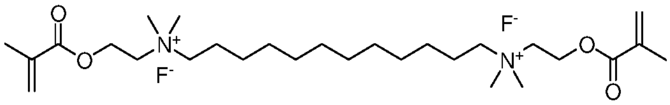

,

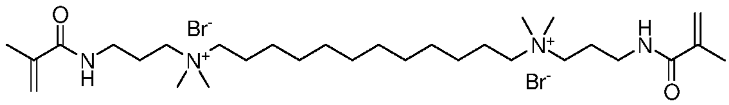

,

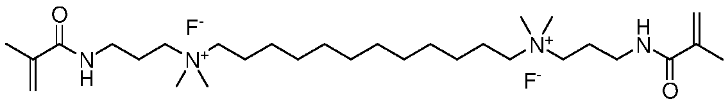

,

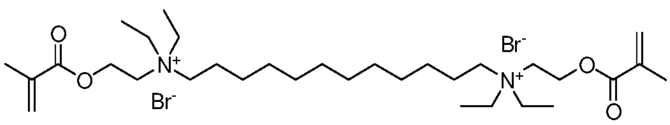

,

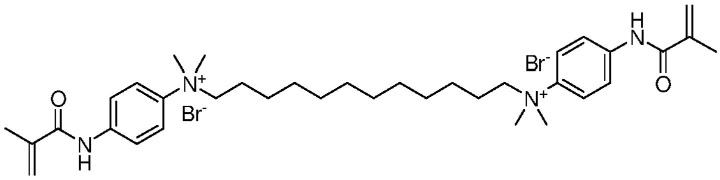

,

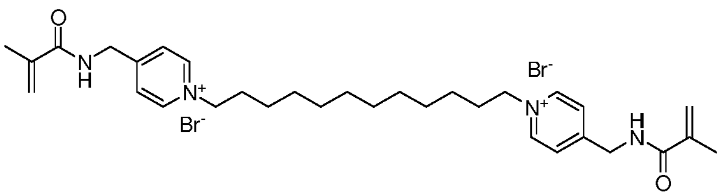

,

and

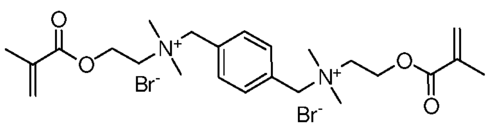

.

In a preferred fulfilment, the compound of formula (I) according to the present invention is selected from:

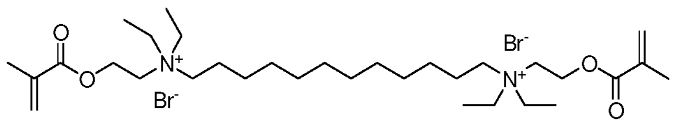

,

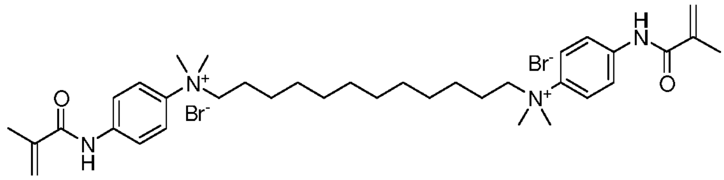

,

and

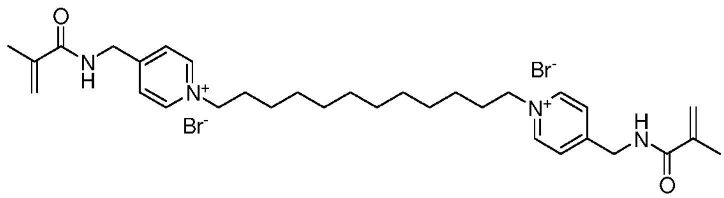

.

Thanks to their specific combination of structural elements, said compounds have in fact been found to be particularly preferable in terms of high antibacterial properties and easy and effective use as antibacterial monomers in a wide range of resinous preparations used as dental adhesives, without compromising their mechanical properties after light curing.

The compound according to the present invention can be prepared according to any of the methods known to the skilled in the art in order to obtain a compound bearing a quaternary ammonium function.

The compound according to the present invention can be synthesized, for example, via the Menschutkin reaction between 1 equivalent of di-halide and 2.5-4 equivalents of tertiary amine in acetonitrile or ethanol (halide concentration 0.1-0.5 M), adopting reaction temperatures in the range 20-120 °C, reaction times from 1-7 days. Under these conditions, a final yield generally higher than 70% is obtained. At the end of the reaction, the product is then isolated by spontaneous precipitation in the reaction medium or by precipitation induced by acetonitrile/diethyl ether, acetonitrile/ethyl acetate, dichloromethane/ethyl acetate, dichloromethane/diethyl ether.

In an additional aspect thereof, the present invention further relates to a photopolymerizable composition comprising at least one compound of formula (I) according to the present invention, and at least one photopolymerization activator.

The structural and antibacterial properties of the compound according to the present invention, in fact, allow the compound according to the invention and the photopolymerizable compositions containing it to be used in dental treatments, for example in methods of restorative dentistry in order to prevent bacterial colonization phenomena, such as for example caries, in the sites of said treatments. In said compositions, the compound of formula (I) according to the present invention acts as a photopolymerizable monomer bearing quaternary ammonium functionality with an antibacterial effect, thus allowing the prevention of bacterial colonization, such as for example caries, in the sites where said composition is applied and subsequently photopolymerized.

Preferably, the photopolymerizable composition comprises from 0.1% to 10% by weight of the at least one compound of formula (I) according to the present invention.

Preferably, in the photopolymerizable composition the at least one photopolymerization activator is selected from any of the photopolymerization activators known for the purpose to the person skilled in the art, more preferably in the group consisting of: camphorquinone (CQ), diphenyl-(2,4,6-trimethylbenzoyl)phosphine oxide (TPO), and phenylpropanedione (PPD), and 2-hydroxy-4-methoxy benzophenone (UV-9).

Preferably, the photopolymerizable composition comprises from 0.05 to 0.5% by weight, more preferably from 0.15 to 0.35% by weight, with respect to the total weight of the photopolymerizable compounds of the composition, of the at least one photopolymerization activator.

Preferably, the photopolymerizable composition according to the present invention comprises at least one photopolymerization co-activator.

Preferably, in the photopolymerizable composition the at least one photopolymerization co-activator is selected from any of the photopolymerization co-activators known for the purpose to the skilled in the art, more preferably in the group consisting of: ethyl-4-dimethylamino benzoate (EDMAB), and 2-(ethylhexyl)-4-(dimethylamino) benzoate (ODMAB), and N,N-di(2-hydroxy ethyl)-4-toluidine (DHEPT).

Preferably, the photopolymerizable composition comprises from 0.5 to 2% by weight, more preferably from 0.75 to 1.25% by weight, with respect to the total weight of the photopolymerizable compounds of the composition, of the at least one photopolymerization co-activator.

Preferably, the photopolymerizable composition comprises, in addition to the at least one compound of formula (I) according to the present invention, at least one further compound comprising at least one ethylenic unsaturated group.

The at least one ethylenic unsaturated group allows said further compound to act as a monomer in the photopolymerizable composition according to the invention.

Preferably, in the photopolymerizable composition the at least one further compound comprising at least one ethylenic unsaturated group is selected from any of the compounds known for the purpose to the skilled in the art, more preferably from the group consisting of: hydroxyethyl methacrylate (HEMA), urethane-dimethacrylate (UDMA), bisphenol A glycidyl dimethacrylate (Bis-GMA), triethylene glycol dimethacrylate (TEGDMA), 10-methacryloyloxydecyl dihydrogen phosphate (10-MDP), Bis[2-(methacryloyloxy)ethyl]phosphate (Bis-MP), methacrylic acid (MA), methyl methacrylate (MMA), 2-hydroxyethyl methacrylate phosphate (HEMA-phosphate), 2-hydroxypropyl methacrylate (HPMA), 2-acrylamido-2-methyl-sulfonic acid (AMPS), ethylene glycol dimethacrylate (EGDMA), glycerol dimethacrylate (GDMA), 1,10-dodecanediol dimethacrylate (DDDMA), glycerophosphoric acid dimethacrylate (GPDM), bis[2-(methacryloyloxy)ethyl] phosphate (Bis-MEP), mono(2-methacryloyloxy)ethyl phthalate (MMEP or PAMA), mono(2-methacryloyloxy-1-hydroxy) ethyl phthalate (PAMM), N-(2-hydroxy-3-((2 methyl-1-oxo-2-propenyl)oxy)propyl)-N-tolyl glycine (NTG-GMA), N-phenylglycine glycidyl methacrylate (NPG-GMA), 4-methacryloyloxyethyl trimellic anhydride (4-META), 4-methacryloyloxyethyl trimellic acid (4-MET), 1,6-hexanediol dimethacrylate (HDDMA), 11-methacryloyloxy-1,10-undecanedicarboxylic acid (MAC-10), polyethylene glycol dimethacrylate (PEGDMA), biphenyl dimethacrylate (BPDM), di-2-methacryloyloxyethyl phosphate (di-HEMA phosphate), dimethylaminoethyl dimethacrylate (DMAEMA), di-2-butane-1,2,3,4-tetracarboxylic acid hydroxyethyl methacrylate (TCB), N-methacryloyl-5-amino salicylic acid (5-NMSA or MASA), pentamethacryloyloxyethyl cyclohexaphosphazene fluoride (PEM-F), di-pentaerythritol penta-acrylate monophosphate (PENTA), 2-(methacryloxyethyl)phenyl hydrogen phosphate (Phenyl-P), 2,5-dimethacryloyloxyethyloxycarbonyl-1,4-benzene dicarboxylic acid (PMDM), 2,5-bis(1,3-dimethacryloyloxyprop-2-yloxycarbonyl)benzen-1,4-dicarboxylic acid (PMGDM), tetra-methacryloyloxyethyl pyrophosphate (Pyro-HEMA), 4-acryloxyethyl trimellic anhydride (4-AETA), 4-acryloxyethyl trimellic acid (4-AET), trimethylpropane trimethacrylate (TMPTMA).

In a preferred embodiment, the photopolymerizable composition according to the present invention comprises from 10% to 50% by weight, with respect to the total weight of photopolymerizable compounds of the composition, of at least one further compound comprising at least one ethylenic unsaturated group, wherein said at least a further compound comprising at least one ethylenic unsaturated group is of the hydrophilic type and is selected from the group consisting of: hydroxyethyl methacrylate (HEMA), methacrylic acid (MA), methyl methacrylate (MMA), 2-hydroxyethyl methacrylate phosphate (HEMA-phosphate), 2-hydroxy propyl methacrylate (HPMA), 2-acrylamido-2-methyl-sulfonic acid (AMPS).

The presence of said amount of the at least one further compound comprising at least one ethylenic unsaturated group of the hydrophilic type makes such a composition particularly useful in supporting and hydrating the collagen fibrils, advantageously allowing the use of the composition for the so-called *primer* formulations.

In a further preferred embodiment, the photopolymerizable composition according to the present invention comprises from 30 to 90% by weight, with respect to the total weight of photopolymerizable compounds of the composition, of at least one further compound comprising at least one ethylenic unsaturated group, wherein said at least a further compound comprising at least one ethylenic unsaturated group is of the hydrophobic-type and is selected from the group consisting of: urethane-dimethacrylate (UDMA), bisphenol A glycidyl dimethacrylate (Bis-GMA), triethylene glycol dimethacrylate (TEGDMA), 10-methacryloyloxydecyl dihydrogen phosphate (10-MDP), Bis[2-(methacryloyloxy)ethyl] phosphate (Bis-MP), ethylene glycol dimethacrylate (EGDMA), glycerol dimethacrylate (GDMA), 1,10-dodecanediol dimethacrylate (DDDMA), glycerophosphoric acid dimethacrylate (GPDM), bis[2-(methacryloyloxy)ethyl] phosphate (Bis-MEP), mono(2-methacryloyloxy)ethyl phthalate (MMEP or PAMA), mono(2-methacryloyloxy-1-hydroxy)ethyl phthalate (PAMM), N-(2-hydroxy-3-((2 methyl-1-oxo-2-propenyl)oxy)propyl)-N-tolyl glycine (NTG-GMA), N-phenylglycine glycidyl methacrylate (NPG-GMA), 4-methacryloyloxyethyl trimellic anhydride (4-META), 4-methacryloyloxyethyl trimellic acid (4-MET), 1,6-hexanediol dimethacrylate (HDDMA), 11-methacryloyloxy-1,10-undecanedicarboxylic acid (MAC-10), polyethylene glycol dimethacrylate (PEGDMA), biphenyl dimethacrylate (BPDM), di-2-methacryloyloxyethyl phosphate (di-HEMA phosphate), dimethylaminoethyl dimethacrylate (DMAEMA), di-2-butane-1,2,3,4-tetracarboxylic acid hydroxyethyl methacrylate (TCB), N-methacryloyl-5-amino salicylic acid (5-NMSA or MASA), pentamethacryloyloxyethyl cyclohexaphosphazene fluoride (PEM-F), di-pentaerythritol penta-acrylate monophosphate (PENTA), 2-(methacryloxyethyl)phenyl hydrogen phosphate (Phenyl-P), 2,5-dimethacryloyloxyethyloxycarbonyl-1,4-benzene dicarboxylic acid (PMDM), 2,5-bis(1,3-dimethacryloyloxyprop-2-yloxycarbonyl)benzen-1,4-dicarboxylic acid (PMGDM), tetra-methacryloyloxyethyl pyrophosphate (Pyro-HEMA), 4-acryloxyethyl trimellic anhydride (4-AETA), 4-acryloxyethyl trimellic acid (4-AET), trimethylpropane trimethacrylate (TMPTMA).

The presence of said amount of at least one further compound comprising at least one ethylenic unsaturated group of the hydrophobic-type makes such a composition particularly useful for the formation of a hybrid layer with dentin, advantageously allowing the use of the composition for the so-called *bonding* formulations.

Preferably, the photopolymerizable composition according to the present invention comprises at least one solvent. Said solvent is advantageously selected from all the solvents commonly used for the production of photopolymerizable compositions for dental use.

Preferably said solvent is selected from the group consisting of: ethanol, acetone, water, and isopropanol.

In addition to the aforementioned components, the photopolymerizable composition according to the present invention advantageously contains one or more additional components known to the skilled in the art, such as for example: inorganic fillers, 4-methoxy phenol (MEHQ, inhibitor), 2,6-di(tert-butyl)-4-methyl phenol (BHT, inhibitor), sodium fluoride (NaF).

In a further aspect, the present invention also relates to a compound of formula (I) according to the present invention or a photopolymerizable composition according to the present invention, for use in a method of dental treatment.

Thanks to the antibacterial properties deriving from the presence of the quaternary ammonium functionality, the compound of formula (I) and the photopolymerizable composition according to the invention prevent bacterial colonization phenomena in the sites of said dental treatments, especially in restorative dental methods.

In a preferred embodiment, said dental treatment is a restorative method.

Finally, as an additional aspect, the present invention also relates to a compound of formula (I) according to the present invention or a photopolymerizable composition according to the present invention for use as a medicament.

The antibacterial properties deriving from the presence of quaternary ammonium functionality make the compound of formula (I) and the photopolymerizable composition according to the invention suitable for use in the medical field.

Finally, in a still further aspect, the present invention relates to the use of at least one compound of formula (I) according to the present invention as an antibacterial monomer in polymeric compositions.

The structure and antibacterial properties deriving from the presence of the quaternary ammonium functionality make the compound of formula (I) particularly suitable for this use, even in fields other than the medical one.

Additional characteristics and advantages of the invention will appear more clearly from the following description of some of its preferred embodiments, given below by way of nonlimiting example with reference to the following exemplary examples.

## EXPERIMENTAL SECTION

### Methods

Determination of the minimum inhibitory concentration (MIC)

A colony of bacterium taken from agar plate is inoculated in Brain Hearth Infusion medium (BHI) and grown overnight at 37 °C. The next day, the bacteria are diluted in fresh medium containing the phenol red indicator, up to a density of $10^6$ bacteria/mL.

A stock solution of 2 mg/mL of compound is prepared in BHI medium containing phenol red.

In a 96-well plate, the compound stock solution is diluted serially (the concentration is halved at each step) to obtain the following final concentrations after addition of the bacteria suspension: 1 mg/mL; 0.5 mg/mL; 0.25 mg/mL; 0.125 mg/mL; 0.065 mg/mL; 0.032 mg/mL; 0.016 mg/mL; 0.008 mg/mL; 0.004 mg/mL; 0.002 mg/mL; 0.001 mg/mL. A compound-free medium is used as a growth control. A volume of bacterial suspension equal to $0.5*10^6$ bacteria/mL is added to each well containing the compound and the control. The dish is incubated at 37 °C for 24 hours. Bacterial growth in each well is detected by the phenol red, which turns from red to yellow following acidification of the medium induced by bacterial metabolism. The lowest concentration of the compound causing no evident color change corresponds to the MIC.

Determination of the minimum bactericidal concentration (MBC)

A 20 mg/mL compound stock solution in BHI medium is prepared. In a 96-well plate, the compound solution is diluted serially (the concentration is halved at each step) to obtain the following final concentrations after addition of the bacteria suspension: 10 mg/mL; 5 mg/mL; 2.5 mg /mL; 1.25 mg/mL; 0.625 mg/mL; 0.31 mg/mL; 0.15 mg/mL; 0.08 mg/mL; 0.04 mg/mL; 0.02 mg/mL. Wells containing a mixture of penicillin and streptomycin antibiotics are used as a positive control. Compound-free medium is used as a growth control. A volume of bacterial suspension equal to $0.5*10^6$ bacteria/mL is added to each well containing the compound and the controls. The dish is incubated at 37 °C for 24

hours. The lowest compound concentration at which no turbidity of the culture medium is evident is defined as MIC. For the calculation of MBC, aliquots equal to 50 μL are taken from the wells showing evident no turbidity of the medium and are plated on BHI agar plates for 48 hours. The MBC is calculated as the concentration of compound that did not produce bacterial colony growth on the dish. Bacterial suspensions taken from the positive control (growth medium only) and the negative control (growth medium with Ampicilin 100 μg/mL) are also seeded.

Determination of the cytotoxicity

*Tested cells:* primary from dental pulp in complete high glucose DMEM medium + 1 mg ascorbic acid, seeded in 96-well dishes;

*Cell viability detection method:* MTS assay from Promega (CellTiter 96® AQueous One Solution Cell Proliferation Assay) colorimetric metabolic assay that allows to evaluate variations in the number of cells being a dye reduced by cellular dehydrogenases. The greater the number of cells, the greater the amount of reduced compound;

*Execution time:* 24 hours and 72 hours from treatment;

*Negative control:* cells seeded in complete DMEM medium (CNT) are used;

*Number of samples:* for 24 hours, 6 samples of each type; for 72 hours 8 samples of each type.

*Procedure:* Cells (6000/well) are seeded on a 96-well dish to form a monolayer at approximately 40% confluence. After 24 hours the medium is replaced by a medium in which the compound has previously been solubilized, sterilized by 0.22 micron filtration. Growth medium alone is used as a negative control (CNT). At the following 24 and 72 hours the colorimetric test is performed. The absorbance values for each sample are averaged and the standard deviation calculated. The percent viability is calculated on the negative control.

Determination of bacterial inhibition - Agar diffusion test

A test adapted from a commonly used method for determining the sensitivity of a bacterial strain to antibiotics in solution, known as Kirby-Bauer antibiotic testing or disc diffusion antibiotic sensitivity testing was used (Brown DF, Kothari D (1975), "Comparison of antibiotic discs from different sources ", J. Clin. Pathol. 28 (10): 779-83. Doi: 10.1136 / jcp.28.10.779). The test performed involves the use of discs imbued with a known concentration of the substance under examination that are placed on a layer of bacteria grown on agar. The diffusion of the bactericidal molecule from the disk into the agar causes the inhibition of bacterial growth in the area surrounding the disk itself where the molecule has spread with the formation of what are called *zones of growth inhibition.* The diameter of these inhibition zones (transparent circular regions in comparison to the bacterial film) is proportional to the antibacterial efficacy of the molecule to its concentration and to its diffusion ability in the medium.

Direct Contact Test (DCT)

The DCT test is based on the turbidimetric determination of bacterial growth in 96-well microplates.

On the wall of each well, 15 μL of the resin to be tested are polymerized for 40 s, using a VALO® Grand LED curing light (Ultradent, Milan, Italy) in standard mode (1000 $mW/cm^2$, 2 maximum of emission in the ranges of wavelengths 395-415 nm and 440-480 nm);

In this way the side walls of the wells are coated with the polymerized material under test. The wells are then washed with phosphate buffered saline (PBS) before inoculating them with bacteria. Keeping the plate in vertical position, 10 μL of *S. Mutans* bacterial suspension (ATCC 25175) in brain heart infusion medium (BHI) from an o/n culture, is then deposited on the layer of material present on the side walls of the wells. The plate is held upright for 1 hour to allow direct contact between bacteria and the cured material. 200 μL culture broth is then added to each of the wells with gentle mixing. The microplate is then placed in the chamber of a spectrophotometer for ELISA plates reading at 37 °C and the optical density in each well is measured at 600 nm at regular intervals (every 30 minutes for 18 hours). The whole experiment is conducted under aseptic conditions and is repeated on three microplates to ensure reproducibility of the result. 24 wells for each microplate were tested for each sample.

Determination of the Degree of Conversion (DC)

The degree of conversion (DC) was measured with a Fourier Transform Infra-Red Attenuated Total Reflectance equipment (FTIR-ATR, Nicolet 6700, Thermo Scientific, Milan, Italy).

The photopolymerization of the experimental adhesives took place according to the following protocol:

- using a micropipette, 10 μL of adhesive were placed in contact with the ATR system of the instrument inside a mold of 0.12 mm thickness and 3.5 mm diameter, in order to mimic as good as possible the clinical situation, standardize the photopolymerized material quantity and allow a good data reproducibility;
- the adhesive was then dried with a gentle air-flow for 10 seconds;
- a strip of Kerr Hawe Striproll (KerrHawe SA, Bioggio, Switzerland; 8 mm/0.05 mm) was applied to the material before and during photopolymerization in order to avoid contact between the material and the air oxygen;
- a transparent 1 mm-thick glass slide was placed above the sample with the aim of standardizing the distance between the curing light tip and the material;
- the tip of the VALO®GRAND curing light was placed onto the glass slide and the light switched on in standard-mode for 20 s (Ultradent; nominal power in standard-mode 1000 $mW/cm^2$; 2 maximum emission peaks in the ranges 395-415 nm and 440-480 nm respectively);
- 5 samples (N = 5) of each resinous mixture were measured;
- the polymerization kinetics (1 spectrum/sec.), up to 20 s of photopolymerization, were recorded for each sample, acquiring the IR spectrum in the range 4000-500 $cm^{-1}$ with a resolution of 16 $cm^{-1}$;
- the DC value was calculated using the following equation:

$$DC\ (\%) = [1 - (R_{t=x}/R_{t=0})]*100$$

where R indicates the ratio between the intensities of the stretching peak of the internal reference, the carbonyl group (C=O) at 1720 $cm^{-1}$ and the vinyl group (C=C), peak at 1636 $cm^{-1}$, respectively calculated before polymerization (t=0) and during the irradiation time (t=x).

Determination of the resistance to tensile strength application (Microtensile Bond Strength - μTBS)

To determine the μTBS value, human teeth not affected by caries or ruptures and without signs of previous restorations were used .

The protocol followed for the preparation of dental samples using the commercial total-etch Adper™ Scotchbond™ Multi-Purpose adhesive composed by the *primer* (Adper™ Scotchbond™ Multi-Purpose Primer) and the *bonding* (Adper™ Scotchbond™ Multi-Purpose Adhesive) (SBMP; 3M™ ESPE, St Paul, MN, USA) with 3-step adhesion technique, is described below:

1) Sectioning of the crown above the pulp chamber roof in order to obtain as much dentinal surface as possible for each sample;

2) 3 passes of the sample surface on abrasive paper to simulate the action of a cutter;

3) Adhesion protocol:

- 15 s etching of the dentinal surface with phosphoric acid in 35% Ultra-Etch® gel preparation (Ultradent Products Inc., Utah, USA);
- 45 s washing with running distilled water;
- 20 s drying with a gentle air-flow;
- 60 s application of the Adper™ Scotchbond™ Multi-Purpose Primer using a microbrush;
- 5 s drying with a gentle air-flow;
- 60 s application of the commercial bonding Adper™ Scotchbond™ Multi-Purpose Adhesive as such or added with 1% by weigh of the antibacterial compound (I) according to the invention, using a microbrush;

- 10s drying with a gentle air-flow;
- 40s photopolymerization with Valo®Grand curing-light;
- 3 applications of 1.5/2 mm-thickness composite were light-cured for 20 s each and then for 60 s as final polymerization;
- storage of the sample for 24 h in distilled water at 4 °C.

4) Sectioning of the samples with a microtome obtaining specimens (herein and after defined: stick) with a square base of side 1x1 mm (± 0.01 mm) and thickness between 5 and 10 mm;

5) Elimination of peripheral enamel sticks considered invalid for the test;

6) Positioning of the individual sticks in the appropriate slots for the microtensile tests with cyanoacrylate glue using the specifications reported in the guidelines of the Academy of Dental Materials for this test (S. Armstrong, L. Breschi, M. Ozcan, F. Pfefferkorn , M. Ferrari, B. Van Meerbeek, Academy of Dental Materials guidance on in vitro testing of dental composite bonding effectiveness to dentin/enamel using micro-tensile bond strength (μTBS) approach, Dent. Mater. 33 (2) (2017) 133-143.). In particular, the "active gripping" configuration was used with the aid of the Zapit glue (Dental Ventures of America, Corona, CA, USA;) and the "Microtensile tester" tool produced by Bisco Inc., Schaumburg, IL, USA;

7) Recording and analysis of the data obtained;

The number of sticks obtained for each sample ranges from 71-83.

<u>1H-NMR, 13C-NMR, 19F-NMR, HH-Cosy, gHSQC analysis</u>

The solutions for NMR analysis were prepared by dissolving a portion of material (5-20 mg) in 0.60 mL of deuterated water ($D_2O$, Sigma-Aldrich, 99.9% D) or 0.75 mL of deuterated dimethyl sulfoxide (DMSO-d6, Sigma-Aldrich 99.96% D). 1H-NMR (16 scans), 13C-NMR (5200 scans), HH-Cosy (200 scans), gHSQC (128 scans) of the monomers were obtained at room temperature using a Varian 400 MHz NMR (Nuclear Magnetic Resonance) spectrometer.

<u>FTIR-ATR analysis</u>

The analysis was carried out with a Fourier transform infrared spectrometer (Nicolet 6700, deterctor: DTGS KBr, Thermo scientific), equipped with a diamond ATR (Attenuated total reflectance). The background was recorded against air prior to the characterization of each monomer. A portion of solid monomer (10-15 mg), non-derivatized and untreated, was placed on the ATR and pressed by a piston in order to ensure optimal contact. The transmittance (T%) spectrum was recorded in the 4000-500 $cm^{-1}$ range, with 24 scans and 4 $cm^{-1}$ resolution.

<u>**Example 1**</u>

7.30 grams of 1,12-dibromo dodecane and 9.4 mL of 2-(dimethylamino)ethyl methacrylate in 110 mL of acetonitrile were added to a Schlenk-type reactor equipped with a magnetic stir bar. The reaction mixture was heated to 65 °C for 3 days obtaining via precipitation with ethyl ether and filtration 13.85 grams (97% yield) of the compound represented below in Formula (1)

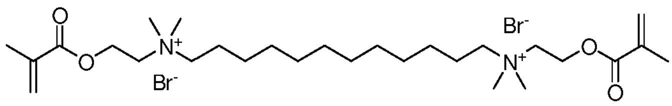

(1)

The compound prepared (herein and after also referred to as "DDM") was characterized by 1H-NMR, 13C-NMR, HH-Cosy, gHSQC obtained with NMR spectrometer (Nuclear Magnetic Resonance) Varian 400 MHz and by FTIR-ATR analysis (Nicolet 6700, Thermo scientific), spectra are shown in Figures 1, 2, 3, 4, and 5 respectively.

**Example 2**

0.5 grams of the compound represented in Formula (1) obtained through the procedure in Example 1, are placed in 19 mL of water in a light-shielded Schlenk-type reactor equipped with a magnetic stir bar. 0.22 grams of silver fluoride (AgF) in 4.3 mL of water is added dropwise to the reactor. The reaction is left at room temperature for 24 h. The raw product is filtered and lyophilized obtaining quantitatively the compound represented in Formula (2)

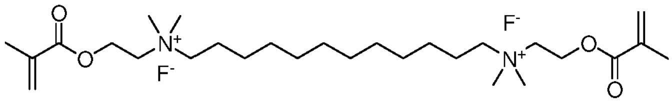

(2)

The compound prepared (herein and after also referred to as "DDM-F") was characterized by 1H-NMR, 13C-NMR, 19F-NMR, HH-Cosy, gHSQC obtained with NMR (Nuclear Magnetic Resonance) Varian 400 MHz spectrometer, spectra are shown in Figures 6, 7, 8, 9, and 10 respectively.

**Example 3**

3.23 grams of 1,12-dibromo dodecane and 4.45 mL of 3-(dimethylamino)propyl methacrylamide in 50 mL of acetonitrile were added to a Schlenk-type reactor equipped with a magnetic stir bar. The reaction mixture was heated to 65 °C for 6 days obtaining via precipitation with ethyl ether and filtration 6.28 grams (95% yield) of the compound represented below in Formula (3)

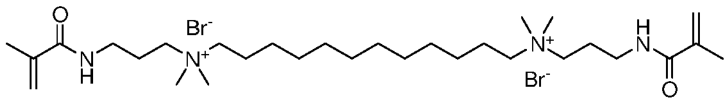

(3)

The prepared compound (herein and after also referred to as "DDMP") was characterized by 1H-NMR, 13C-NMR, HH-Cosy, gHSQC obtained with NMR spectrometer (Nuclear Magnetic Resonance) Varian 400 MHz and by FTIR-ATR analysis (Nicolet 6700, Thermo scientific), spectra are shown in Figures 11, 12, 13, 14 and 15 respectively.

**Example 4**

0.5 grams of the compound represented in Formula (3) obtained through the procedure in Example 3, are placed in 19 mL of water in a Schlenk type reactor shielded from light and equipped with a magnetic stir bar. 0.21 grams of silver fluoride (AgF) in 4.1 mL of water is added dropwise to the reactor. The reaction is left at room temperature for 24 h. The raw product is filtered and lyophilized obtaining quantitatively the compound represented in Formula (4)

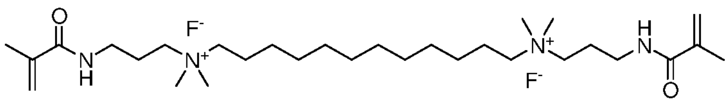

(4)

The prepared compound (herein and after also referred to as "DDMP-F") was characterized by 1H-NMR, 19F-NMR, HH-Cozy, obtained with a NMR (Nuclear Magnetic Resonance) Varian 400 MHz spectrometer, spectra are shown in Figure 16, 17, and 18 respectively.

**Example 5**

0.5 grams of 1,12-dibromo dodecane and 1.22 mL of 2-(diethylamino)ethyl methacrylate in 15 mL of acetonitrile were added to a Schlenk-type reactor equipped with a magnetic stir bar. The reaction mixture was heated to 70 ° C for 5 days obtaining via precipitation from dichloromethane/ethyl ether and filtration 0.776 grams (74% yield) of the compound represented in Formula (5)

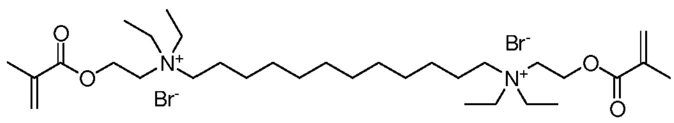

(5)

The compound prepared (herein and after also referred to as "DDE") was characterized by 1H-NMR, 13C-NMR, HH-Cozy, gHSQC obtained with a NMR (Nuclear Magnetic Resonance) Varian 400 MHz spectrometer and by FTIR-ATR analysis (Nicolet 6700, Thermo scientific), spectra are shown in Figures 19, 20, 21, 22, and 23 respectively.

**Example 6**

0.43 grams of 1,12-dibromo dodecane and 0.8 grams of 4-amino-N, N-dimethylaniline in 6.5 mL of acetonitrile were added to a Schlenk-type reactor equipped with a magnetic stir bar. The reaction mixture was heated to 60 ° C for 6 days obtaining by precipitation with ethyl ether and filtration 0.83 grams (87% yield) of the compound represented below in Formula (6)

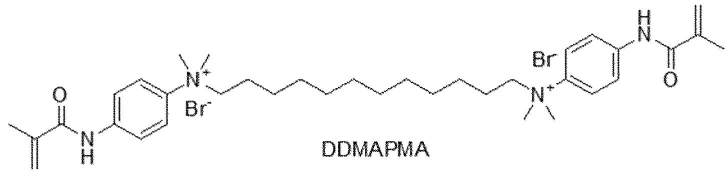

DDMAPMA

(6)

The compound prepared (herein and after also referred to as "DDMAPMA") was characterized by 1H-NMR, 13C-NMR, HH-Cosy, gHSQC obtained with NMR (Nuclear Magnetic Resonance) Varian 400 MHz and FTIR-ATR analysis (Nicolet 6700, Thermo scientific), spectra are shown in Figures 24, 25, 26, 27 and 28 respectively.

**Example 7**

0.75 grams of 1,12-dibromo dodecane and 1.0 grams of N-(4-pyridylmethyl) methacrylamide in 11 mL of ethanol were added to a Schlenk-type reactor equipped with a magnetic stir bar. The reaction mixture was heated to 120 ° C for 2 days obtaining via precipitation with ethyl ether and filtration 1.33 grams (87% yield) of the compound represented below in Formula (7)

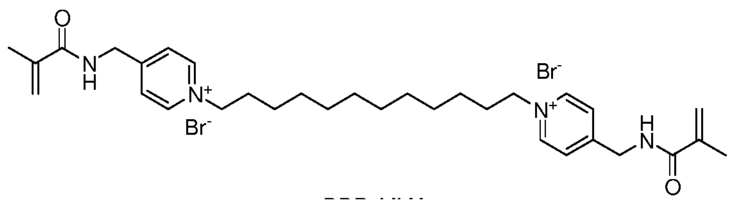

(7)

The compound prepared (herein and after also referred to as "DDPyMMA") was characterized in terms of by 1H-NMR, 13C-NMR, HH-Cozy, gHSQC obtained with NMR (Nuclear Magnetic Resonance) Varian 400 MHz spectrometer and by FTIR analysis. ATR (Nicolet 6700, Thermo scientific), spectra are shown in Figures 29, 30, 31, 32, and 33 respectively.

**Example 8**

3.82 grams of 1,4-dibromo xylene and 5.3 mL of 2-(dimethylamino)ethyl methacrylate in 63 mL of acetonitrile were added to a Schlenk-type reactor equipped with a magnetic stir bar. The reaction mixture was left at room temperature (20 °C) for 24 hours obtaining via precipitation with ethyl ether and filtration 8.34 grams (91% yield) of the compound represented below in Formula (8)

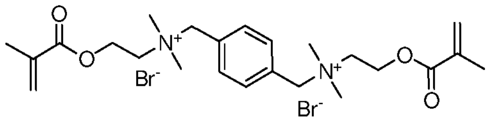

(8)

The compound prepared (herein and after also referred to as "XyDM") was characterized in terms of by 1H-NMR, 13C-NMR, HH-Cozy, gHSQC obtained with NMR spectrometer (Nuclear Magnetic Resonance) Varian 400 MHz and by FTIR analysis- ATR (Nicolet 6700, Thermo scientific), spectra are shown in Figures 34, 35, 36, 37, and 38 respectively.

## Example 9

In order to test the antibacterial properties of the compounds prepared in Examples 1-8, on colonies of *S. Mutans* (ATCC 25175), a bacterium commonly found in the human oral cavity, a test was set up to determine the minimum inhibitory concentration (MIC) and the minimum bactericidal concentration (MBC) according to the "Determination of minimum inhibitory concentration (MIC)" methods and "Determination of minimum bactericidal concentration (MBC)" described above.

The results are reported in Table 1.

TABLE 1 - MIC and MBC values against *S. Mutans*

| Compound | MIC (mg/ml) | MBC (mg/ml) |
|---|---|---|
| Example 1 (DDM) | 0.15 | 0.3 |
| Example 2 (DDM-F) | 0.156 | 0.313 |
| Example 3 (DDMP) | 0.15 | 0.31 |
| Example 4 (DDMP-F) | 0.156 | 0.156 |
| Example 5 (DDE) | 0.01-0.02 | 0.02 |
| Example 6 (DDMAPMA) | 0.005 | 0.01 |
| Example 7 (DDPyMMA) | 0.0025 | 0.0025 |
| Example 8 (XyDM) | 2.5 | 2.5 |

The compounds according to Examples 1, 3, 5, 6, and 7 were tested on bacterial strains different from *S. Mutans* with the same methods described above, obtaining the MIC and MBC values reported in Table 2.

TABLE 2 - MIC and MBC values against bacterial strains different from *S. Mutans*

| Compound | *E. Coli* [a] | | *S. Aureus* [b] | | *S. Sanguis* [c] | | *S. Mitis* [d] | |
|---|---|---|---|---|---|---|---|---|
| | MIC [e] | MCB[e] | MIC[e] | MCB[e] | MIC[e] | MCB [e] | MIC[e] | MCB [e] |
| DDM | 0.625 | 0.625 | 0.156 | 0.156 | 0.31 | 0.62 | 0.31 | 0.62 |
| DDMP | 1.25 | 1.25 | 0.625 | 1.25 | 0.62 | 0.62 | 0.62 | 0.62 |
| DDE | 0.078 | 0.156 | 0.02 | 0.039 | 0.02 | 0.039 | 0.039 | 0.039 |
| DDMAPMA | 0.002 | 0.002 | 0.01 | 0.01 | 0.005 | 0.005 | 0.01 | 0.01 |
| DDPyMMA | 0.0025 | 0.0025 | 0.0025 | 0.005 | 0.01 | 0.02 | 0.02 | 0.02 |
| a: E. coli *(ATCC25922);* b: S. Aureus *(ATCC 25923)*;c: S. Sanguis *(ATCC 10556)*; d: S. Mitis *(ATCC 49456* ); e: values as mg/mL. | | | | | | | | |

## Example 10

The compounds according to Examples 1, 5, 6, and 7 were tested in order to evaluate their cytotoxicity on primary cells from human dental pulp according to the "Determination of cytotoxicity" method described above, obtaining the values reported respectively in the following Tables 3, 4 ,5, and 6. Cells were extracted from healthy third molars of patients after informed consent (Authorization Single Regional Committee FVG, study ID 2433, 29 August 2018). After extraction,

the stem cell nature was ascertained by evaluating the expression of the positive markers CD 90, CD 73, CD 29 and the negative markers CD 14, CD 34 and CD 45.

TABLE 3 - Cytotoxicity of compound DDM according to Example 1

| DDM Concentration | % Viability 24 h | % Viability 72 h |
|---|---|---|
| 2 mg/mL | 80.6 ± 5.3 | 76.9 ± 8.4 |
| 500 μg/mL | 86.6 ± 5.5 | 115.3 ± 11.6 |
| 100 μg/mL | 102.7 ±7.2 | 122.4 ± 7.0 |
| 50 μg/mL | 105.6 ±11.2 | 111.0 ± 7.2 |
| 10 μg/mL | 95.3 ± 12.5 | 110.0 ± 13.8 |

From the obtained data, the compound according to Example 1 did not show significant signs of decrease in cell viability even at concentrations higher than the MIC and MBC, and therefore can also be used in contact with the oral mucosa.

TABLE 4 - Cytotoxicity of compound DDE according to Example 5

| DDE Concentration | % Viability 24 h | % Viability 72 h |
|---|---|---|
| 1 mg/mL | (-0.4) ± (-0.7) | (-1.9) ± (-3.6) |
| 500 μg/mL | 2.5 ± 1.0 | (-2.8) ± (-2.0) |
| 100 μg/mL | 95.9 ± 7.2 | 68.8 ± 17.5 |
| 50 μg/mL | 83.4 ± 4.5 | 70.8 ± 17.8 |
| 10 μg/mL | 119.2 ± 6.8 | 69.7 ± 17.0 |
| 1 μg/mL | 124.2 ± 6.3 | 72.6 ± 16.5 |

From the obtained data, compound DDE according to Example 5 shown a maximum non-cytotoxic concentration (100 μg/mL) higher than its MIC and MBC values against the analyzed bacteria strains, thereby confirming the suitability of the compound to be used also in contact with the oral mucosa.

TABLE 5 - Cytotoxicity of compound DDMAPMA according to Example 6

| DDMAPMA Concentration | % Viability 24 h | % Viability 72 h |
|---|---|---|
| 1 mg/mL | 2.5 ± 0.9 | 3.5 ± 1.5 |
| 500 μg/mL | 1.6 ± 0.9 | 2.6 ± 3.6 |
| 100 μg/mL | 11.3 ± 2.1 | 2.0 ± 0.8 |
| 50 μg/mL | 81.7 ± 8.2 | 71.5 ± 16.3 |
| 10 μg/mL | 99.5 ± 13.5 | 97.7 ± 14 |
| 1 μg/mL | 95.2 ± 13.2 | 103.2 ± 18.9 |

The Applicant observed that the maximum tested non-cytotoxic concentration of the compound DDMAPMA according to Example 6 is higher than the respective MIC and MBC values (5-10 μg/mL) against the analyzed bacteria strains, thereby confirming the suitability of the compound to be also used in contact with the oral mucosa.

TABLE 6 - Cytotoxicity of compound DDPyMMA according to Example 7

| DDPyMMA Concentration | % Viability 24 h | % Viability 72 h |
|---|---|---|
| 1 mg/mL | 49.1 ± 8.1 | 5.0 ± 2.3 |
| 500 μg/mL | 91.9 ± 16.8 | 26.5 ± 5.1 |
| 100 μg/mL | 91.7 ± 12.0 | 71.8 ± 13.4 |
| 50 μg/mL | 96.1 ± 10.5 | 86.3 ± 16.4 |
| 10 μg/mL | 105.9 ± 11.9 | 100.6 ± 17.7 |
| 1 μg/mL | 110.8 ± 15.9 | 97.3 ± 17.5 |

The Applicant observed that for the compound DDPyMMA according to Example 7 the concentrations corresponding to the MIC and MBC values against the analyzed bacteria strains are lower than the maximum tested non-cytotoxic concentration (50 μg/mL), thereby confirming the suitability of DDPyMMA for it use in contact with the oral mucosa.

In conclusion, from the data shown in Examples 9 and 10 it is possible to conclude that the compounds according to the invention show an adequate balance of antibacterial properties and cytotoxicity profile which allow their use in contact with the oral mucosa, especially in compositions for dental adhesives.

**Example 11**

The bactericidal properties of the compounds according to Examples 1, 3, 5, 6 and 7 were tested in various *primer*-type preparations, using the method "Bacterial inhibition determination - Agar diffusion test " described above. The disk soaked in the tested *primer* formulation without addition of compounds according to Examples 1, 3, 5, 6 and 7 was used as a negative reference.

For the tests, the following *primer*-type formulations were used, identified by the following codes:

*Primer L1 (% by weight):*

- 59.25% Bis-GMA (bisphenol A glycidyl methacrylate, CAS 1565-94-2, Sigma-Aldrich);
- 39.5% HEMA (hydroxyethyl methacrylate, CAS 868-77-9, Sigma-Aldrich);
- 0.25% CQ (Camphorquinone, CAS 10373-78-1, Sigma-Aldrich);
- 1% EDMAB (ethyl-4-dimethylamino benzoate, CAS 10287-53-3, Sigma-Aldrich)

*Primer* "L1" was diluted with 20% by weight of solvent, water ($H_2O$) or ethanol (EtOH) obtaining the following mixtures:

$$L1A = 80\%\ L1 + 20\%\ H_2O;$$

$$L1B = 80\%\ L1 + 10\% H_2O + 10\%\ EtOH;$$

and

$$L1C = 80\%\ L1 + 20\%\ EtOH.$$

*2. Primer L2 (% by weight):*

- 38% Bis-MP (Bis[2-(methacryloyloxy)ethyl]phosphate, CAS 32435-46-4, Sigma-Aldrich)
- 40% HEMA (hydroxyethyl methacrylate, CAS 868-77-9, Sigma-Aldrich);
- 20.75% TEGDMA (triethylene glycol dimethacrylate, CAS 109-16-0, Sigma-Aldrich);
- 0.25% CQ (Camphorquinone, CAS 10373-78-1, Sigma-Aldrich);
- 1% EDMAD (ethyl-4-dimethylamino benzoate, CAS 10287-53-3, Sigma-Aldrich)

*Primer* "L2" was diluted with 15% by weight of solvent (water or ethanol) obtaining the following mixtures:

$$L2A = 85\%\ L2 + 15\%\ H_2O;\ e$$

$$L2B = 85\%\ L2 + 7.5\% H_2O + 7.5\%\ EtOH.$$

*3. Primer R5 (% by weight):*

- 30% Bis-MP (Bis[2-(metacriloilossi)etil] phosphate, CAS 32435-46-4, Sigma-Aldrich);
- 28.75% HEMA (hydroxyethyl methacrylate, CAS 868-77-9, Sigma-Aldrich);
- 40% Bis-GMA (bisphenol A glycidyl methacrylate, CAS 1565-94-2, Sigma-Aldrich);
- 0.25% CQ (Camphorquinone, CAS 10373-78-1, Sigma-Aldrich);
- 1% EDMAD (ethyl-4-dimethylamino benzoate, CAS 10287-53-3, Sigma-Aldrich)

*Primer* "R5" was diluted with 20% by weight of solvent (water or ethanol) obtaining the following mixtures:

$$R5A = 80\% \ R5 + 20\% \ H_2O;$$

$$R5B = 80\% \ R5 + 10\% H_2O + 10\% \ EtOH.$$

*4. Primer Pa (% by weight):*

- 50% HEMA (hydroxyethyl methacrylate, CAS 868-77-9, Sigma-Aldrich);
- 30% TEGDMA (triethylene glycol dimethacrylate, CAS 109-16-0, Sigma-Aldrich);
- 20% methacrylic acid (CAS 79-41-4, Sigma-Aldrich)

*Primer* "Pa" was diluted with 20% by weight of water obtaining the following final mixture:

$$PaA = 80\% \ Pa + 20\% \ H_2O.$$

*5. Primer L3 (% by weight):*

- 50% HEMA (hydroxyethyl methacrylate, CAS 868-77-9, Sigma-Aldrich); e
- 50% EtOH (90%).

Tables 7, 8 e 9 show the obtained results.

TABLE 7 - *Primer* formulations with the addition of 1-10% by weight of the DDM compound according to Example 1

| *Primer* formulation | DDM % by weight | Inhibition zone (mm) |
|---|---|---|
| L1A | 0 | 10 |
| | 1 | 15 |
| | 5 | 20 |
| | 10 | 25 |
| L1B | 0 | 0 |
| | 1 | 5 |
| | 5 | 15 |
| | 10 | 19 |
| L1C | 0 | 0 |
| | 1 | 10 |
| | 5 | 15 |
| | 10 | 20 |

For all three formulations of DDM primers, the greater the concentration of the monomer in solution, the higher the bactericidal action (a proportional increase in the range of inhibition of bacterial growth can be noted). Furthermore, the three different formulations containing the same concentration of monomer shown comparable bactericidal activity.

TABLE 8 - *Primer* formulations with the addition of 1-10% by weight of the DDMP compound according to Example 3

| *Primer* formulation | DDMP % by weight | Inhibition zone (mm) |
|---|---|---|
| L1A | 0 | 0 |
| | 1 | 8 |
| | 5 | 11 |
| | 10 | 13 |
| L1B | 0 | 0 |
| | 1 | 0 |
| | 5 | 7 |
| | 10 | 10 |
| L2A | 0 | 0 |
| | 1 | 6 |
| | 5 | 7 |
| | 10 | 10 |
| L2B | 0 | 6 |
| | 1 | 6 |
| | 5 | 7 |
| | 10 | 10 |
| R5A | 0 | 6 |
| | 1 | 6 |
| | 5 | 6 |
| | 10 | 8 |
| R5B | 0 | 0 |
| | 1 | 0 |
| | 5 | 0 |
| | 10 | 8 |

In general, the L1 and L2 primer formulations added with DDMP were found to be more efficient than those of R5 primers. Between them, the bactericidal action is overall comparable, although L1A shown a better effect.

In any case, the greater the concentration of the monomer in solution, the higher the bactericidal action (a proportional increase in the inhibition range of bacterial growth can be noted).

TABLE 9 - *Primer* formulations L1B, L3 and PaA with the addition of 1% by weight of the compound according to Examples 5, 6 e 7

| *Primer* formulation | Compound (1% by weight) | Inhibition zone (mm) |
|---|---|---|
| L1B | - | 0 |
| | DDE | 14 |
| | DDMAPMA | 14 |
| | DDPyMMA | 17 |

(continued)

| *Primer* formulation | Compound (1% by weight) | Inhibition zone (mm) |
|---|---|---|
| L3 | - | 0 |
| | DDE | 13 |
| | DDMAPMA | 15 |
| | DDPyMMA | 14 |
| PaA | - | 8 |
| | DDE | 12 |
| | DDMAPMA | 15 |
| | DDPyMMA | 13 |

All three primer formulations with all 3 monomer types tested shown comparable bactericidal activity. The presence of a modest zone of inhibition even in the control of the acid primer formulation (PaA) is probably due to bacterial death induced by the acidification of the culture medium.

**Example 12**

The bactericidal properties of the compounds according to Examples 1, 5, 6 and 7 within a reference *bonding* resin called R2 were tested using the "Direct contact test (DCT)" method described above. As a negative reference, the reference *bonding* resin was used without adding compounds according to the invention.

The reference resin called R2 has the following composition (% by weight):

- 70% Bis-GMA (bisphenol A glycidyl dimethacrylate, CAS 1565-94-2, Sigma-Aldrich);
- 28.75% TEGDMA (triethylene glycol dimethacrylate, CAS 109-16-0, Sigma-Aldrich);
- 0.25% CQ (Camphorquinone, CAS 10373-78-1, Sigma-Aldrich);
- 1% EDMAD (ethyl-4-dimethylamino benzoate, CAS 10287-53-3, Sigma-Aldrich)

and was diluted with 20% by weight of EtOH for use.

For the tests execution, different quantities of the compounds according to examples 1, 5, 6 and 7 were added to the resin R2 in a quantity range from 0.1 to 10% by weight. The homogeneity of the material to be tested was ensured before application in the wells by carrying out a sonication (50%, 5 min) and mixing with Vortex (800 rpm, 5 min) of the various resins added with the compound according to the invention.

Figure 39 shows the optical density curves recorded during the tests conducted with the DDM compound according to Example 1, added in concentrations of 1%, 5% and 10% by weight to the resin R2 and, for comparison, the curve of optical density of the resin R2 alone.

As can be seen from the low optical density values found during the tests, the resin containing the DDM compound according to Example 1 was able to inhibit bacterial survival by contact even at the lowest concentration tested (1% by weight).

Figure 40 shows the optical density curves recorded during the tests conducted with the DDE compound according to Example 5, added in concentrations of 1%, and 5% by weight to the resin R2 and, for comparison, the density curve R2 resin optics alone.

As can be seen from the low optical density values found during the tests, also the resin containing the DDE compound according to Example 5 was able to inhibit bacterial survival by contact even at the lowest concentration tested (1% by weight).

Figure 41 shows the optical density curves recorded during the tests conducted with the DDMAPMA compound according to Example 6, added in concentrations of 1%, and 0.5% by weight to the resin R2 and, for comparison, the density curve R2 resin optics alone.

As can be seen, the resin containing the DDMAPMA compound according to Example 6 already at a concentration of 0.5% by weight in the resin R2 was able to inhibit bacterial survival by contact.

Figure 42 shows the optical density curves recorded during the tests conducted with the DDPyMMA compound according to Example 7, added in concentrations of 0.1, 0.5 and 1% by weight to the resin R2 and, for comparison, the optical density curve of the R2 resin alone.

As can be seen, the resin containing the DDPyMMA compound according to Example 7 was able to inhibit bacterial survival by contact at a concentration of 1% by weight in the R2 resin.

**Example 13**

The ability of the compounds according to examples 5, 6 and 7 to enter the polymer chain during photopolymerization, and therefore to act as antibacterial monomers, was tested using the "Determination of the degree of conversion (DC)" method described above. As a reference, the reference resin was used without adding compounds according to the invention.

The compounds were added in quantities of 1% by weight to a reference resin called RT3 having the following composition:

- 70% Bis-GMA (bisphenol A glycidyl dimethacrylate, CAS 1565-94-2, Sigma-Aldrich);
- 28.75% HEMA (hydroxyethyl methacrylate, CAS 868-77-9, Sigma-Aldrich);
- 0.25% CQ (Camphorquinone, CAS 10373-78-1, Sigma-Aldrich);
- 0.5% TPO (diphenyl- (2,4,6-trimethylbenzoyl) phosphine oxide, CAS 75980-60-8, Sigma-Aldrich) ;
- 0.5% EDMAB (ethyl-4-dimethylamino benzoate, CAS 10287-53-3, Sigma-Aldrich)

diluted with 30% by weight of EtOH for use.

For the tests execution, the homogeneity of the material to be tested was ensured by carrying out a sonication (50%, 5 min) and mixing with Vortex (800 rpm, 5 min) of the various resins added with the compound according to the invention.

Figure 43 shows the comparison between the polymerization kinetics of the RT3 resin as such and with 1% by weight of the compounds according to Examples 5, 6, and 7. All three resins showed a value of DC% not significantly different from the reference resin, free of compounds according to the invention.

**Example 14**

The ability of the compounds according to examples 5, 6 and 7 to not affect the mechanical properties of the dental adhesives with which they are formulated, was tested using the method "Determination of the applied tensile strength (Microtensile Bond Strength - μTBS)" described above, by adding 1% by weight of compounds according to the invention to the commercial adhesive Adper™ Scotchbond™ Multi-Purpose Adhesive (SBMP; 3M ™ ESPE, St Paul, MN, USA), according to the following procedure.

1% by weight of a compound according to Examples 5, 6 and 7 was added to an exactly weighed quantity of SBMP inside a dark Eppendorf tube. The mixtures were sonicated (50%, 5 min) and mixed with Vortex (800 rpm, 5 min) until complete solubilization of the tested compounds. Thus, 3 new adhesive systems were obtained which were tested in terms of tensile strength (Microtensile Bond Strength test - μTBS). The test was performed by placing the individual samples (sticks) in the appropriate slots for the microtensile tests with cyanoacrylate glue using the specifications reported in the guidelines of the Academy of Dental Materials for this test (S. Armstrong, L. Breschi, M . Ozcan, F. Pfefferkorn, M. Ferrari, B. Van Meerbeek, Academy of Dental Materials guidance on in vitro testing of dental composite bonding effectiveness to dentin/enamel using micro-tensile bond strength (μTBS) approach, Dent. Mater. 33 (2) (2017) 133-143). In particular, the "active gripping" configuration was used with the aid of the Zapit glue (Dental Ventures of America, Corona, CA, USA;) and the "Microtensile tester" tool produced by Bisco Inc., Schaumburg, IL , USA.

Figure 44 shows the result of the tensile strength characterization tests carried out on the reference resin alone ("SBMP"), and on the resin added with 1% by weight of the compounds according to examples 5, 6 and 7 ("SBMP+1% DDE", "SBMP+1%DDMAPMA", and "SBMP+1% DDPyMMA") respectively.

The value of the tensile strength expressed in MPa is shown on the abscissa axis.

As can be seen, there is no statistical difference between the resistance of the SBMP resin alone and the resin added with 1% by weight of the compounds according to the invention, which therefore demonstrate that they do not affect the mechanical properties of the resin itself.

## Claims

**1.** A compound of formula (I):

$$A\text{-}R_1\text{-}B \qquad (I)$$

wherein:

$R_1$ is selected from the group consisting of:

$-(CH_2)_{12}-$, and

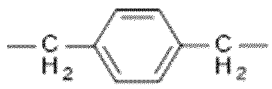

;

and

A and B are independently selected from the group consisting of:

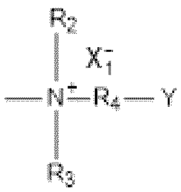

, and

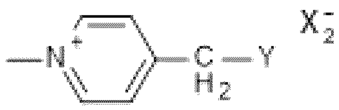

;

wherein:

$R_2$ and $R_3$ are independently selected from the group consisting of: methyl, ethyl, and n-propyl;
$R_4$ is selected from the group consisting of methylene, ethylene, n-propylene, and 1,4-phenylene;
Y is selected from the group consisting of:

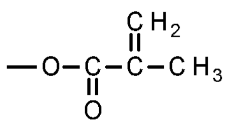

, and

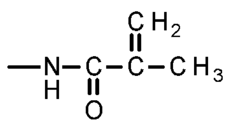

;

and

and $X_2^-$ are independently selected from the group consisting of: $F^-$, $Cl^-$, and $Br^-$.

2. The compound according to claim 1, wherein $R_1$ is the $-(CH_2)_{12}-$ group.

3. The compound according to claim 1 or 2, wherein $R_4$ is selected from the group consisting of ethylene, n-propylene, and 1,4-phenylene.

4. The compound according to any one of claims 1 to 3, wherein $R_2$ and $R_3$ are independently selected from the group consisting of: methyl, and ethyl.

5. The compound according to any one of claims 1 to 4, selected from:

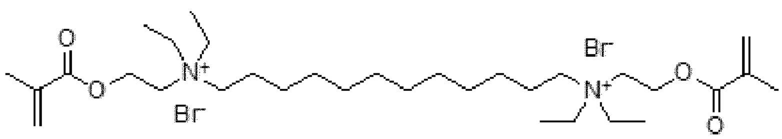

,

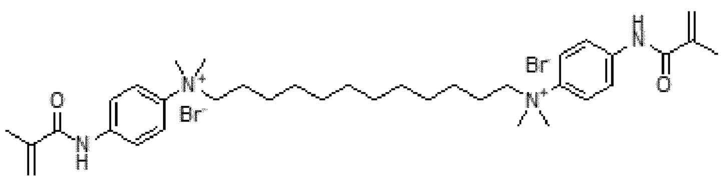

,

and

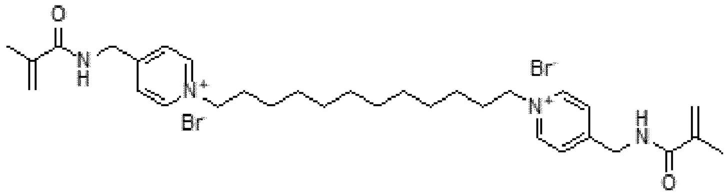

.

6. A photopolymerizable composition comprising at least one compound of formula (I) according to any one of claims 1 to 5, and at least one photopolymerization activator.

7. The photopolymerizable composition according to claim 6, comprising from 0.1% to 10% by weight of the at least one compound of formula (I) according to any one of claims 1 to 5.

8. The photopolymerizable composition according to claim 6 or 7, comprising at least one further compound comprising at least one ethylenic unsaturated group.

9. A compound according to any one of claims 1 to 5 or a photopolymerizable composition according to any of claims 6 to 8, for use as a medicament.

10. A compound according to any one of claims 1 to 5 or a photopolymerizable composition according to any of claims 6 to 8, for use in a dental treatment method.

11. The photopolymerizable compound or composition for use according to claim 10, wherein said method of dental treatment is a restorative method.

12. A use of at least one compound of formula (I) according to any one of claims 1 to 5, as an antibacterial monomer in polymeric compositions.

## Patentansprüche

1. Verbindung der Formel (I):

$$A\text{-}R_1\text{-}B \qquad (I)$$

wobei:

$R_1$ ausgewählt ist aus der Gruppe, bestehend aus:
$-(CH_2)_{12}-$, und

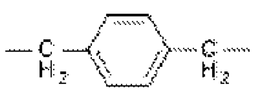

;

und
A und B unabhängig voneinander ausgewählt sind aus der Gruppe, bestehend aus:

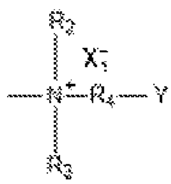

, und

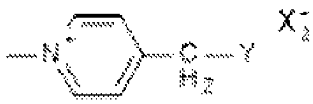

;

wobei:

$R_2$ und $R_3$ unabhängig voneinander ausgewählt sind aus der Gruppe, bestehend aus: Methyl, Ethyl und n-Propyl;
$R_4$ ausgewählt ist aus der Gruppe, bestehend aus Methylen, Ethylen, n-Propylen und 1,4-Phenylen;
Y ausgewählt ist aus der Gruppe, bestehend aus:

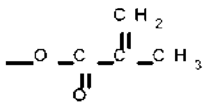 , und 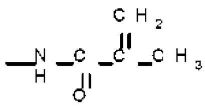 ;

und

$\overline{X_1}$ und $\overline{X_2}$ unabhängig voneinander ausgewählt sind aus der Gruppe, bestehend aus: $F^-$, $Cl^-$ und $Br^-$.

**2.** Verbindung nach Anspruch 1, wobei $R_1$ die Gruppe $-(CH_2)_{12}-$ ist.

**3.** Verbindung nach Anspruch 1 oder 2, wobei $R_4$ ausgewählt ist aus der Gruppe, bestehend aus Ethylen, n-Propylen und 1,4-Phenylen.

**4.** Verbindung nach einem der Ansprüche 1 bis 3, wobei $R_2$ und $R_3$ unabhängig voneinander ausgewählt sind aus der Gruppe, bestehend aus: Methyl und Ethyl.

**5.** Verbindung nach einem der Ansprüche 1 bis 4, ausgewählt aus:

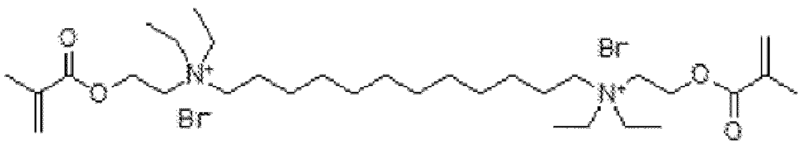 ,

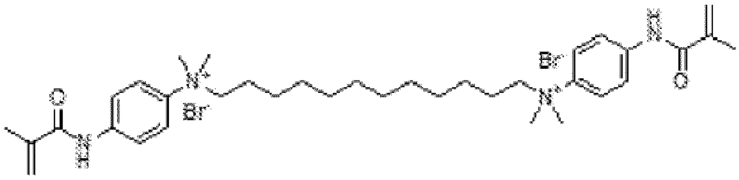 ,

und

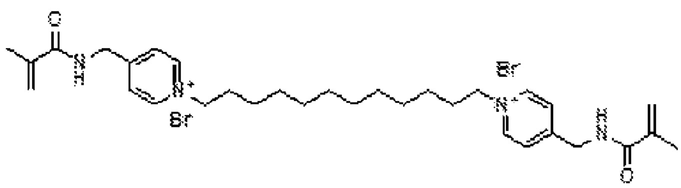 .

**6.** Fotopolymerisierbare Zusammensetzung, umfassend mindestens eine Verbindung der Formel (I) nach einem der Ansprüche 1 bis 5 und mindestens einen fotopolymerisierbaren Aktivator.

**7.** Fotopolymerisierbare Zusammensetzung nach Anspruch 6, umfassend 0,1 Gew.-% bis 10 Gew.-% der mindestens einen Verbindung der Formel (I) nach einem der Ansprüche 1 bis 5.

8. Fotopolymerisierbare Zusammensetzung nach Anspruch 6 oder 7, umfassend mindestens eine weitere Verbindung, die mindestens eine ungesättigte Ethylengruppe umfasst.

9. Verbindung nach einem der Ansprüche 1 bis 5 oder eine fotopolymerisierbare Zusammensetzung nach einem der Ansprüche 6 bis 8 zur Verwendung als Medikament.

10. Verbindung nach einem der Ansprüche 1 bis 5 oder eine fotopolymerisierbare Zusammensetzung nach einem der Ansprüche 6 bis 8 zur Verwendung in einem Zahnbehandlungsverfahren.

11. Fotopolymerisierbare Zusammensetzung oder Verbindung zur Verwendung nach Anspruch 10, wobei das Verfahren zur Zahnbehandlung ein restauratives Verfahren ist.

12. Verwendung der mindestens einen Verbindung der Formel (I) nach einem der Ansprüche 1 bis 5 als antibakterielles Monomer in Polymerzusammensetzungen.

## Revendications

1. Composé de formule (I) :

$$A\text{-}R_1\text{-}B \qquad (I)$$

dans lequel :

$R_1$ est choisi dans le groupe constitué par : $-(CH_2)_{12}-$, et

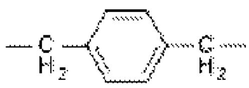

;

et

A et B sont choisis indépendamment dans le groupe constitué par :

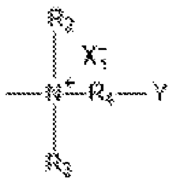

, et

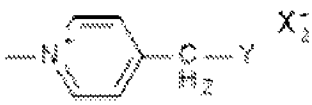

;

dans lequel :

$R_2$ et $R_3$ sont indépendamment choisis dans le groupe constitué par : le méthyle, l'éthyle et le n-propyle ;
$R_4$ est choisi dans le groupe constitué par le méthylène, l'éthylène, le n-propylène et le 1,4-phénylène ;
Y est choisi dans le groupe constitué par :

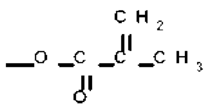

, et

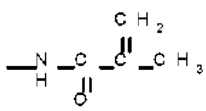

;

et

$X_1^-$ et $X_2^-$ sont indépendamment choisis dans le groupe constitué par : $F^-$ ; $Cl^-$ ; et $Br^-$.

2. Composé selon la revendication 1, dans lequel $R_1$ est le groupe $-(CH_2)_{12}$.

3. Composé selon la revendication 1 ou 2, dans lequel $R_4$ est choisi dans le groupe constitué par l'éthylène, le n-propylène et le 1,4-phénylène.

4. Composé selon l'une quelconque des revendications 1 à 3, dans lequel $R_2$ et $R_3$ sont indépendamment choisis dans le groupe constitué par : le méthyle et l'éthyle.

5. Composé selon l'une quelconque des revendications 1 à 4, choisi parmi :

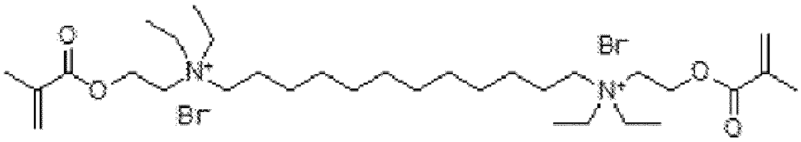

,

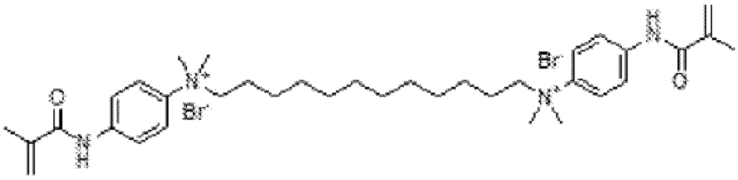

,

et

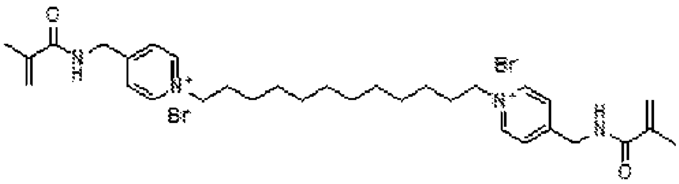

.

6. Composition photopolymérisable comprenant au moins un composé de formule (I) selon l'une quelconque des revendications 1 à 5, et au moins un activateur de photopolymérisation.

7. Composition photopolymérisable selon la revendication 6, comprenant de 0,1 % à 10 % en poids d'un moins un composé de formule (I) selon l'une quelconque des revendications 1 à 5.

8. Composition photopolymérisable selon la revendication 6 ou 7, comprenant au moins un autre composé comprenant au moins un groupe insaturé éthylénique.

9. Composé selon l'une quelconque des revendications 1 à 5, ou composition photopolymérisable selon l'une quelconque des revendications 6 à 8, pour une utilisation comme médicament.

10. Composé selon l'une quelconque des revendications 1 à 5 ou composition photopolymérisable selon l'une quelconque des revendications 6 à 8, pour une utilisation dans un procédé de traitement dentaire.

11. Composé ou composition photopolymérisable pour une utilisation selon la revendication 10, dans lequel ledit procédé de traitement dentaire est un procédé réparateur.

12. Utilisation d'au moins un composé de formule (I) selon l'une quelconque des revendications 1 à 5, comme monomère antibactérien dans des compositions polymériques.

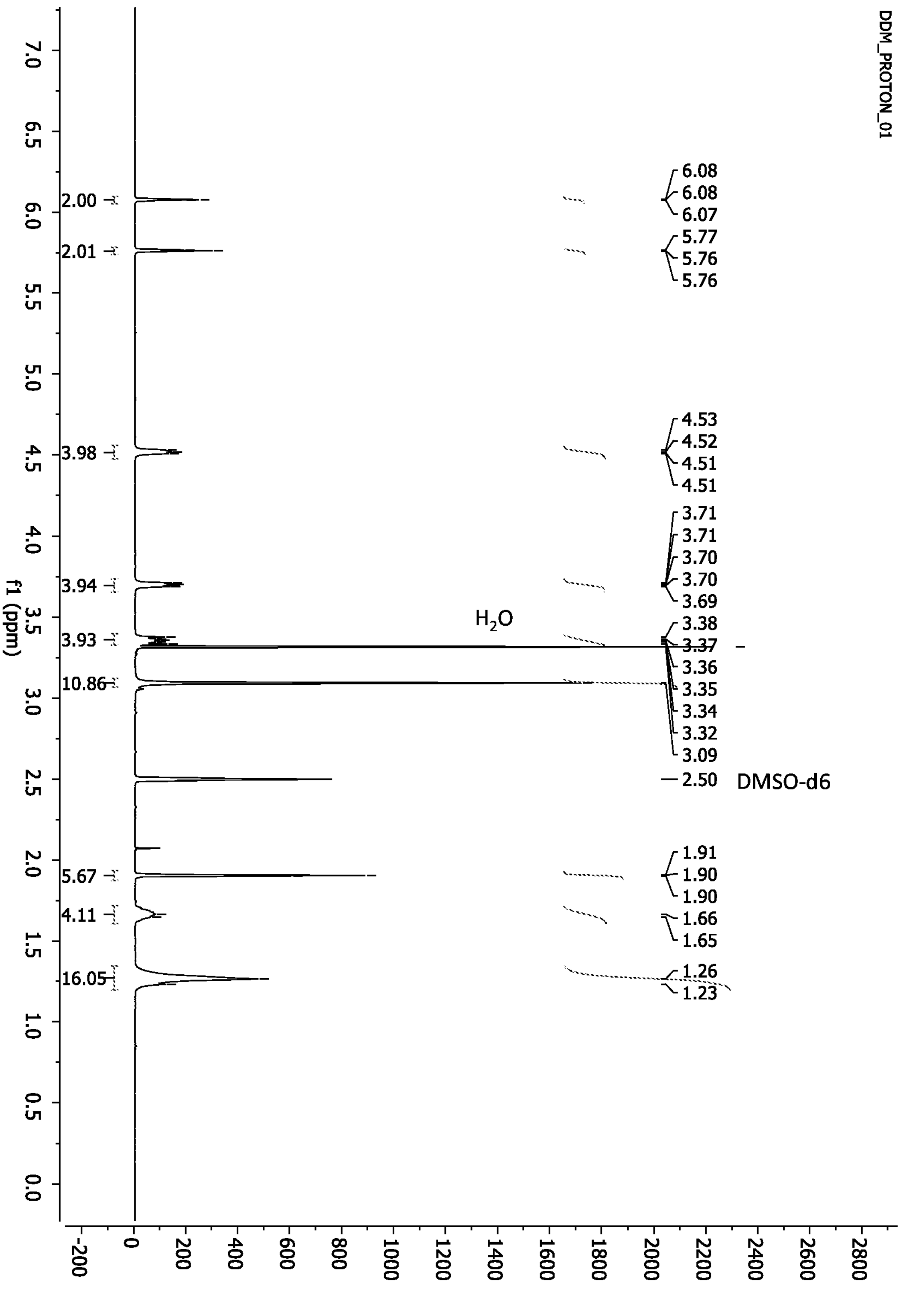


**FIG. 1**

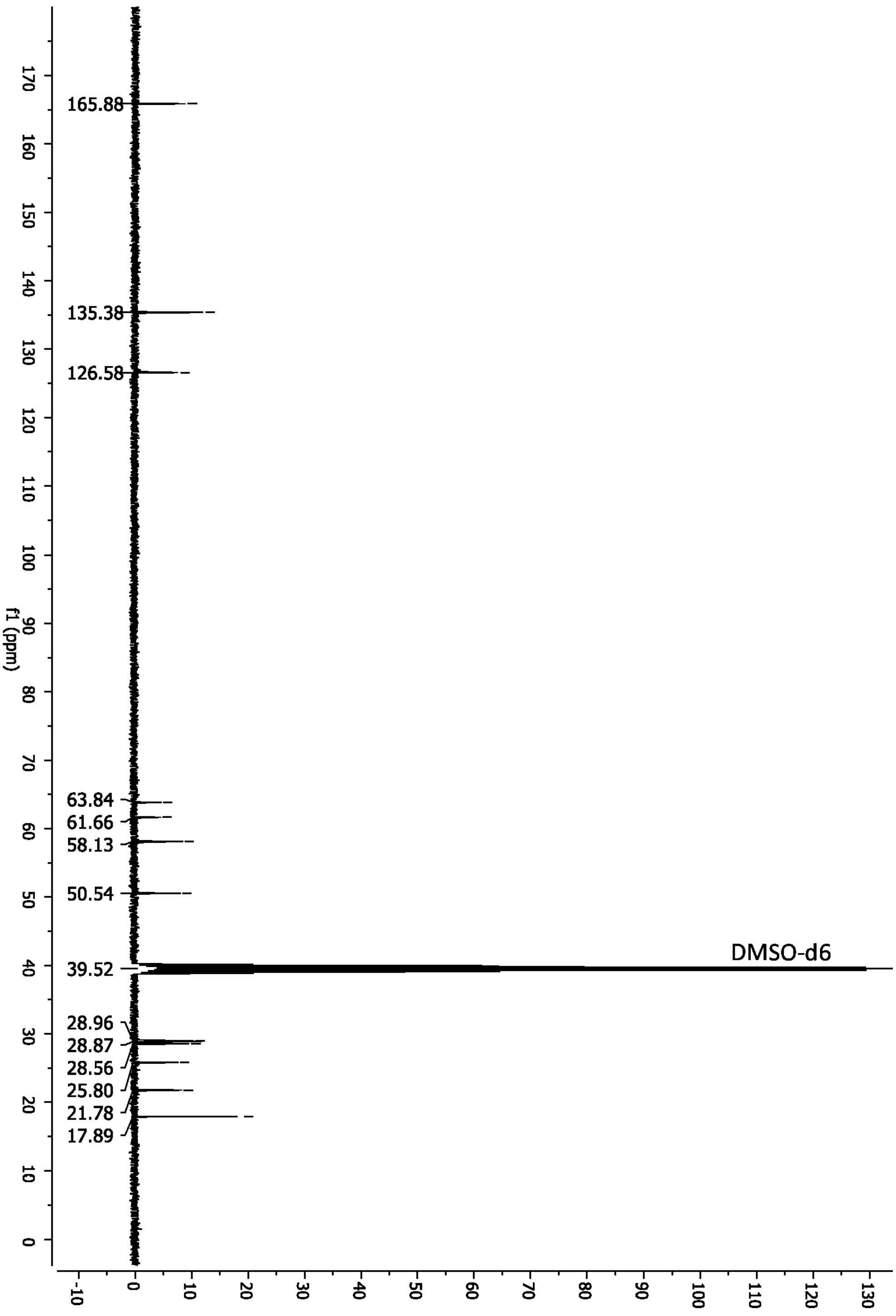
165.88
135.38
126.58
63.84
61.66
58.13
50.54
39.52
28.96
28.87
28.56
25.80
21.78
17.89
DMSO-d6
f1 (ppm)

**FIG. 2**

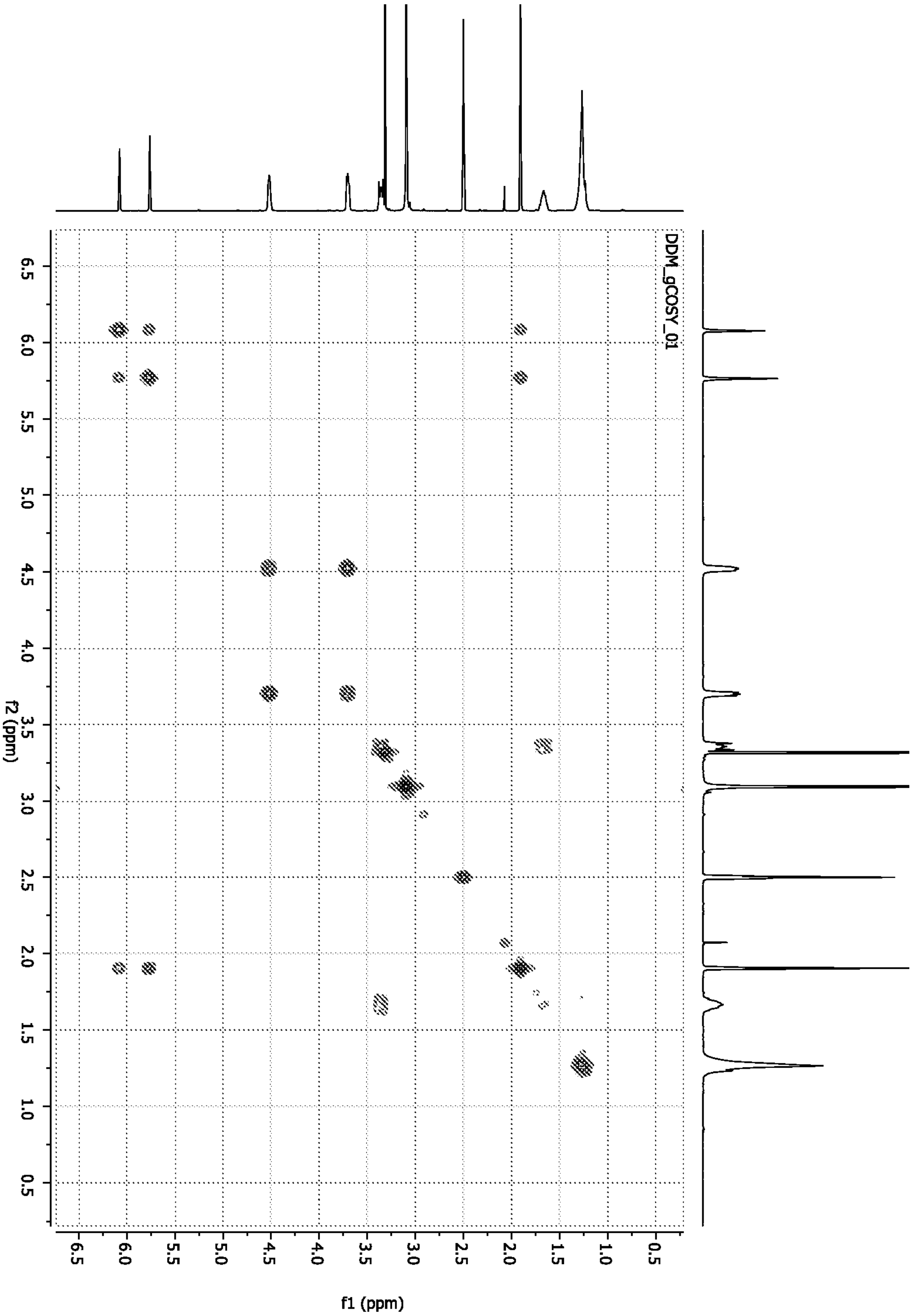
DDM_gCOSY_01
f2 (ppm)
6.5
6.0
5.5
5.0
4.5
4.0
3.5
3.0
2.5
2.0
1.5
1.0
0.5
f1 (ppm)
6.5
6.0
5.5
5.0
4.5
4.0
3.5
3.0
2.5
2.0
1.5
1.0
0.5

**FIG. 3**

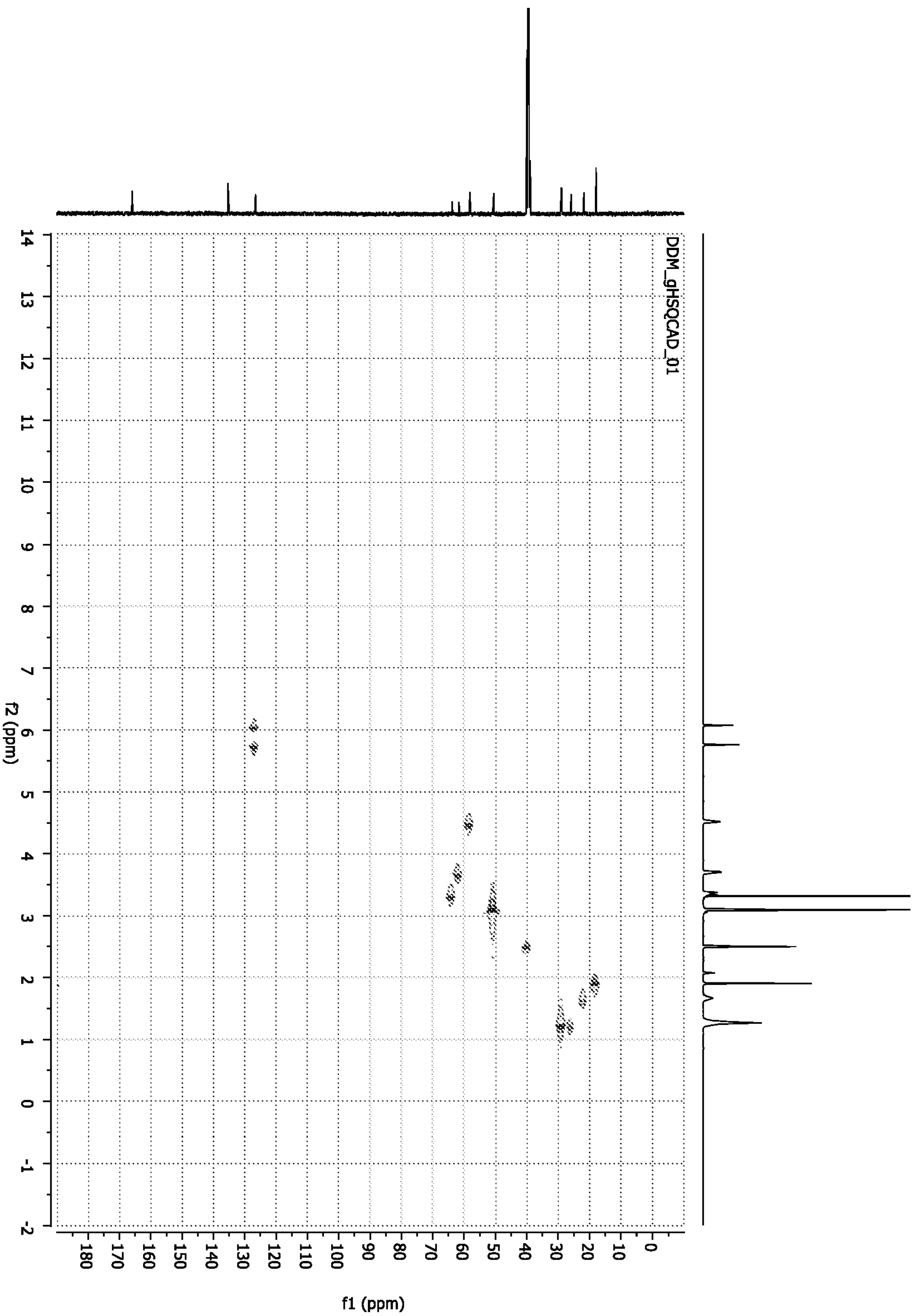


**FIG. 4**

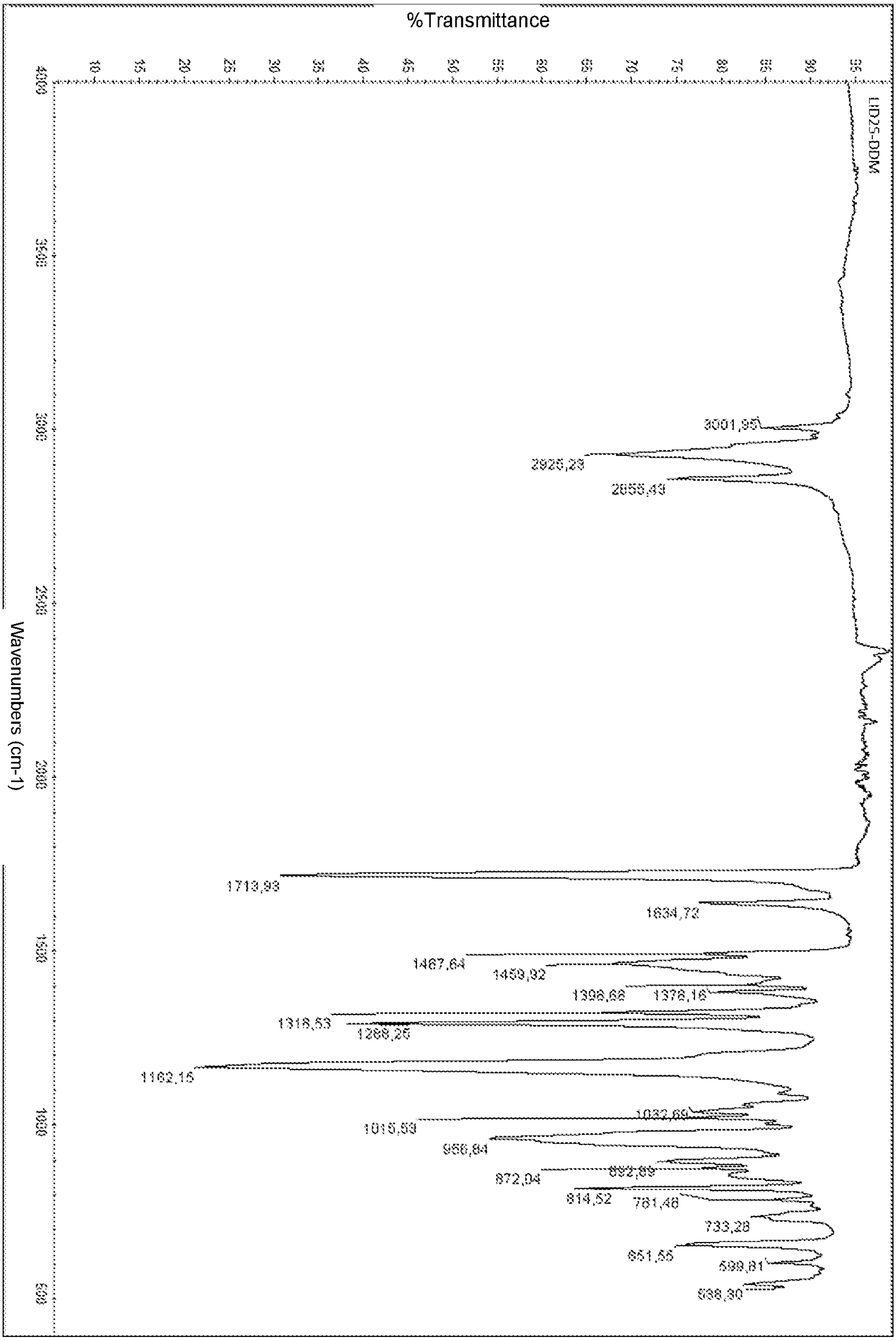
%Transmittance
Wavenumbers (cm-1)
LID25-DDM
3001,95
2925,23
2855,43
1713,93
1634,72
1467,64
1459,32
1398,68
1378,16
1318,53
1268,25
1162,15
1032,69
1015,53
956,84
892,89
872,04
814,52
781,46
733,28
651,55
599,81
538,30

**FIG. 5**

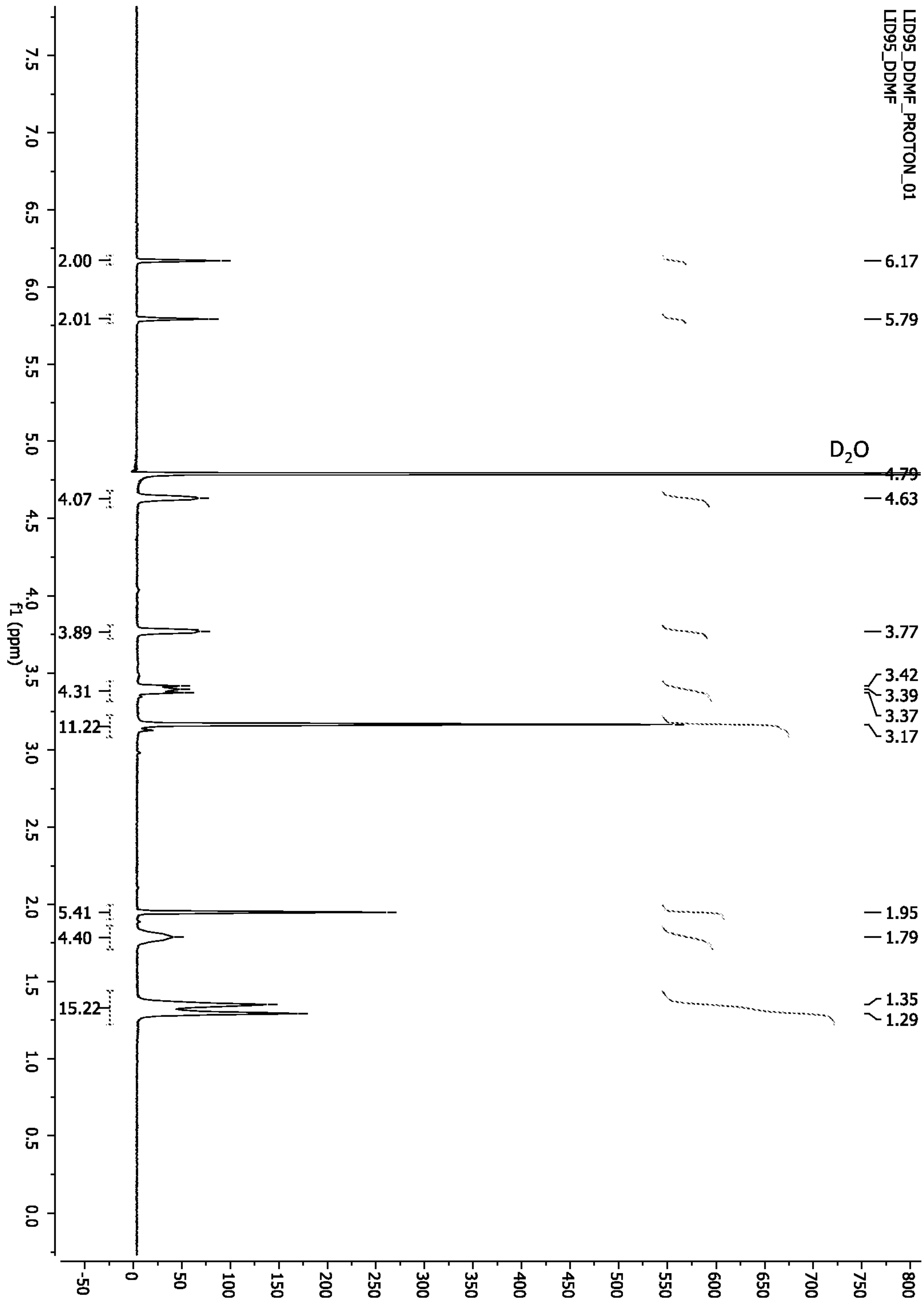
LID95_DDMF_PROTON_01
LID95_DDMF
D2O
6.17
5.79
4.79
4.63
3.77
3.42
3.39
3.37
3.17
1.95
1.79
1.35
1.29
2.00
2.01
4.07
3.89
4.31
11.22
5.41
4.40
15.22
f1 (ppm)

FIG. 6

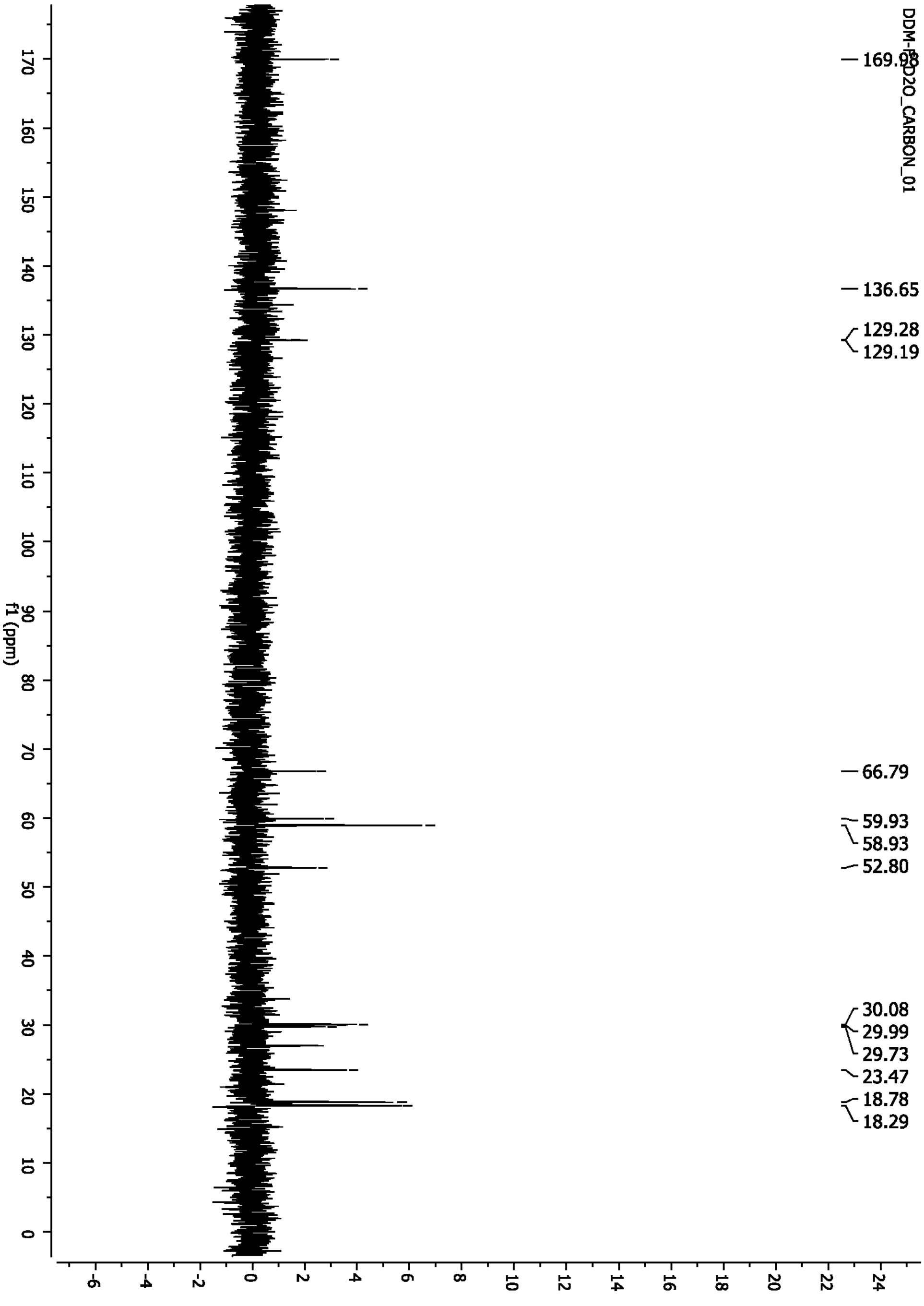
136.65
129.28
129.19
66.79
59.93
58.93
52.80
30.08
29.99
29.73
23.47
18.78
18.29
f1 (ppm)

**FIG. 7**

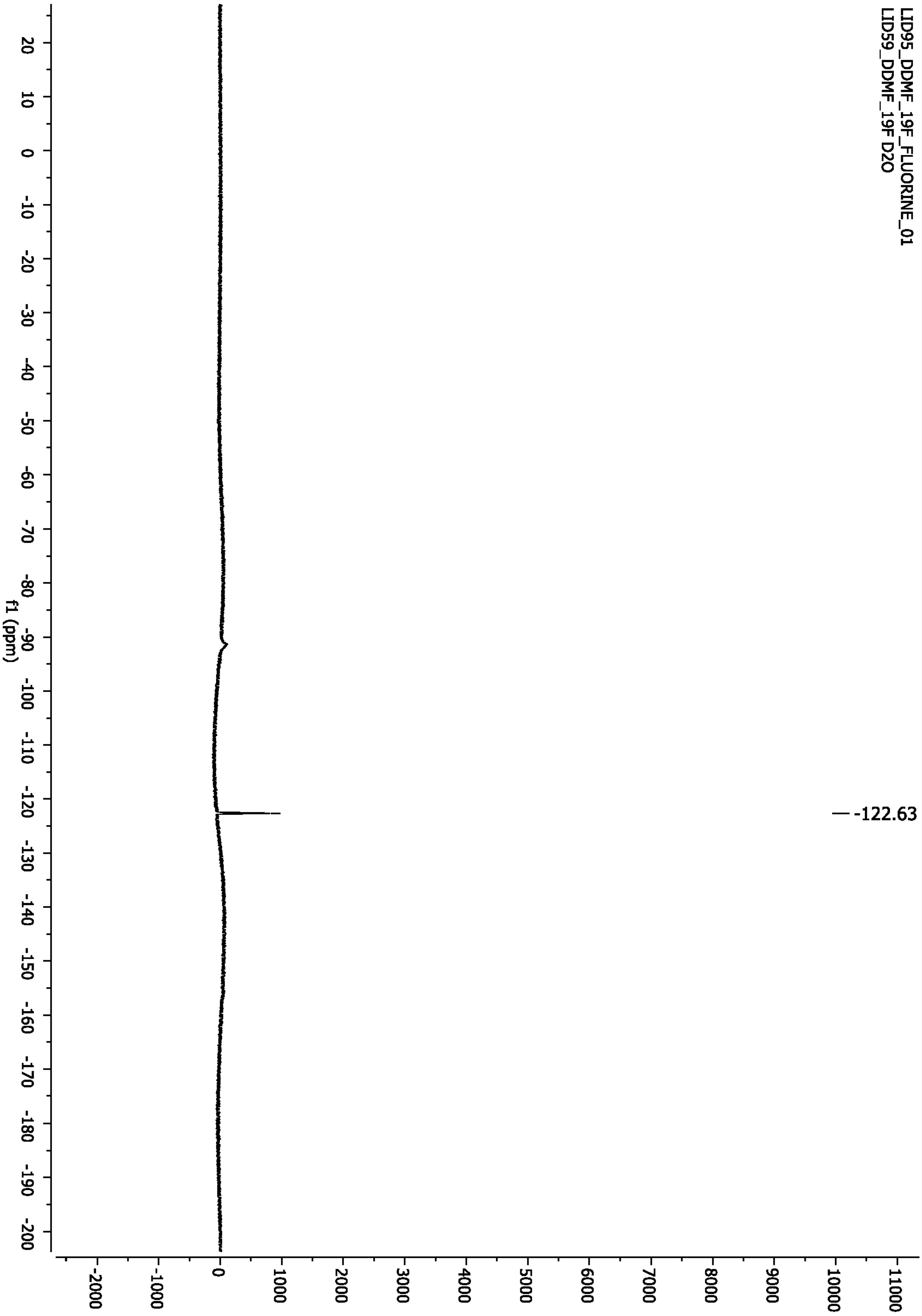


FIG. 8

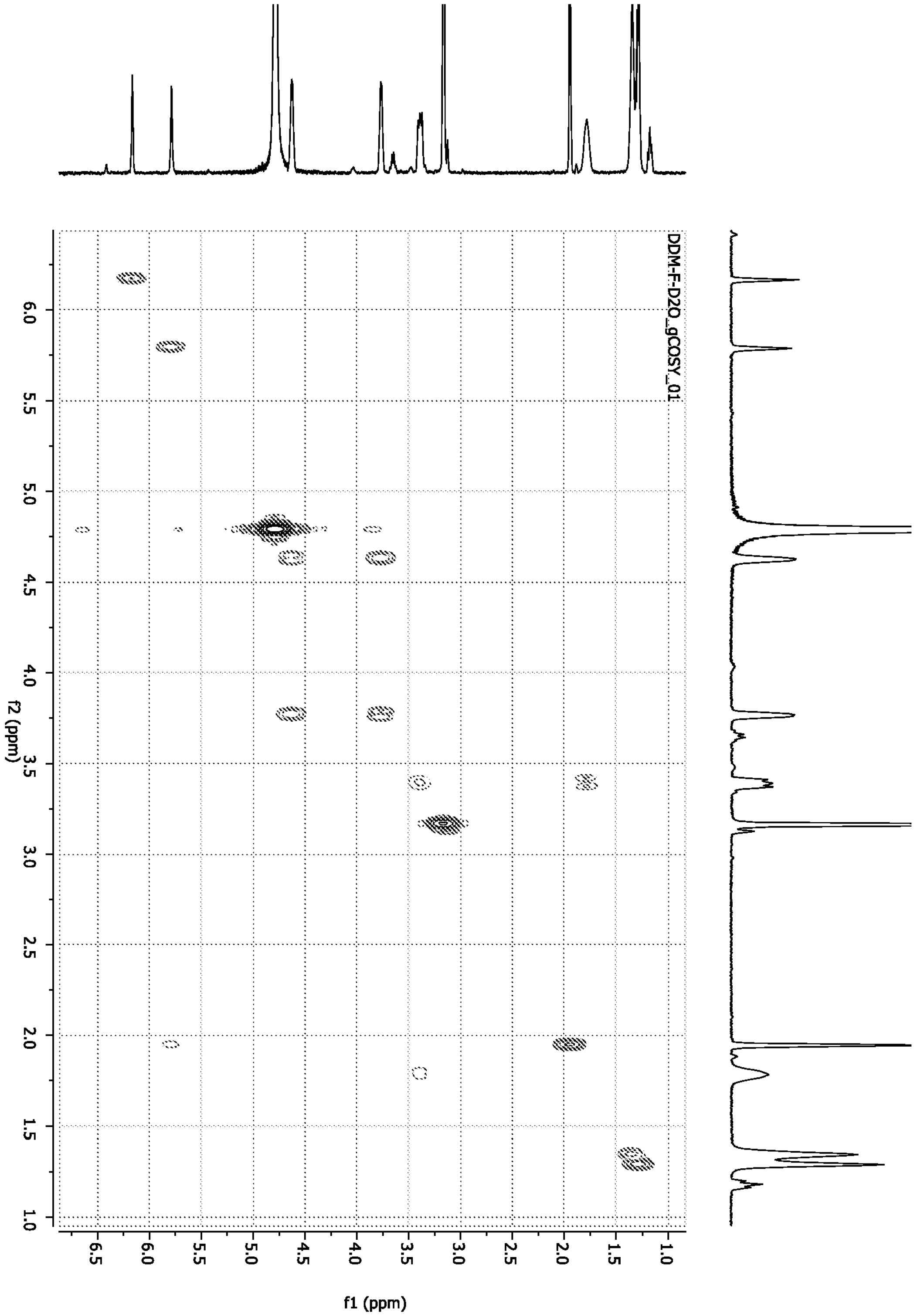


FIG. 9

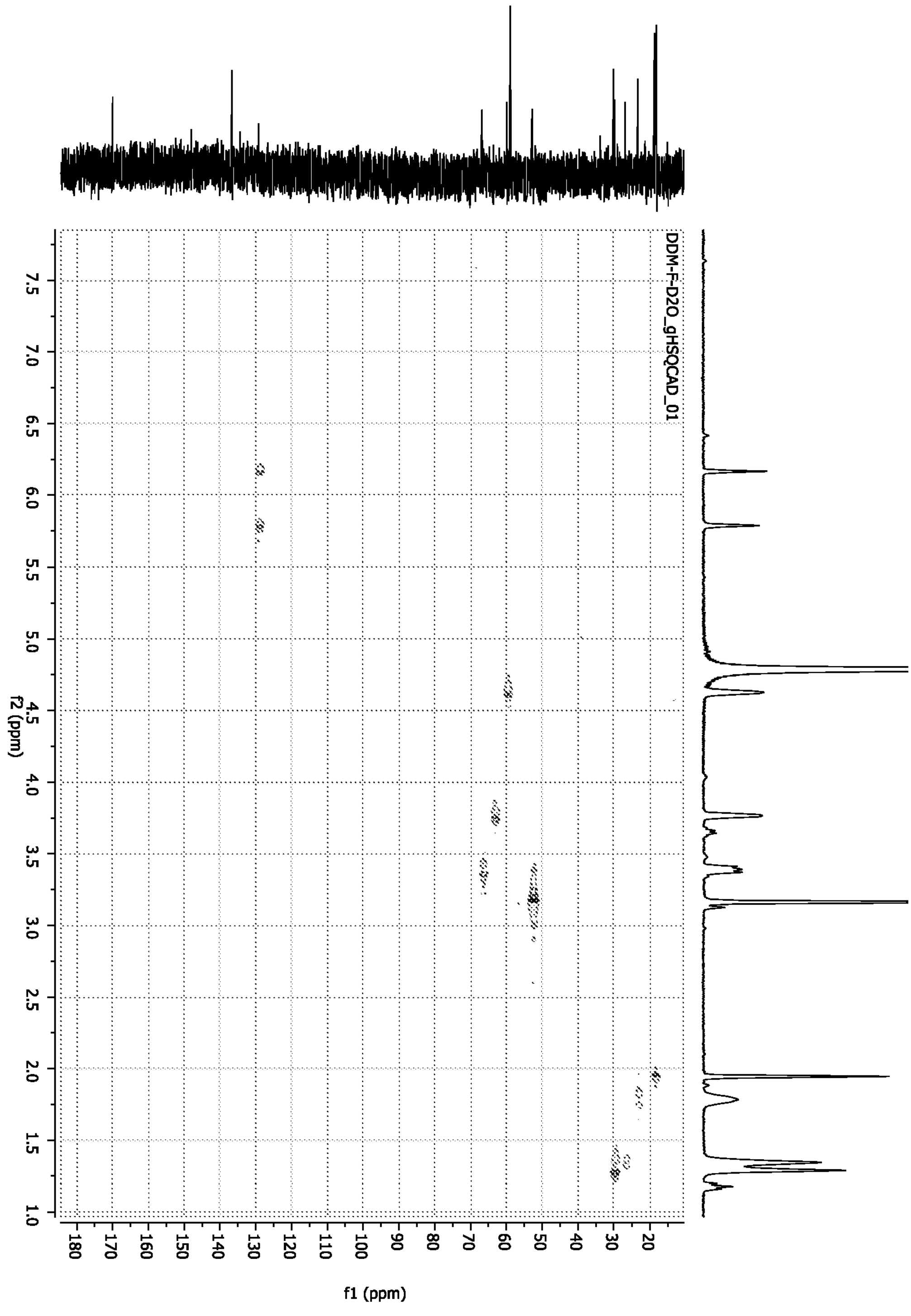


**FIG. 10**

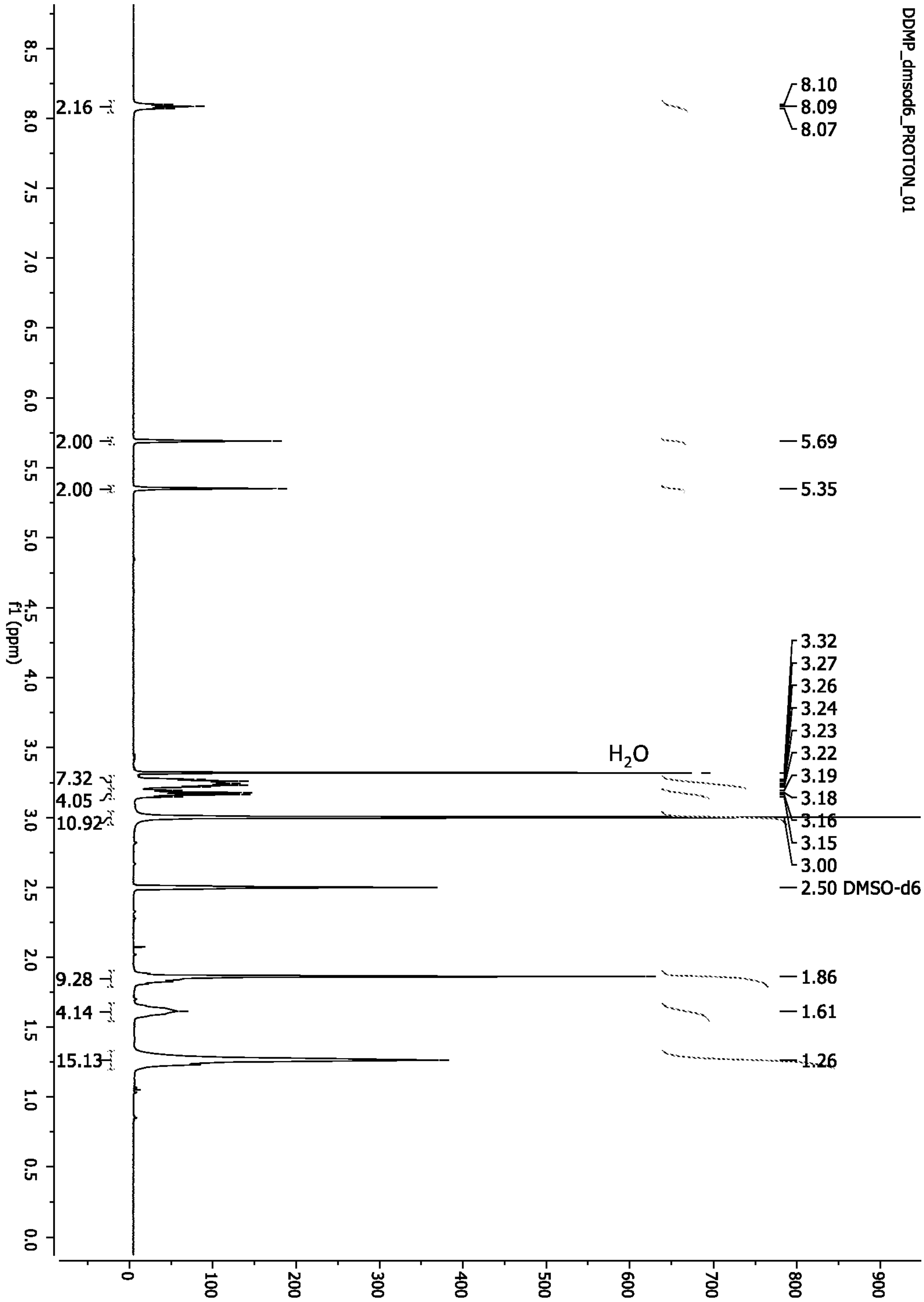
DDMP_dmsod6_PROTON_01
8.10
8.09
8.07
5.69
5.35
3.32
3.27
3.26
3.24
3.23
3.22
3.19
3.18
3.16
3.15
3.00
2.50 DMSO-d6
1.86
1.61
1.26
$H_2O$
2.16
2.00
2.00
7.32
4.05
10.92
9.28
4.14
15.13
f1 (ppm)

**FIG. 11**

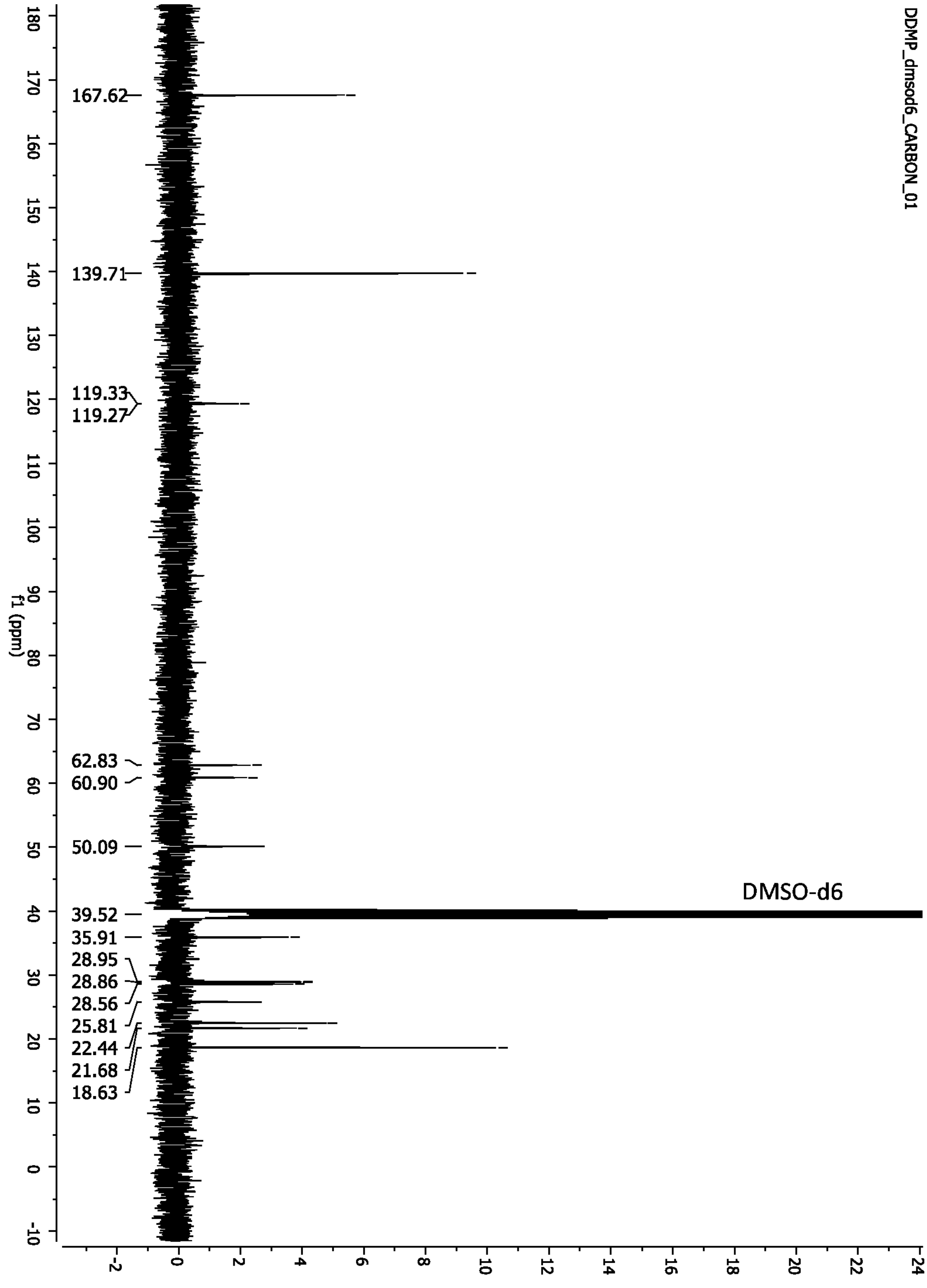
DDMP_dmsod6_CARBON_01
DMSO-d6
167.62
139.71
119.33
119.27
62.83
60.90
50.09
39.52
35.91
28.95
28.86
28.56
25.81
22.44
21.68
18.63
f1 (ppm)

FIG. 12

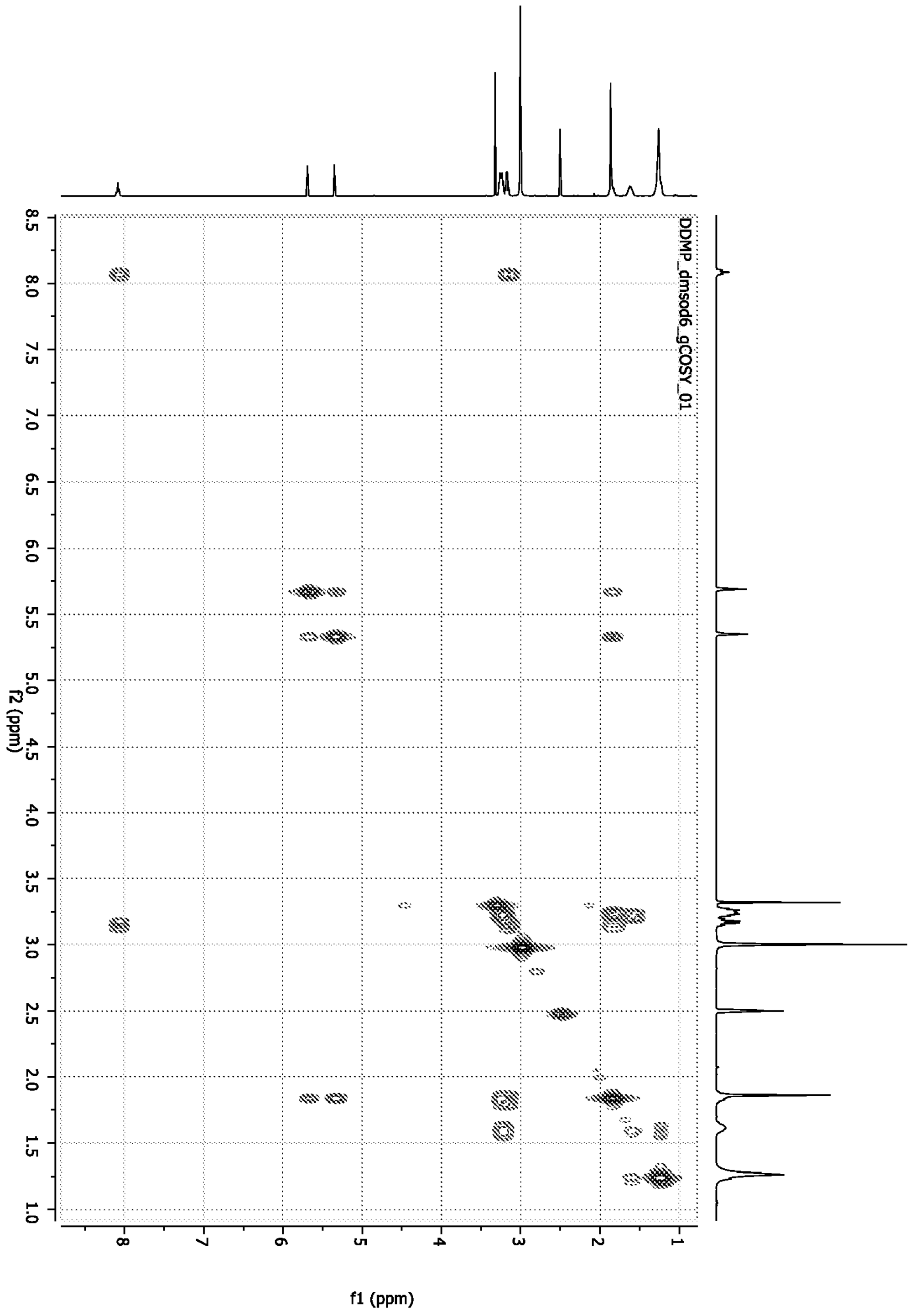


**FIG. 13**

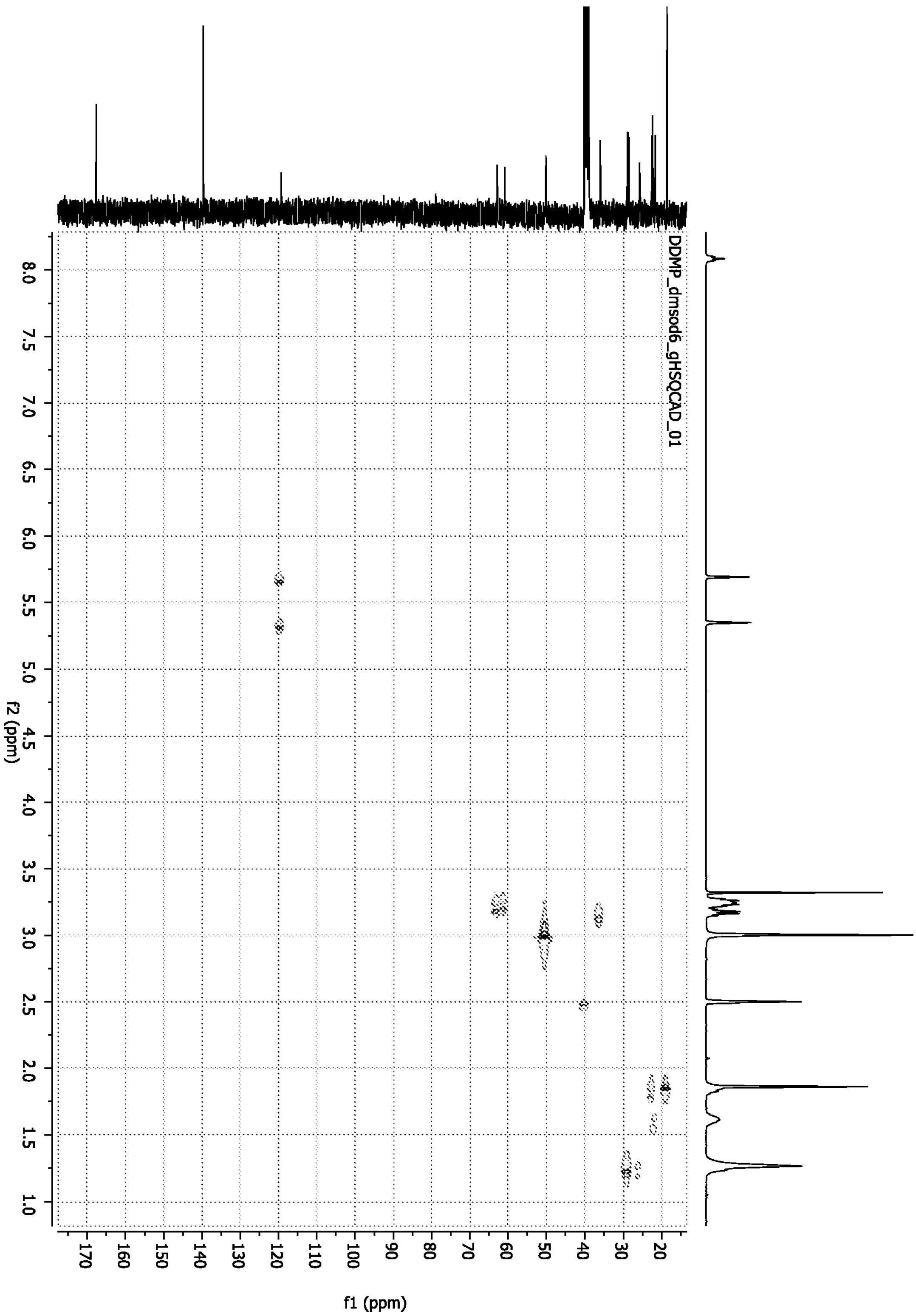


FIG. 14

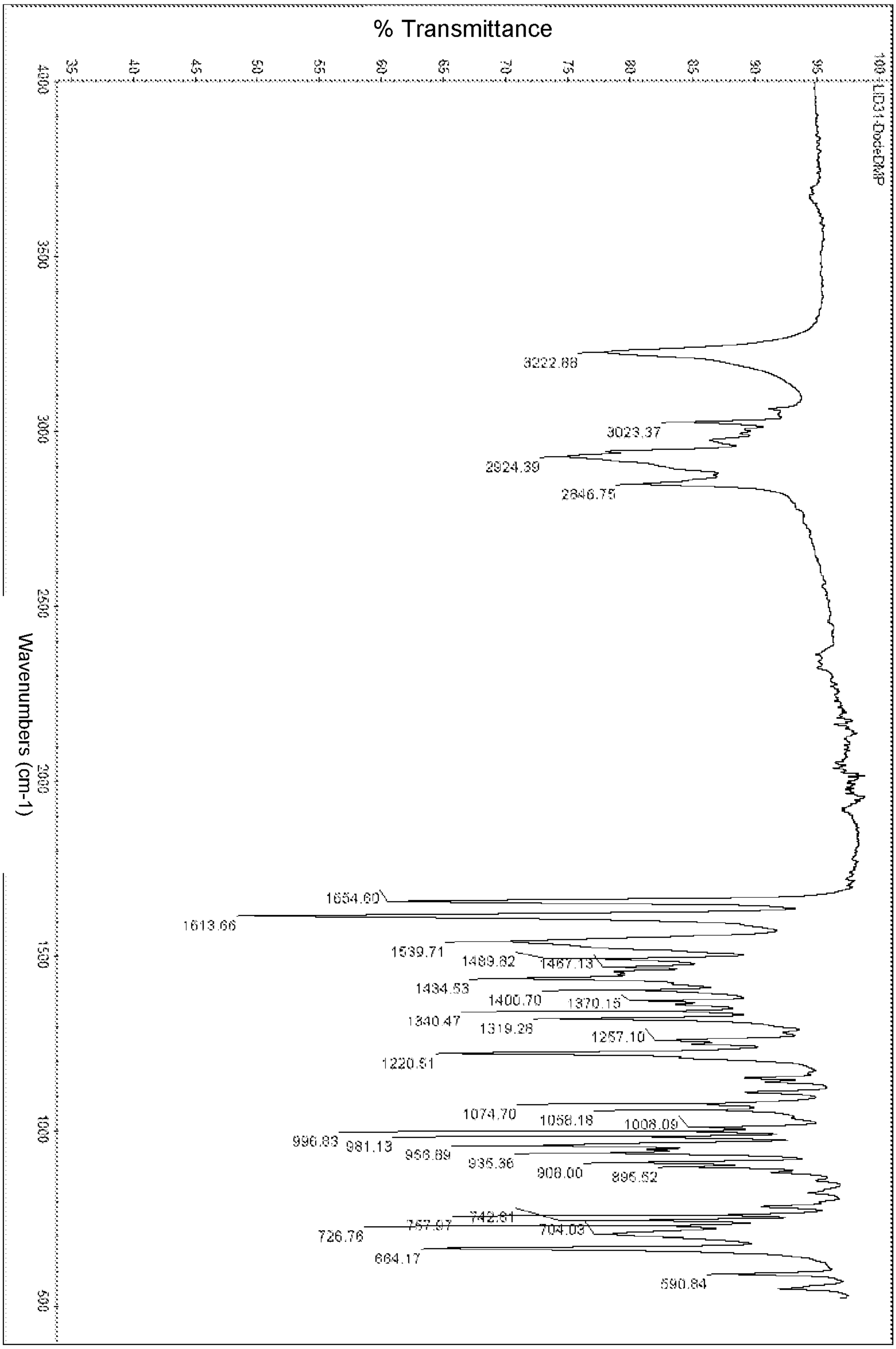
% Transmittance
Wavenumbers (cm-1)
3222.88
3023.37
2924.39
2846.75
1654.60
1613.66
1539.71
1489.82
1467.13
1434.53
1400.70
1370.15
1340.47
1319.28
1257.10
1220.51
1074.70
1058.18
1008.09
996.83
981.13
966.89
935.36
908.00
895.52
742.81
757.07
726.76
704.03
664.17
590.84

**FIG. 15**

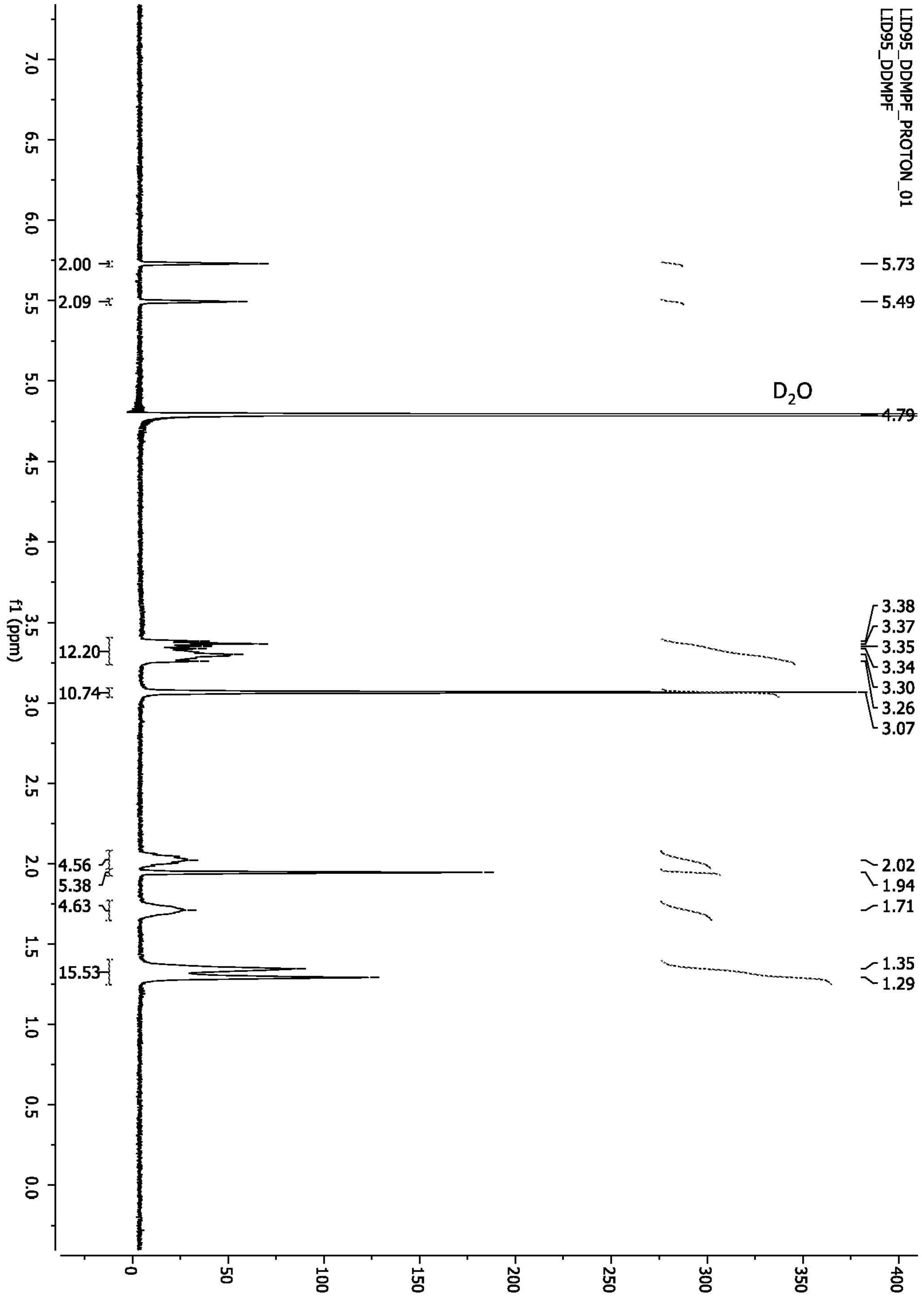
LID95_DDMPF_PROTON_01
LID95_DDMPF
D2O
f1 (ppm)
5.73
5.49
4.79
3.38
3.37
3.35
3.34
3.30
3.26
3.07
2.02
1.94
1.71
1.35
1.29
2.00
2.09
12.20
10.74
4.56
5.38
4.63
15.53

**FIG. 16**

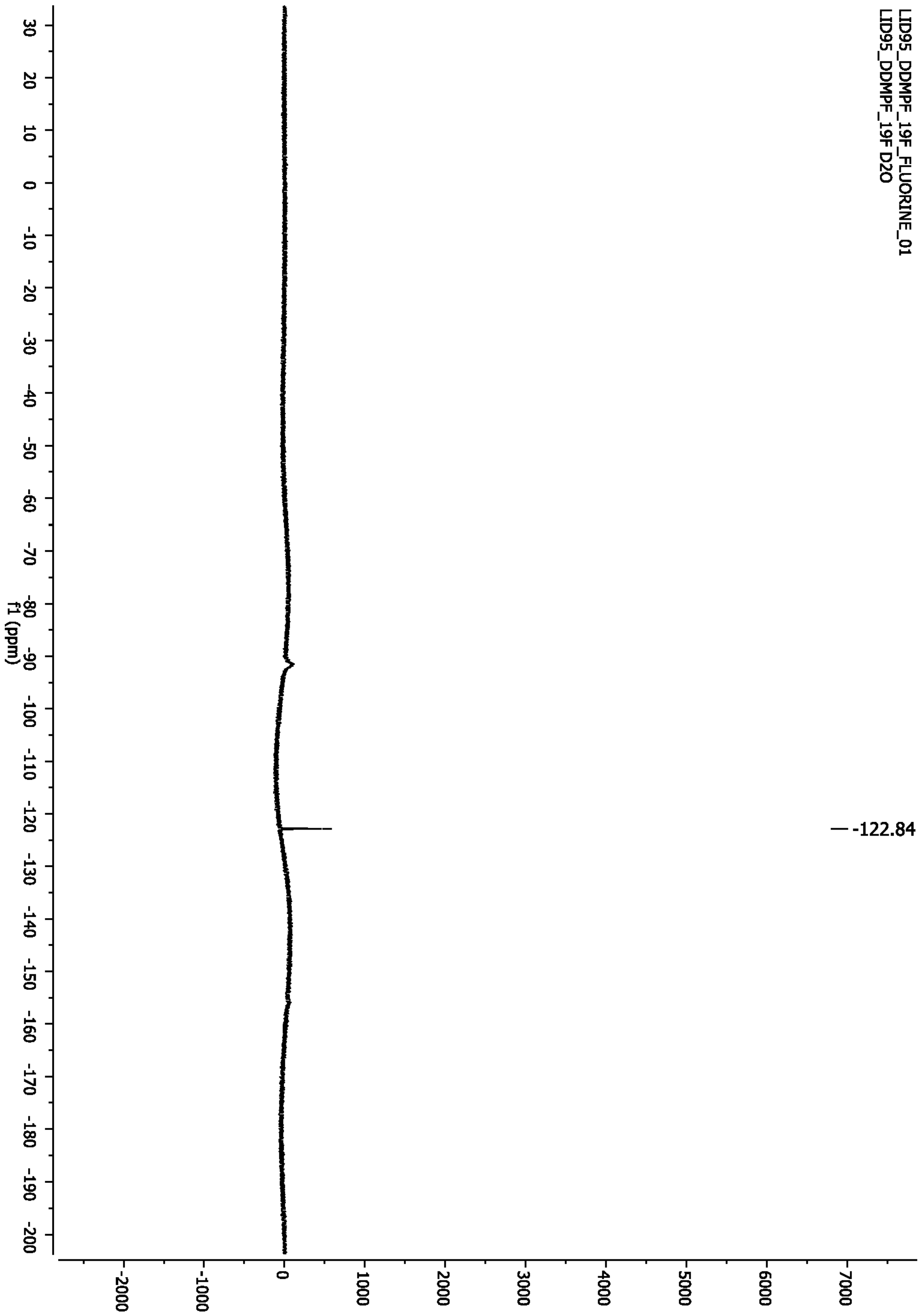
LID95_DDMPF_19F_FLUORINE_01
LID95_DDMPF_19F D2O
-122.84
f1 (ppm)
30
20
10
0
-10
-20
-30
-40
-50
-60
-70
-80
-90
-100
-110
-120
-130
-140
-150
-160
-170
-180
-190
-200
-2000
-1000
0
1000
2000
3000
4000
5000
6000
7000

**FIG. 17**

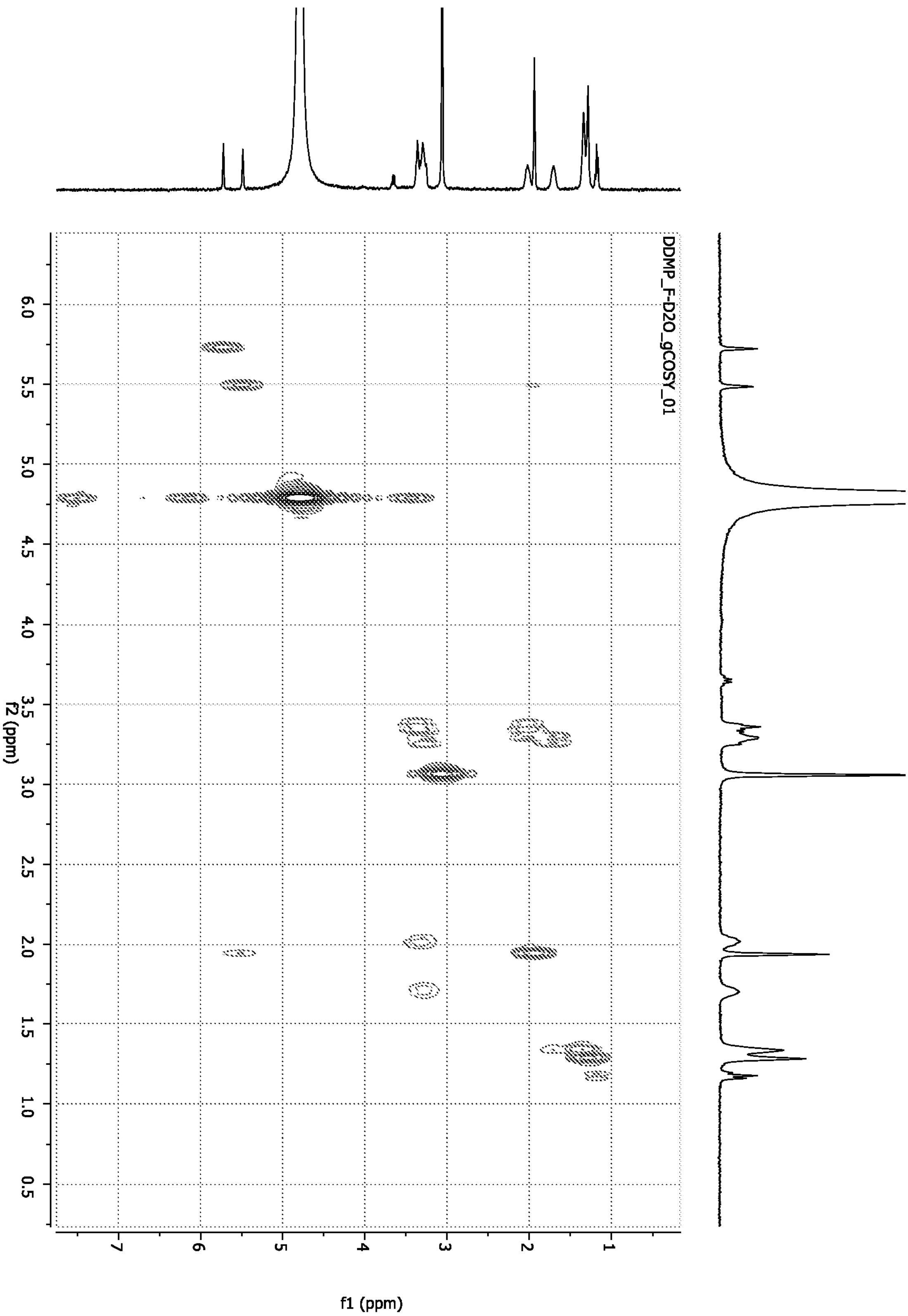


**FIG. 18**

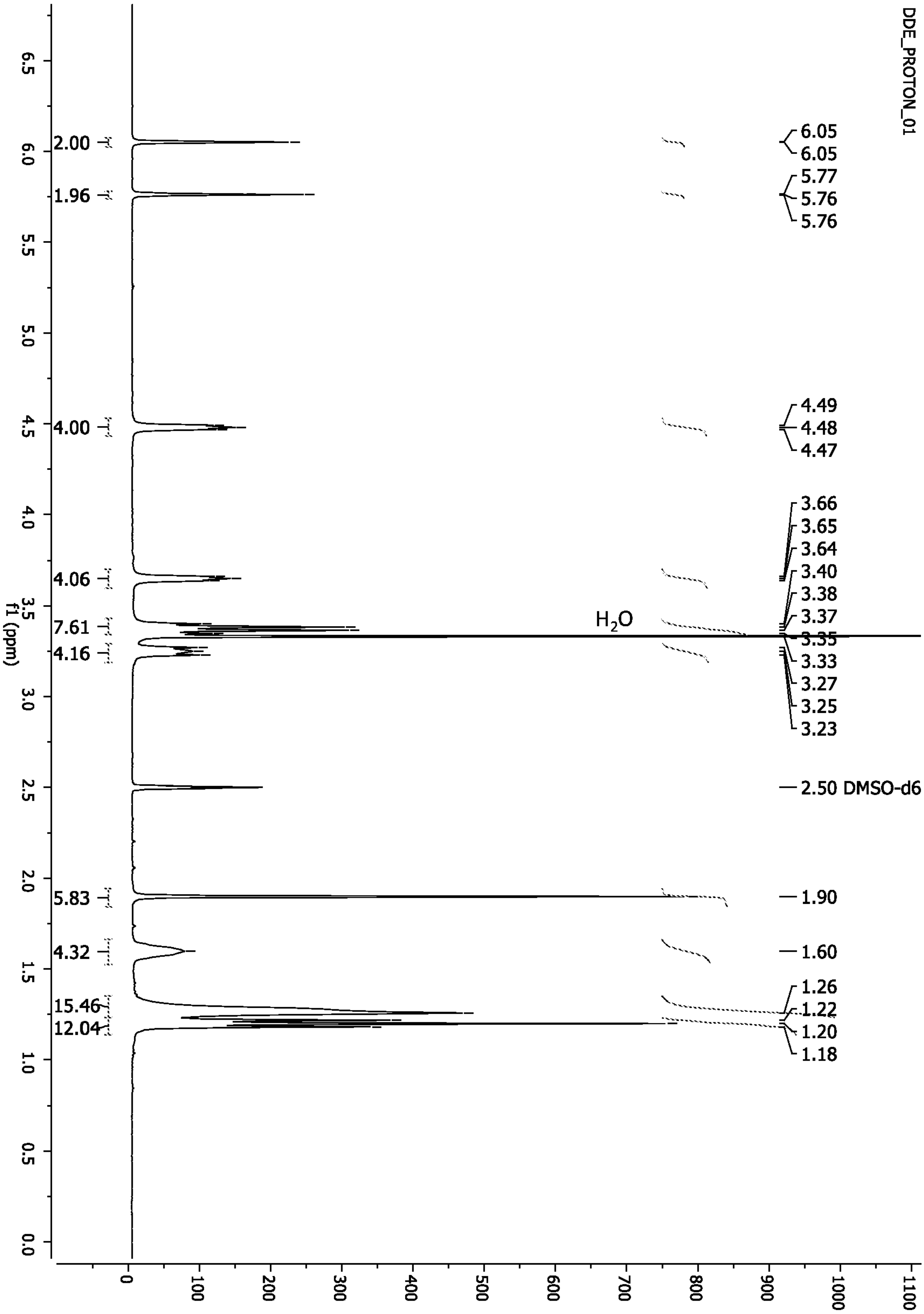
DDE_PROTON_01
2.00
1.96
4.00
4.06
7.61
4.16
5.83
4.32
15.46
12.04
6.05
6.05
5.77
5.76
5.76
4.49
4.48
4.47
3.66
3.65
3.64
3.40
3.38
3.37
3.35
3.33
3.27
3.25
3.23
2.50 DMSO-d6
1.90
1.60
1.26
1.22
1.20
1.18
$H_2O$
f1 (ppm)

**FIG. 19**

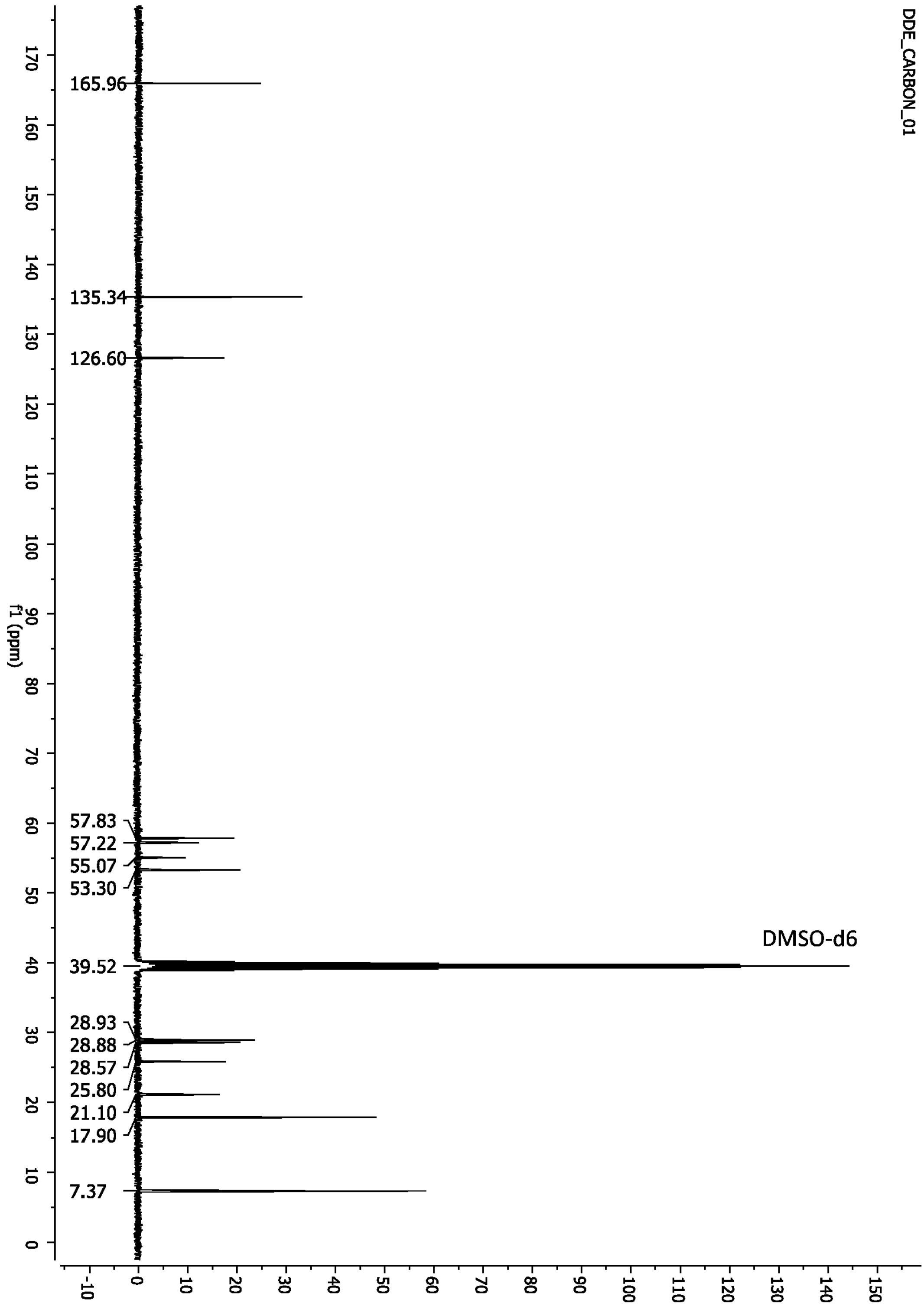
DDE_CARBON_01
165.96
135.34
126.60
57.83
57.22
55.07
53.30
DMSO-d6
39.52
28.93
28.88
28.57
25.80
21.10
17.90
7.37
f1 (ppm)

**FIG. 20**

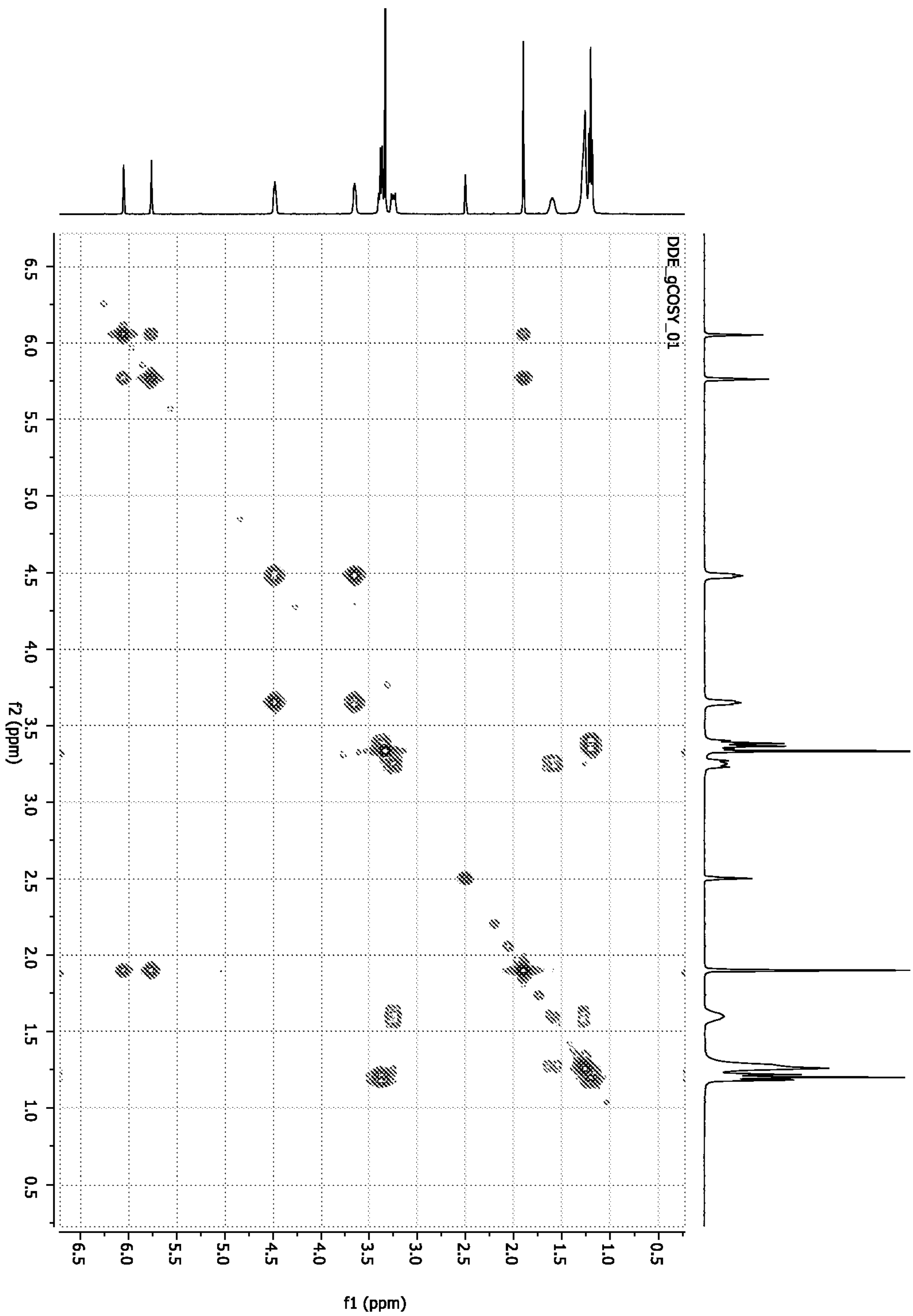


**FIG. 21**

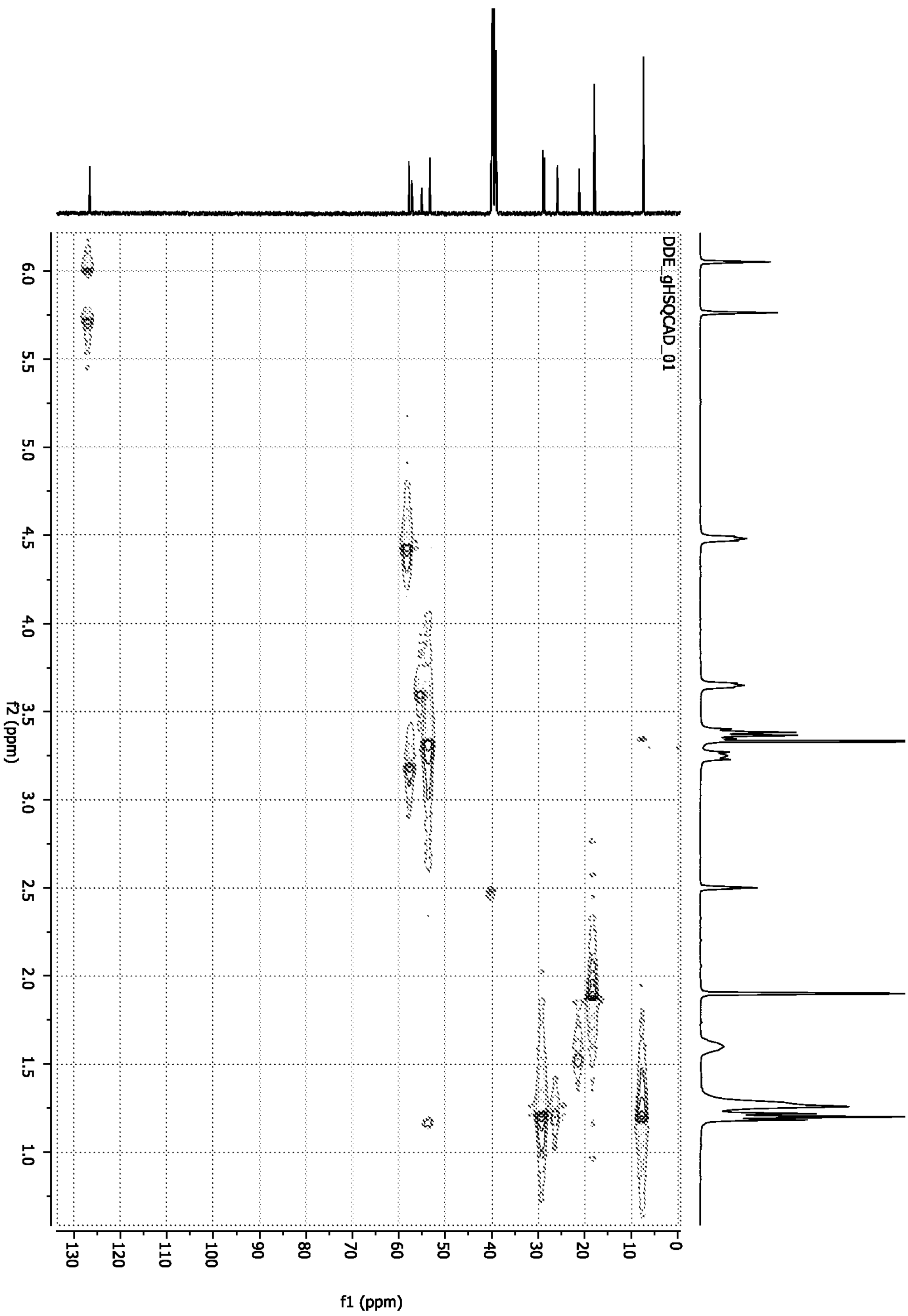


FIG. 22

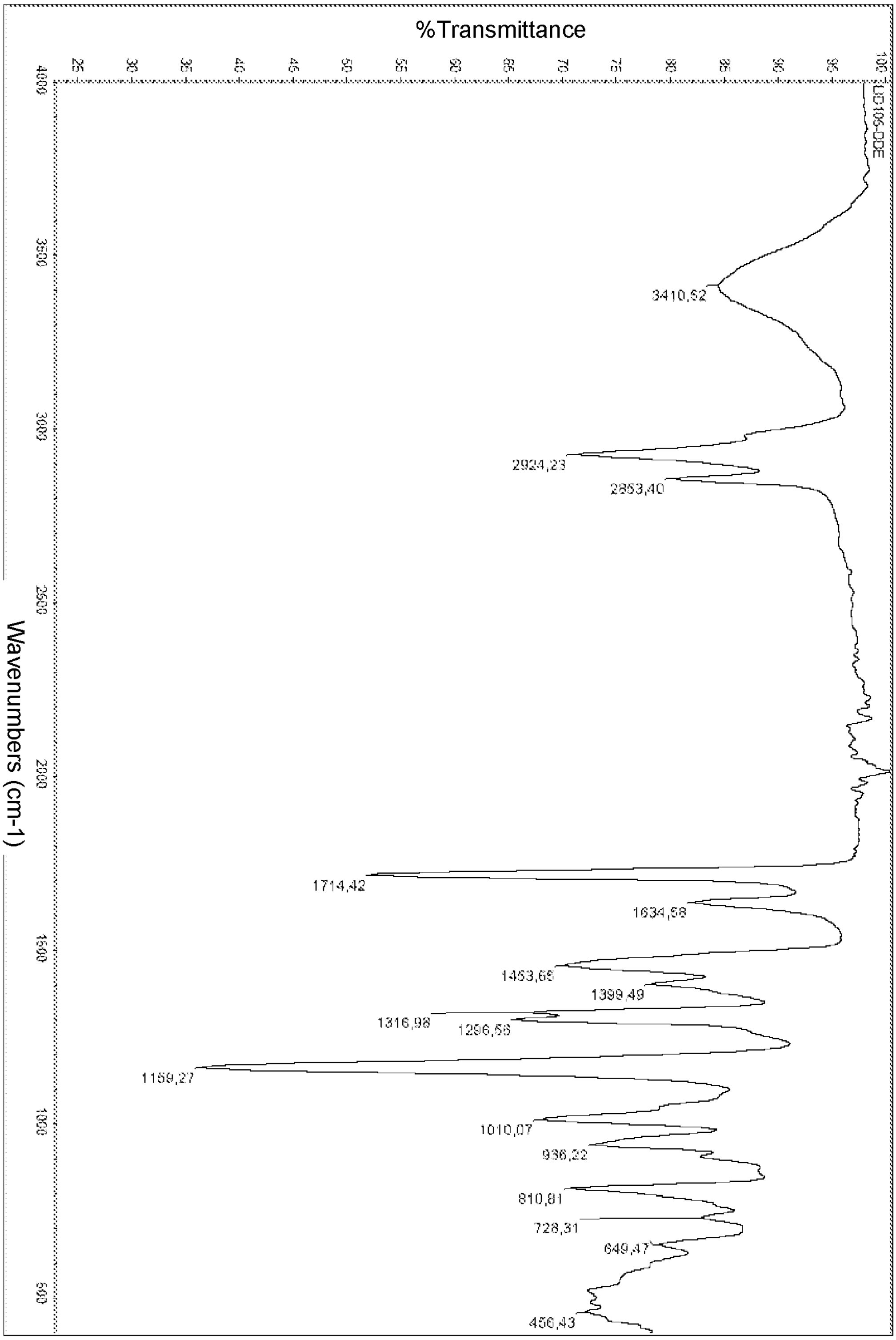
%Transmittance
Wavenumbers (cm-1)
LD105-DDE
3410,92
2924,23
2853,40
1714,42
1634,58
1453,66
1399,49
1316,98
1296,56
1159,27
1010,07
936,22
810,81
728,31
649,47
456,43

**FIG. 23**

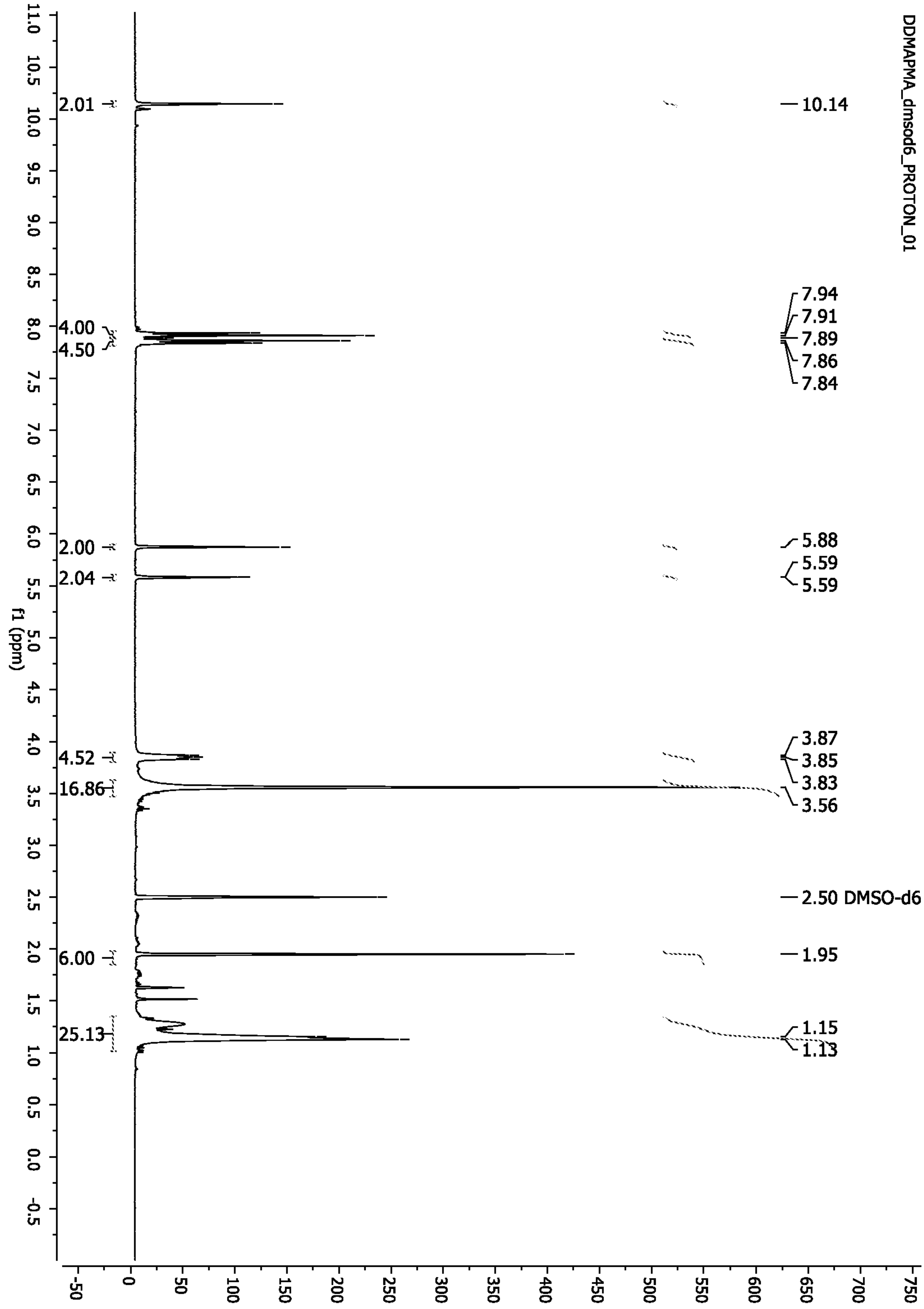
DDMAPMA_dmsod6_PROTON_01
10.14
7.94
7.91
7.89
7.86
7.84
5.88
5.59
5.59
3.87
3.85
3.83
3.56
2.50 DMSO-d6
1.95
1.15
1.13
2.01
4.00
4.50
2.00
2.04
4.52
16.86
6.00
25.13
f1 (ppm)

**FIG. 24**

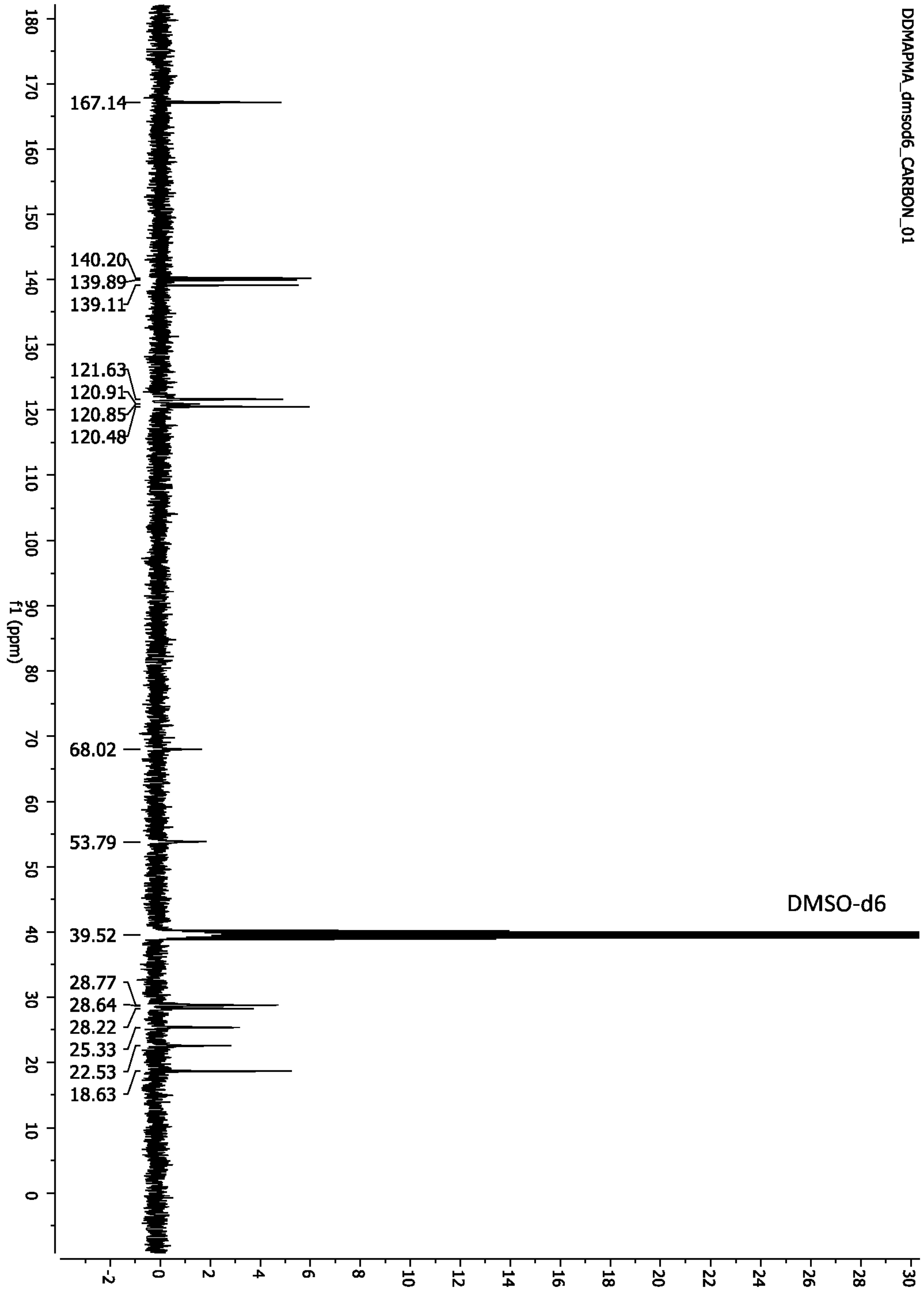
DDMAPMA_dmsod6_CARBON_01
167.14
140.20
139.89
139.11
121.63
120.91
120.85
120.48
68.02
53.79
DMSO-d6
39.52
28.77
28.64
28.22
25.33
22.53
18.63
f1 (ppm)

**FIG. 25**

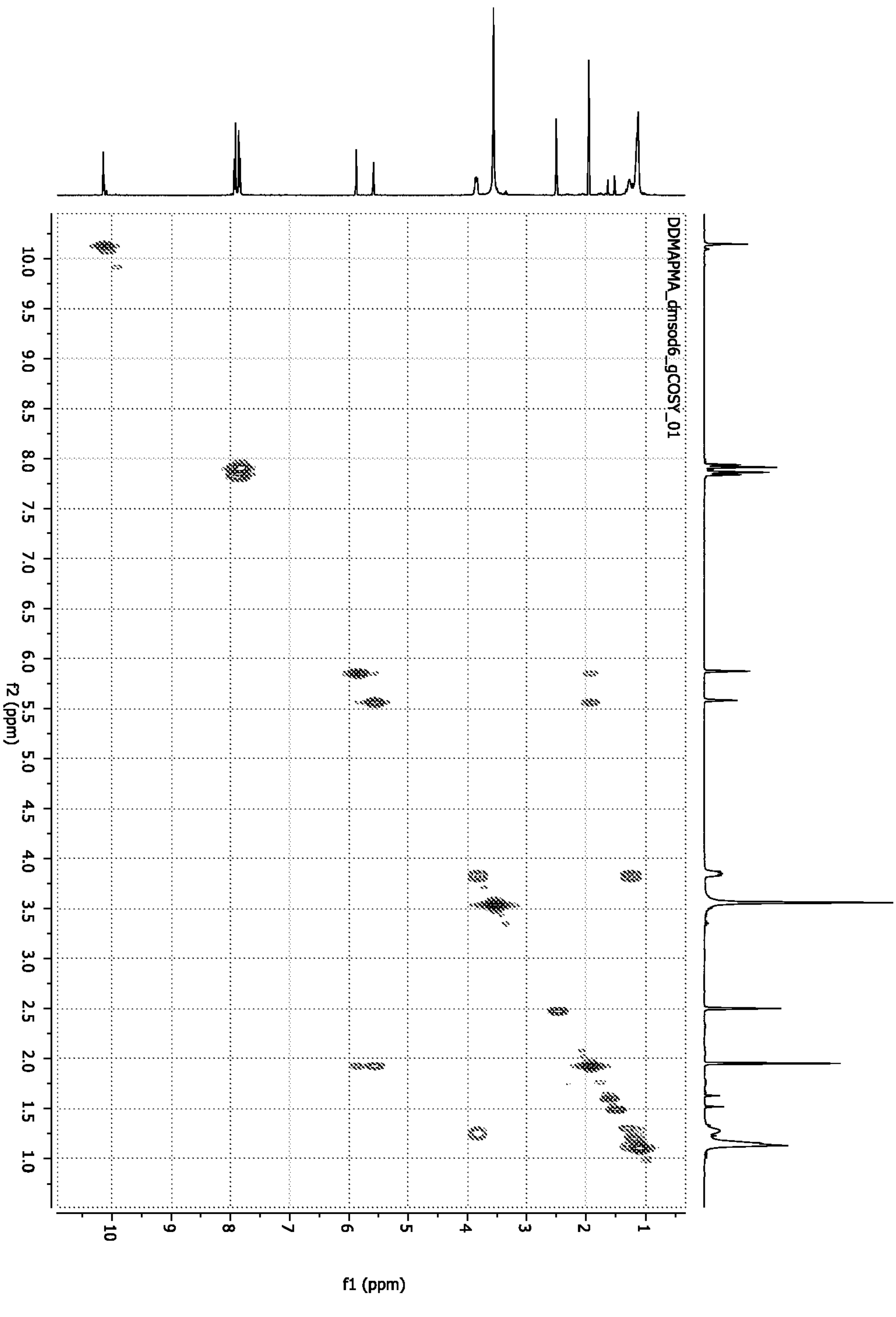


FIG. 26

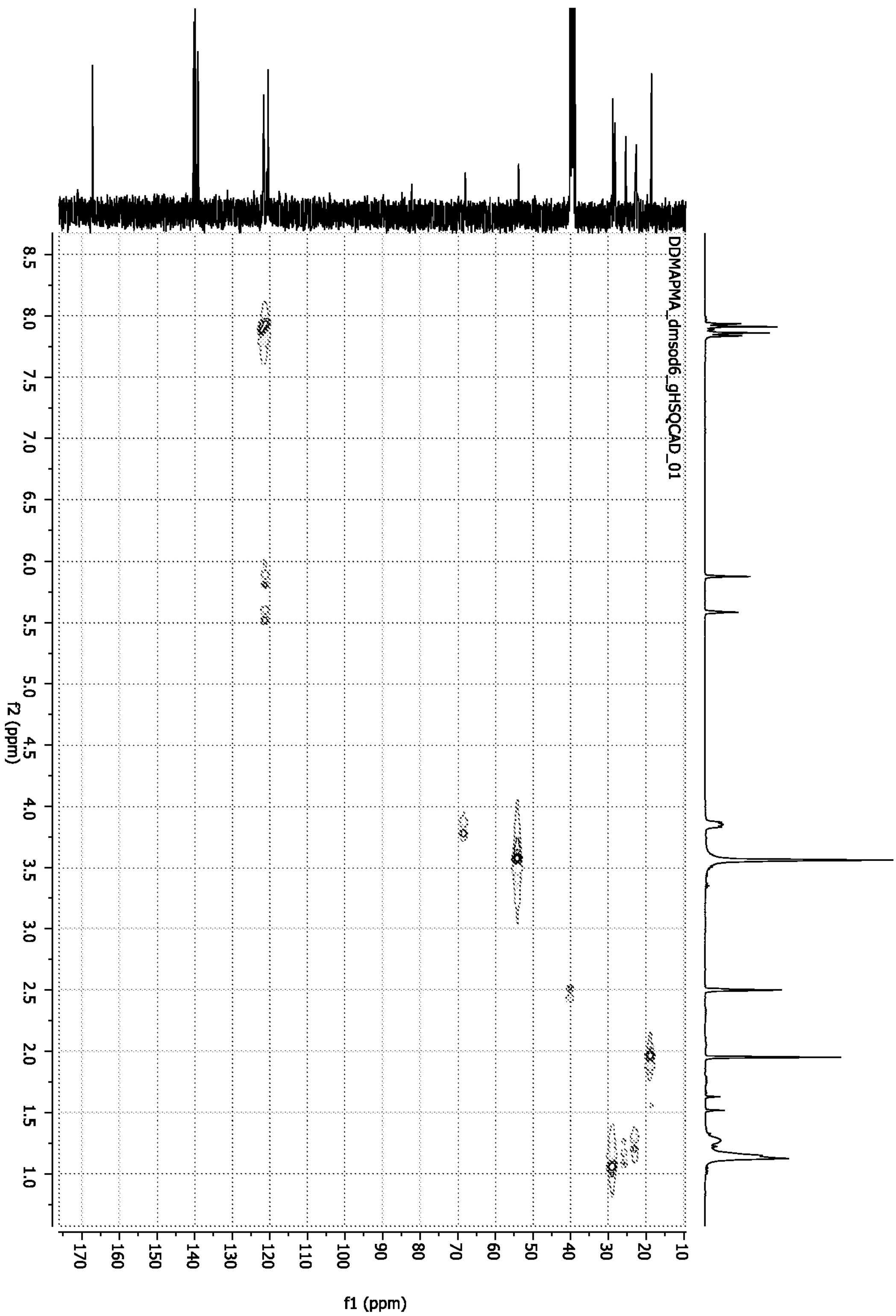


**FIG. 27**

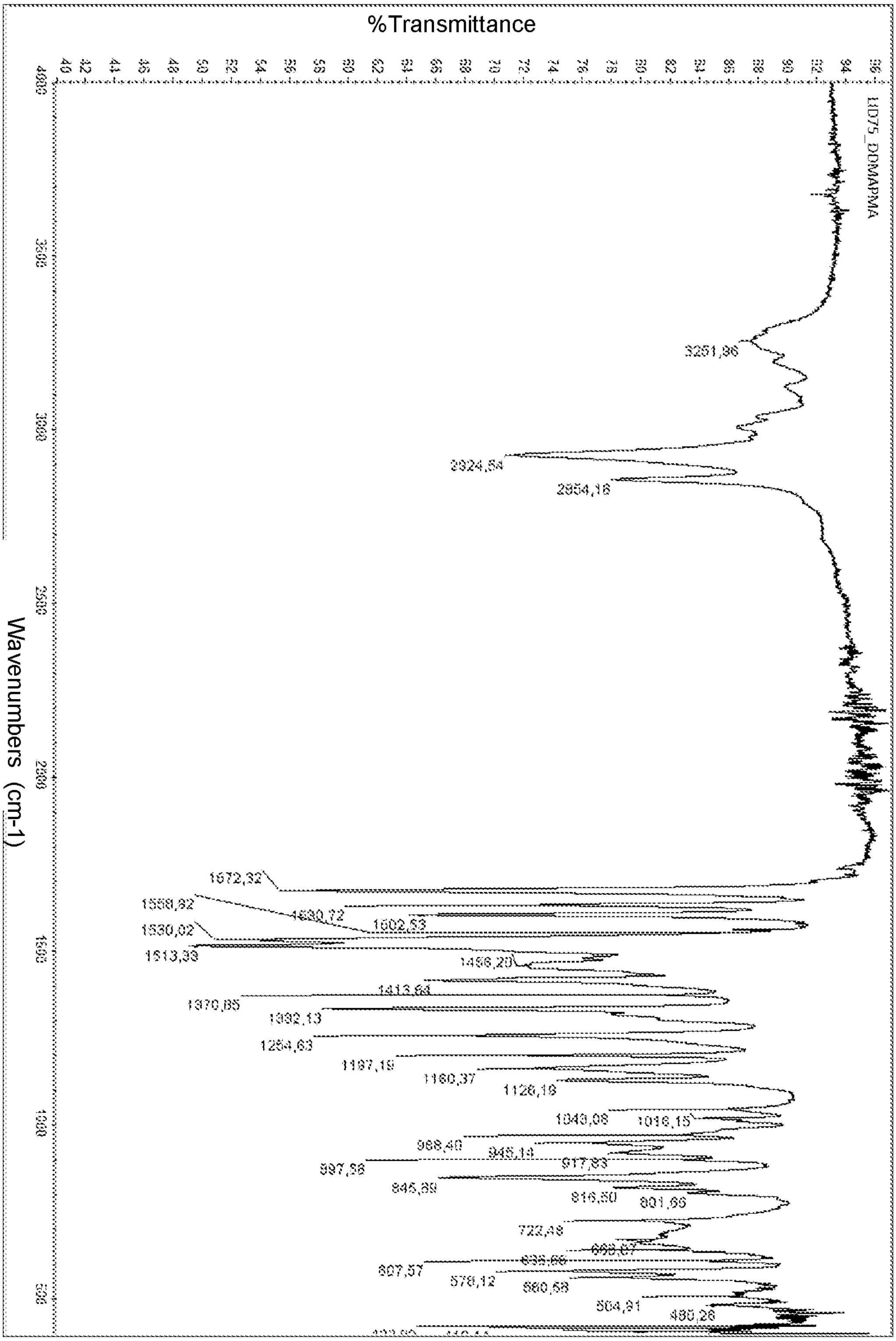
%Transmittance
Wavenumbers (cm-1)
LID75_DDMAPMA
3251,36
2924,54
2854,16
1672,32
1558,82
1630,72
1602,53
1530,02
1513,33
1456,20
1413,64
1370,65
1332,13
1254,63
1197,19
1160,37
1126,19
1043,08
1016,15
968,40
945,14
917,83
897,56
845,89
816,50
801,66
722,48
636,66
607,57
578,12
560,68
504,91
480,26

**FIG. 28**

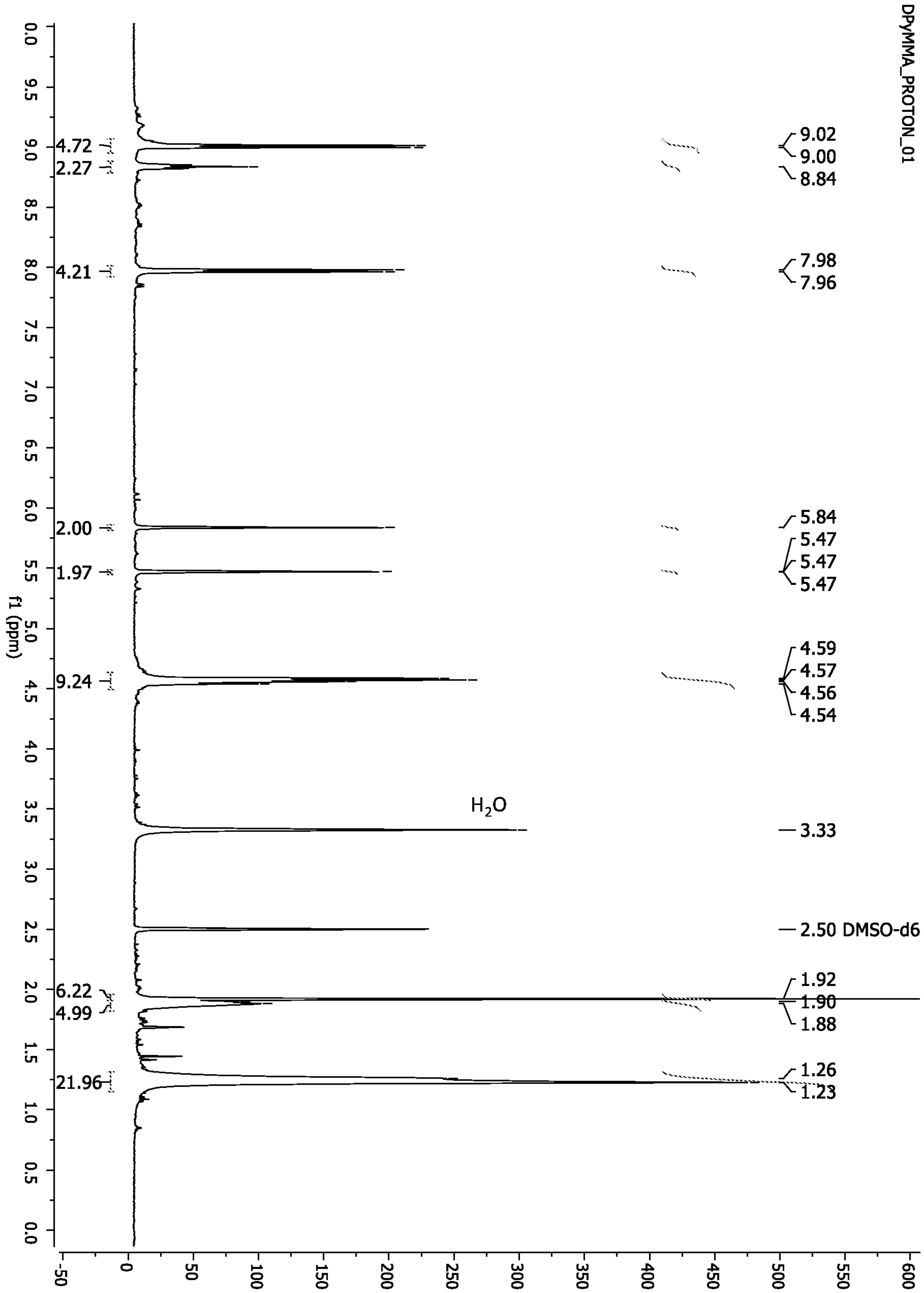
DPyMMA_PROTON_01
9.02
9.00
8.84
7.98
7.96
5.84
5.47
5.47
5.47
4.59
4.57
4.56
4.54
3.33
2.50 DMSO-d6
1.92
1.90
1.88
1.26
1.23
$H_2O$
4.72
2.27
4.21
2.00
1.97
9.24
6.22
4.99
21.96
f1 (ppm)

**FIG. 29**

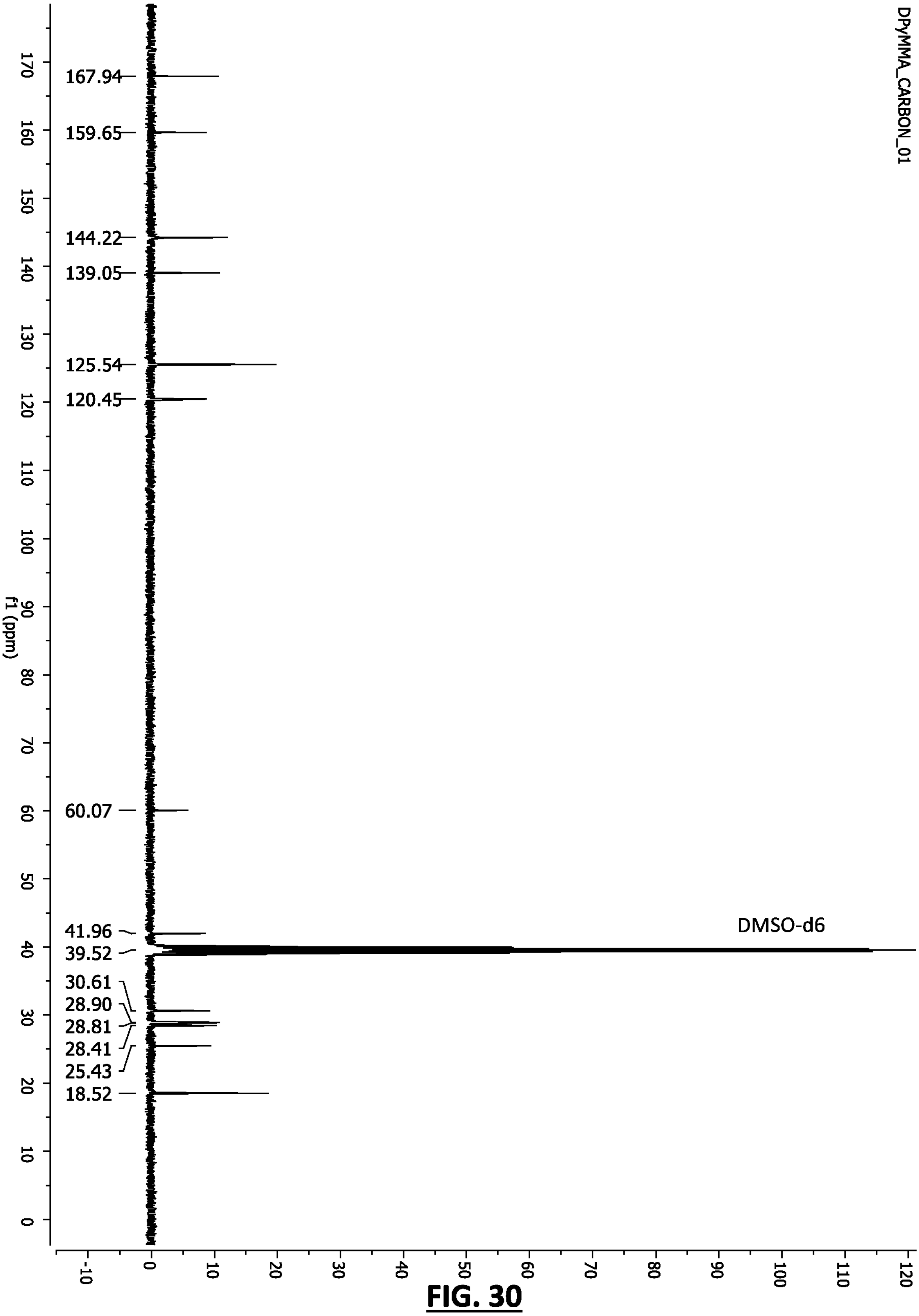
DPyMMA_CARBON_01
167.94
159.65
144.22
139.05
125.54
120.45
60.07
41.96
39.52
30.61
28.90
28.81
28.41
25.43
18.52
DMSO-d6
f1 (ppm)

**FIG. 30**

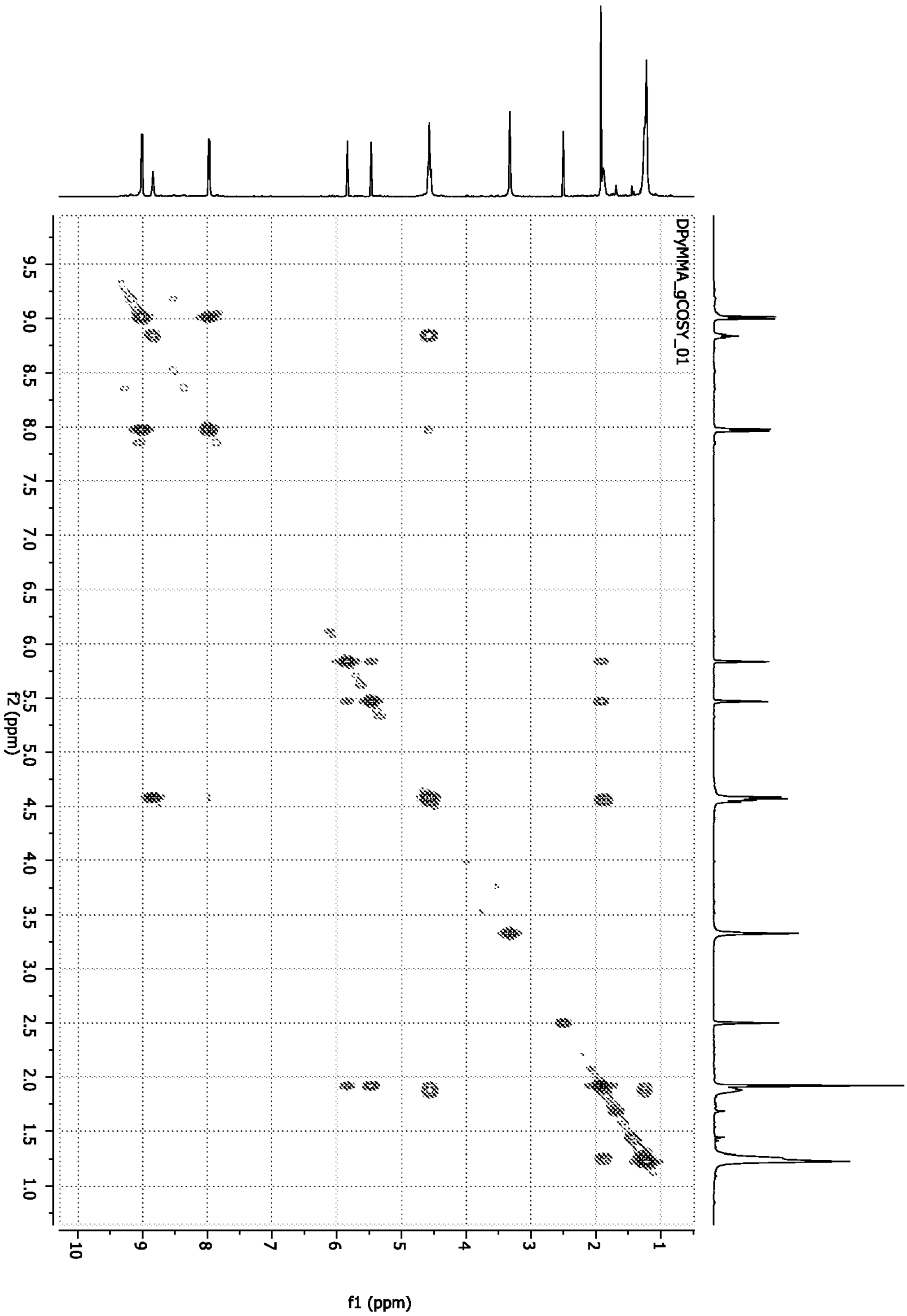


**FIG. 31**

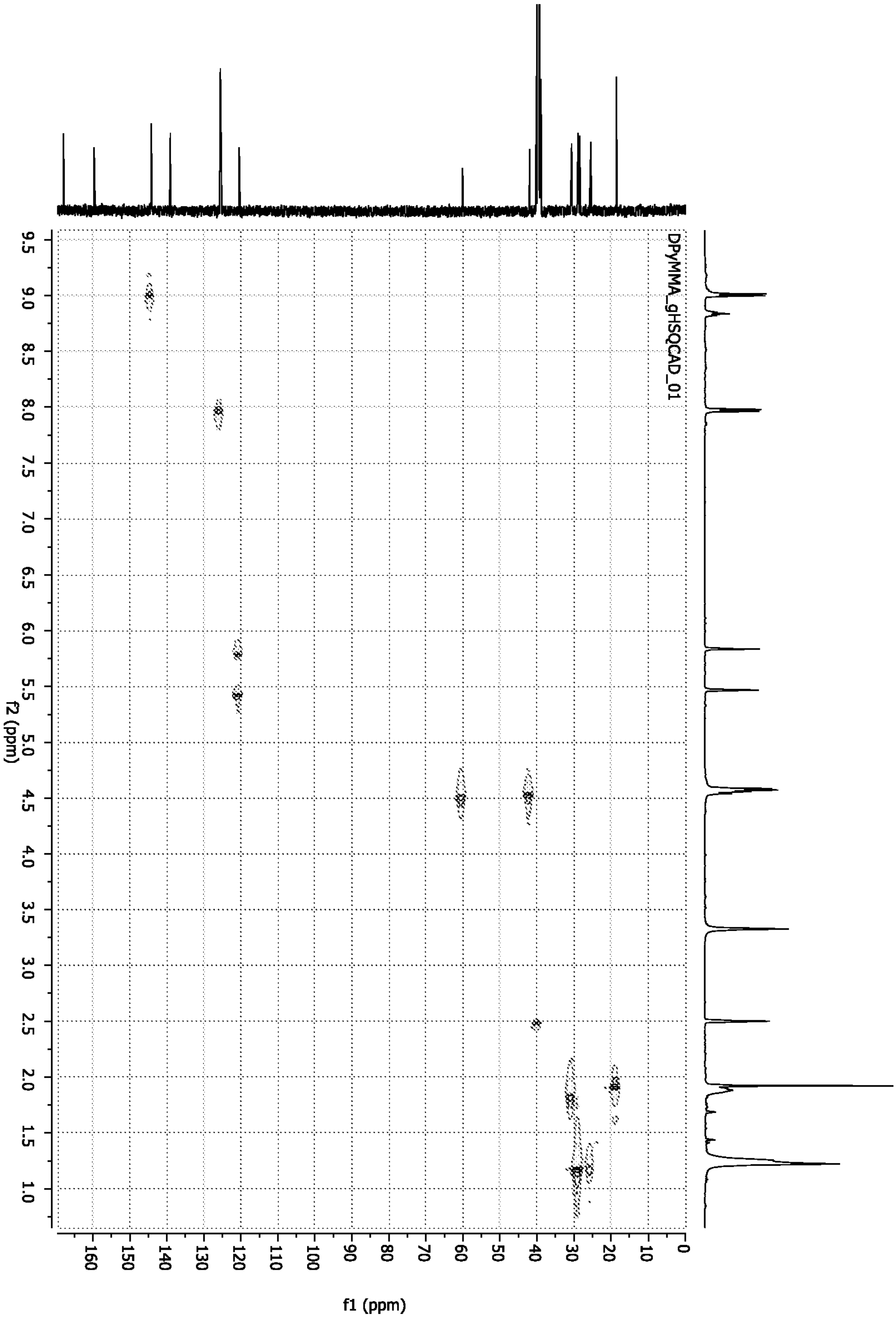


**FIG. 32**

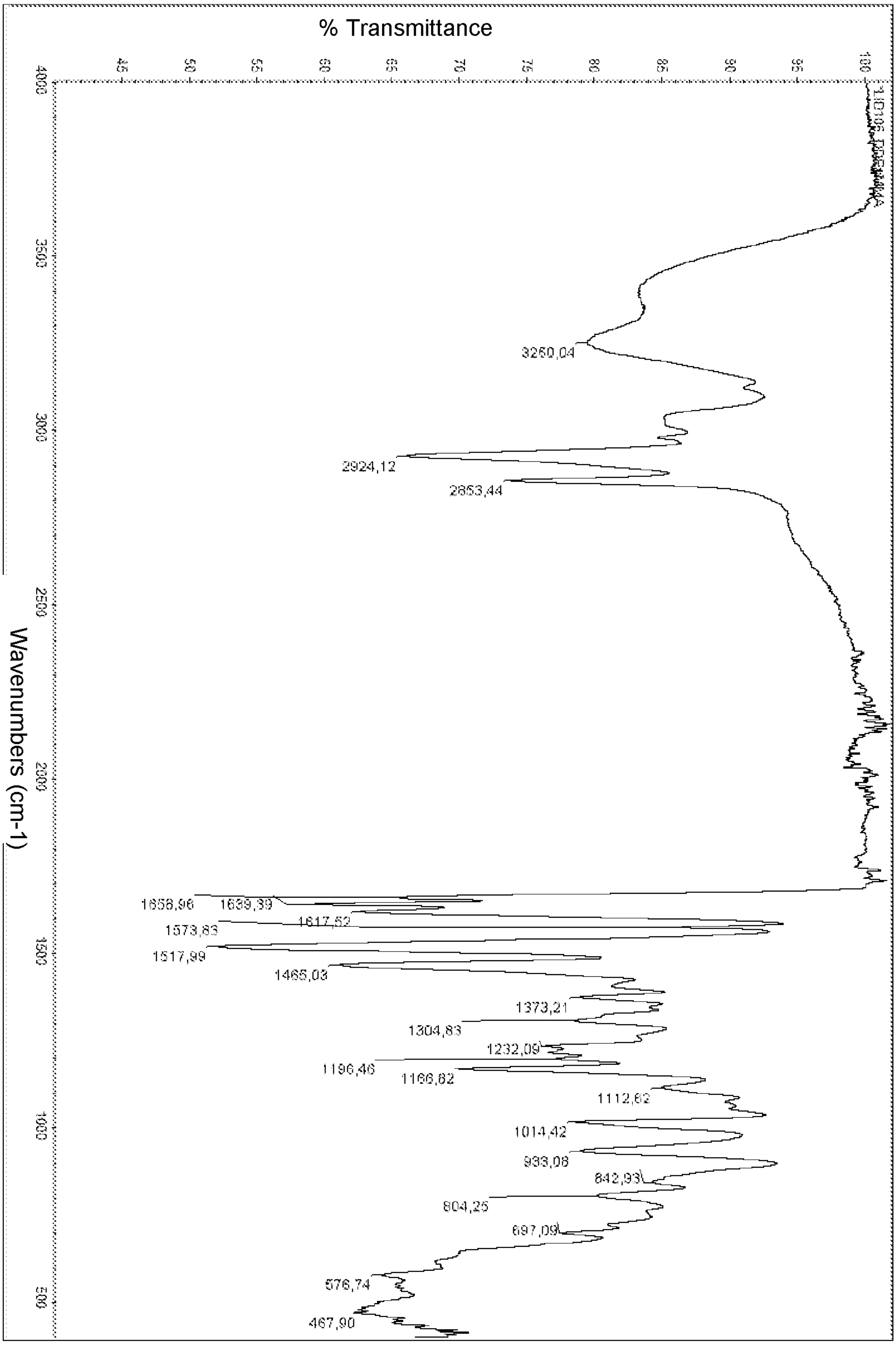
% Transmittance
Wavenumbers (cm-1)
3260,04
2924,12
2853,44
1658,96
1639,39
1617,52
1573,83
1517,99
1465,03
1373,21
1304,83
1232,09
1196,46
1166,82
1112,62
1014,42
933,08
842,93
804,25
697,09
576,74
467,90

**FIG. 33**

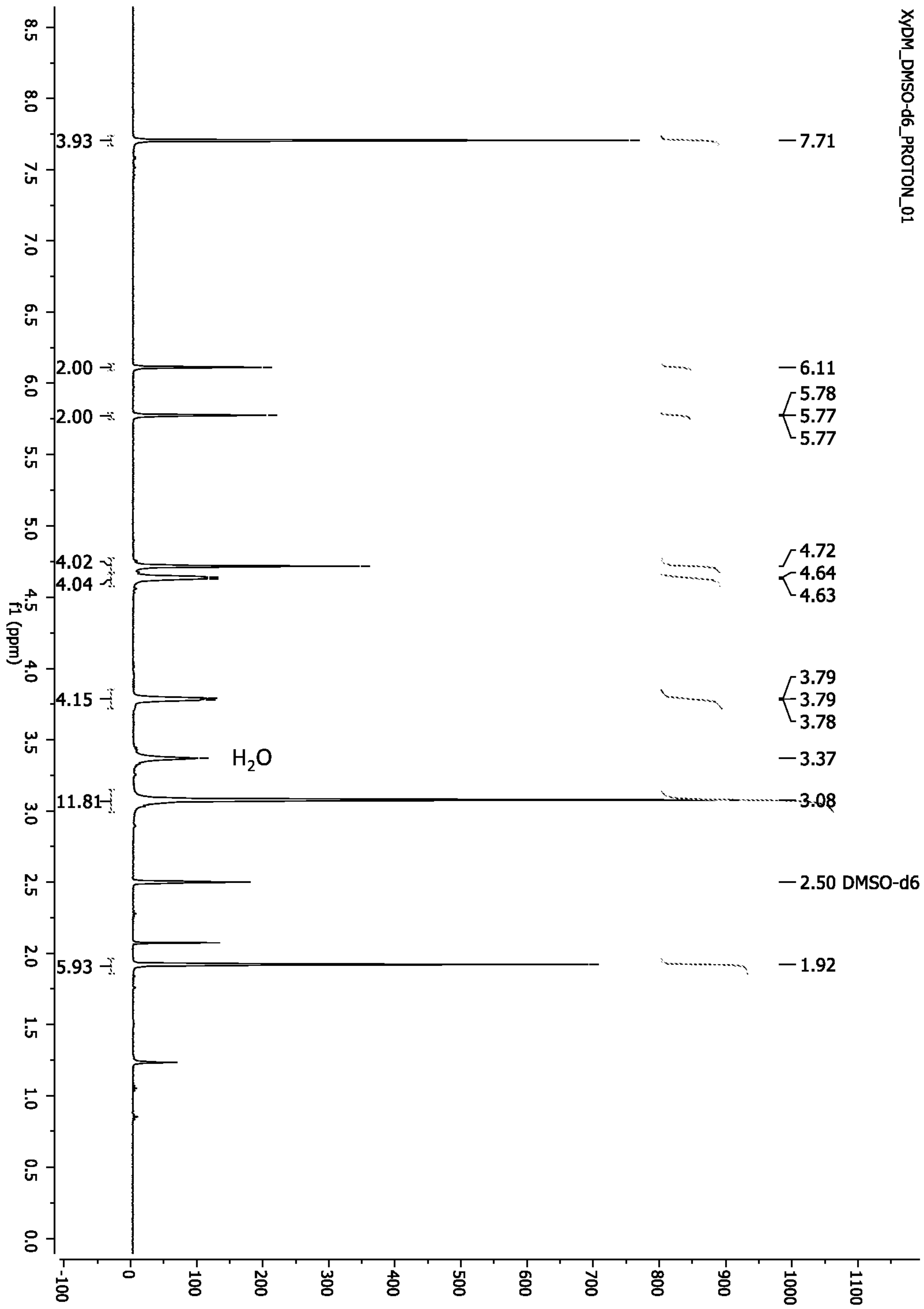
XyDM_DMSO-d6_PROTON_01
f1 (ppm)
H2O
7.71
6.11
5.78
5.77
5.77
4.72
4.64
4.63
3.79
3.79
3.78
3.37
3.08
2.50 DMSO-d6
1.92
3.93
2.00
2.00
4.02
4.04
4.15
11.81
5.93

**FIG. 34**

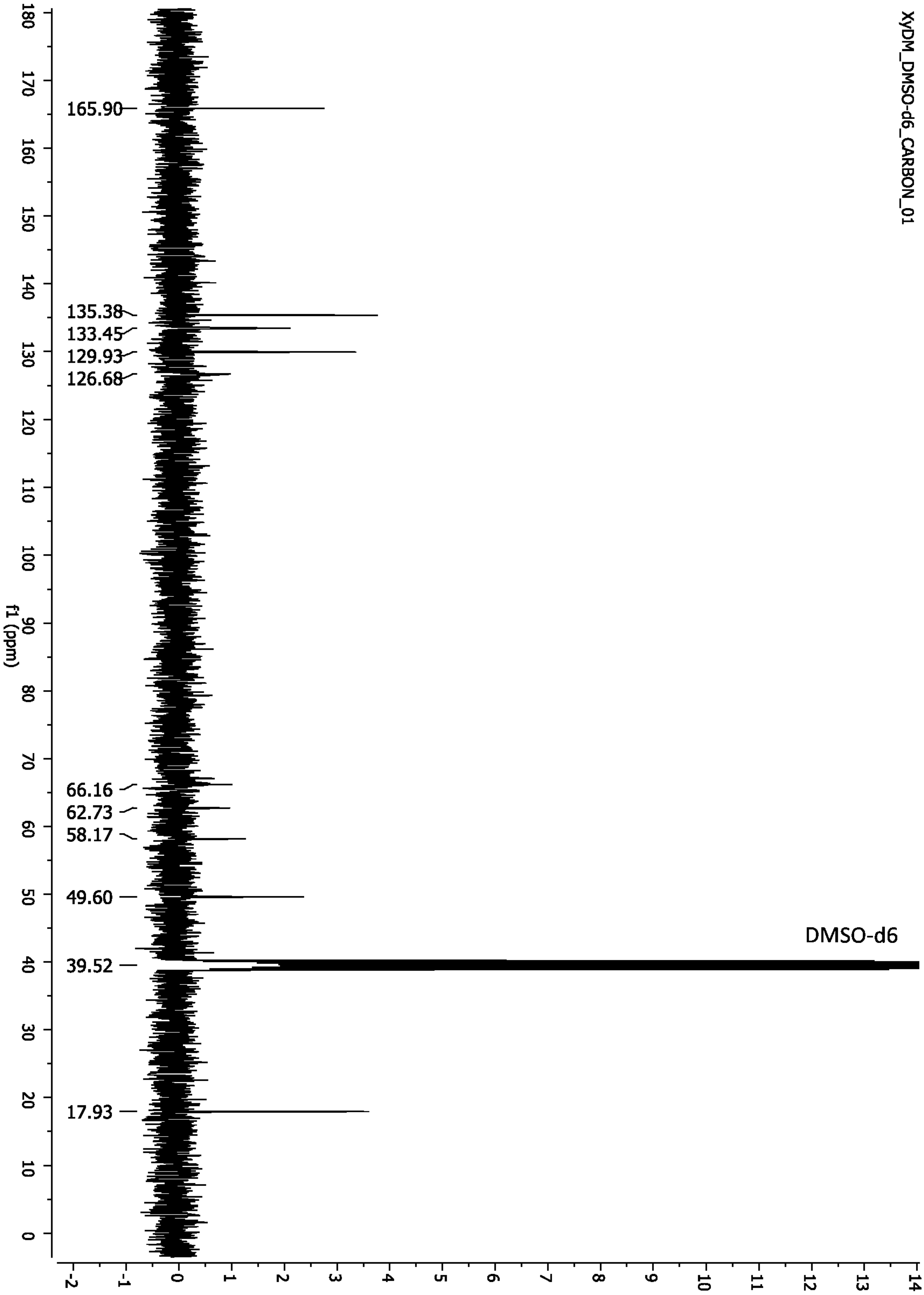
XyDM_DMSO-d6_CARBON_01
165.90
135.38
133.45
129.93
126.68
66.16
62.73
58.17
49.60
39.52
17.93
DMSO-d6
f1 (ppm)


**FIG. 35**

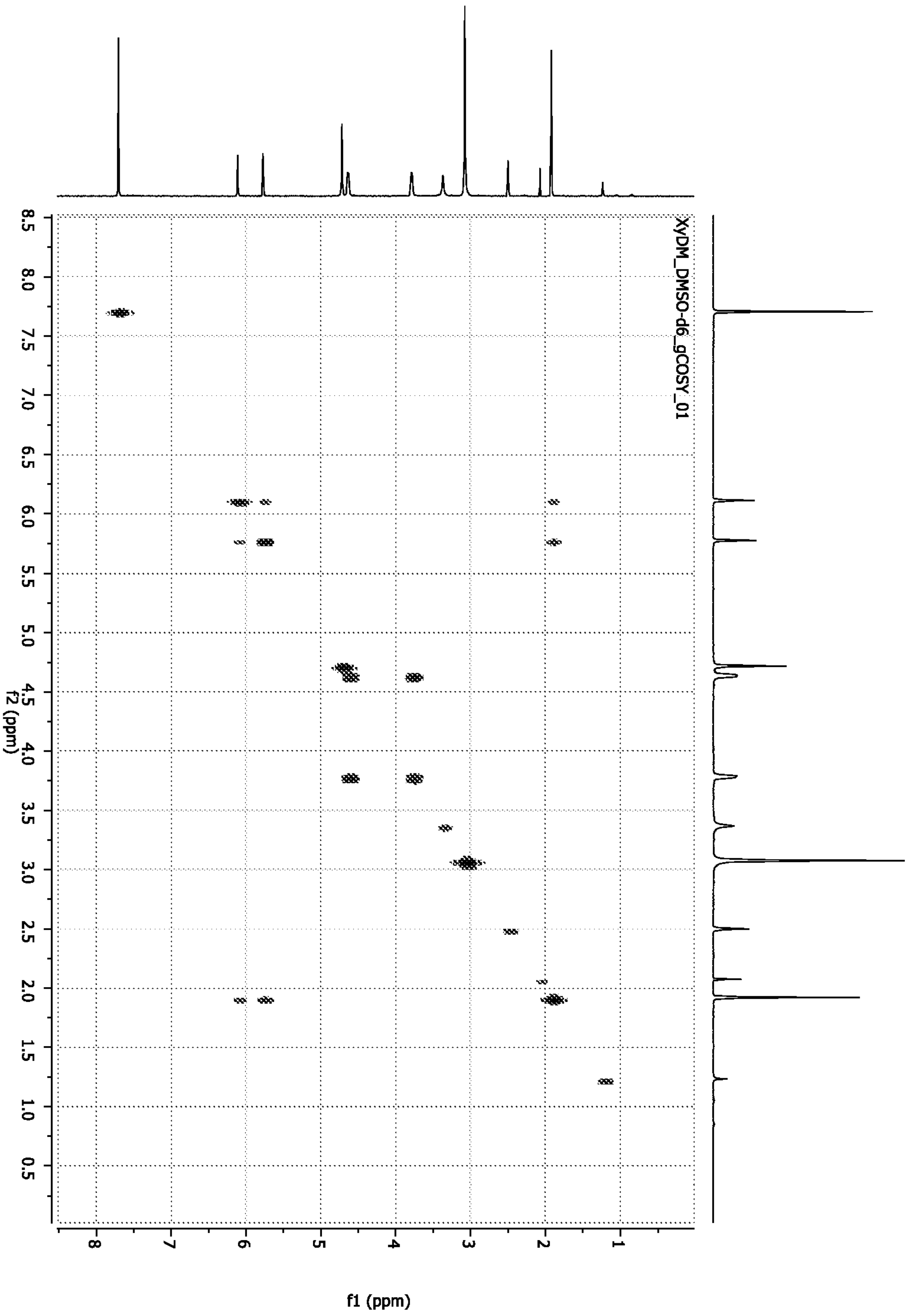


**FIG. 36**

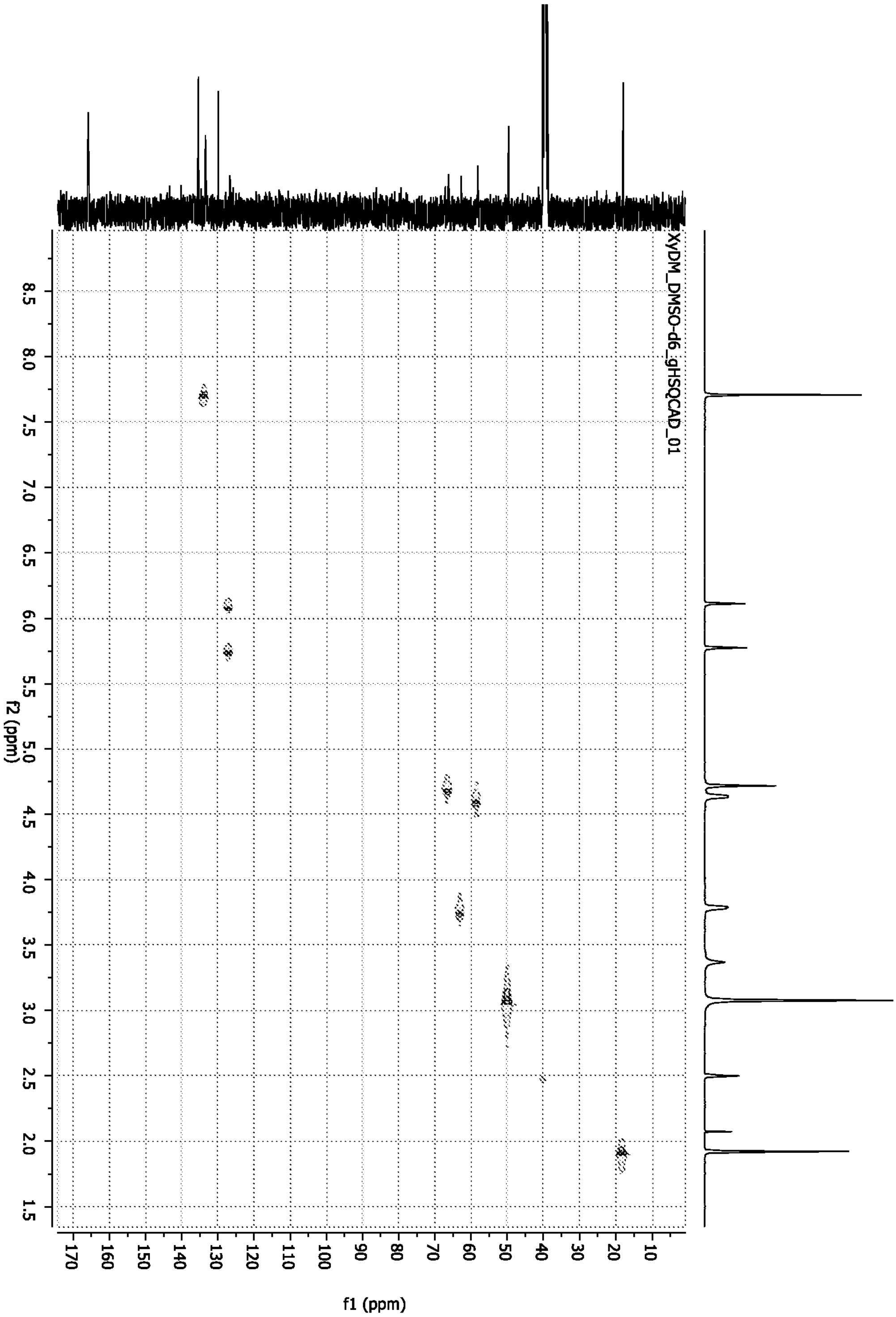
XyDM_DMSO-d6_gHSQCAD_01
f2 (ppm)
f1 (ppm)
8.5
8.0
7.5
7.0
6.5
6.0
5.5
5.0
4.5
4.0
3.5
3.0
2.5
2.0
1.5
170
160
150
140
130
120
110
100
90
80
70
60
50
40
30
20
10

**FIG. 37**

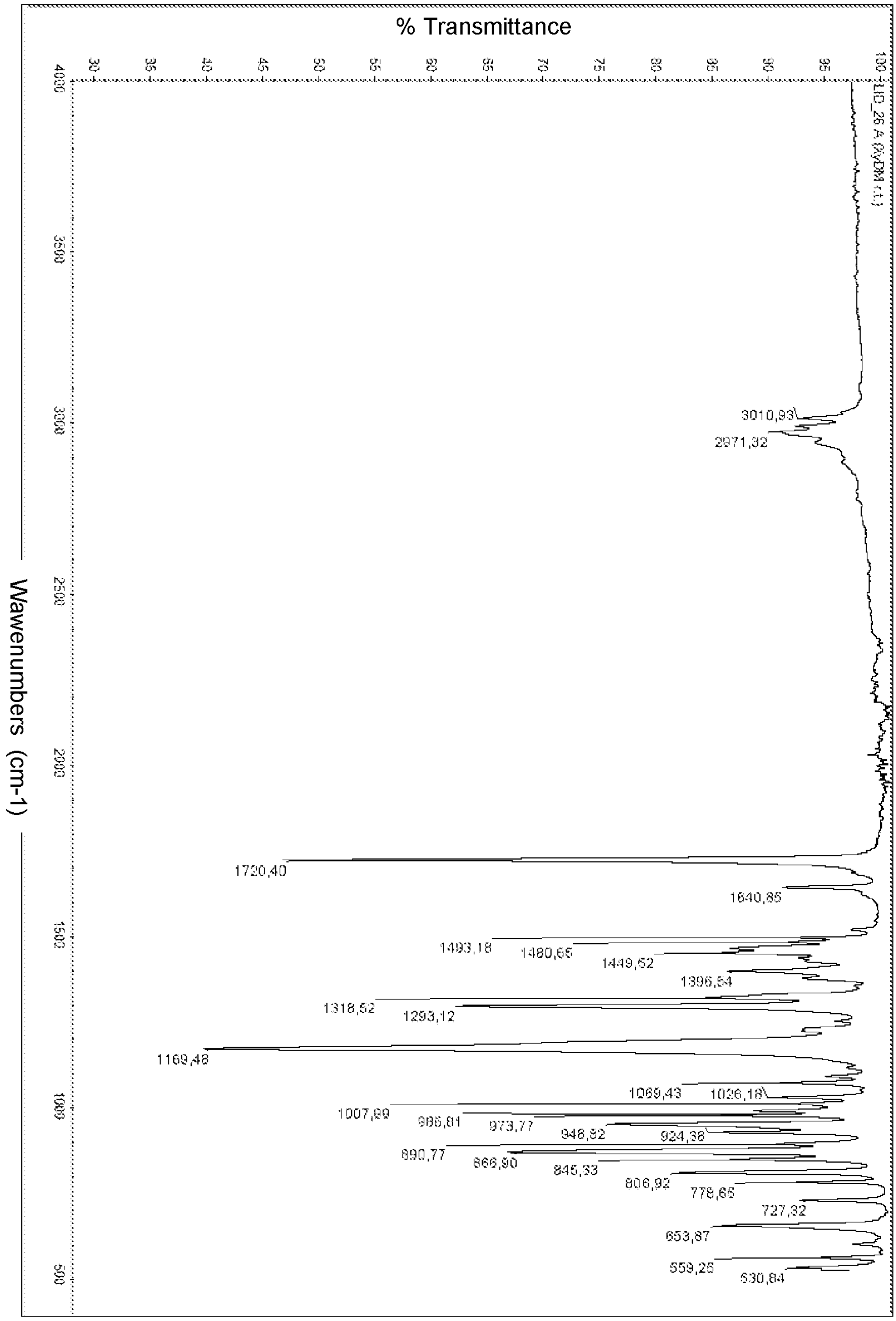

% Transmittance
Wavenumbers (cm-1)
3010,93
2971,32
1720,40
1640,85
1493,18
1480,65
1449,52
1396,54
1318,52
1293,12
1169,48
1069,43
1026,18
1007,99
986,81
973,77
948,82
924,38
890,77
866,90
845,33
806,92
778,65
727,32
653,87
559,25
530,84


FIG. 38

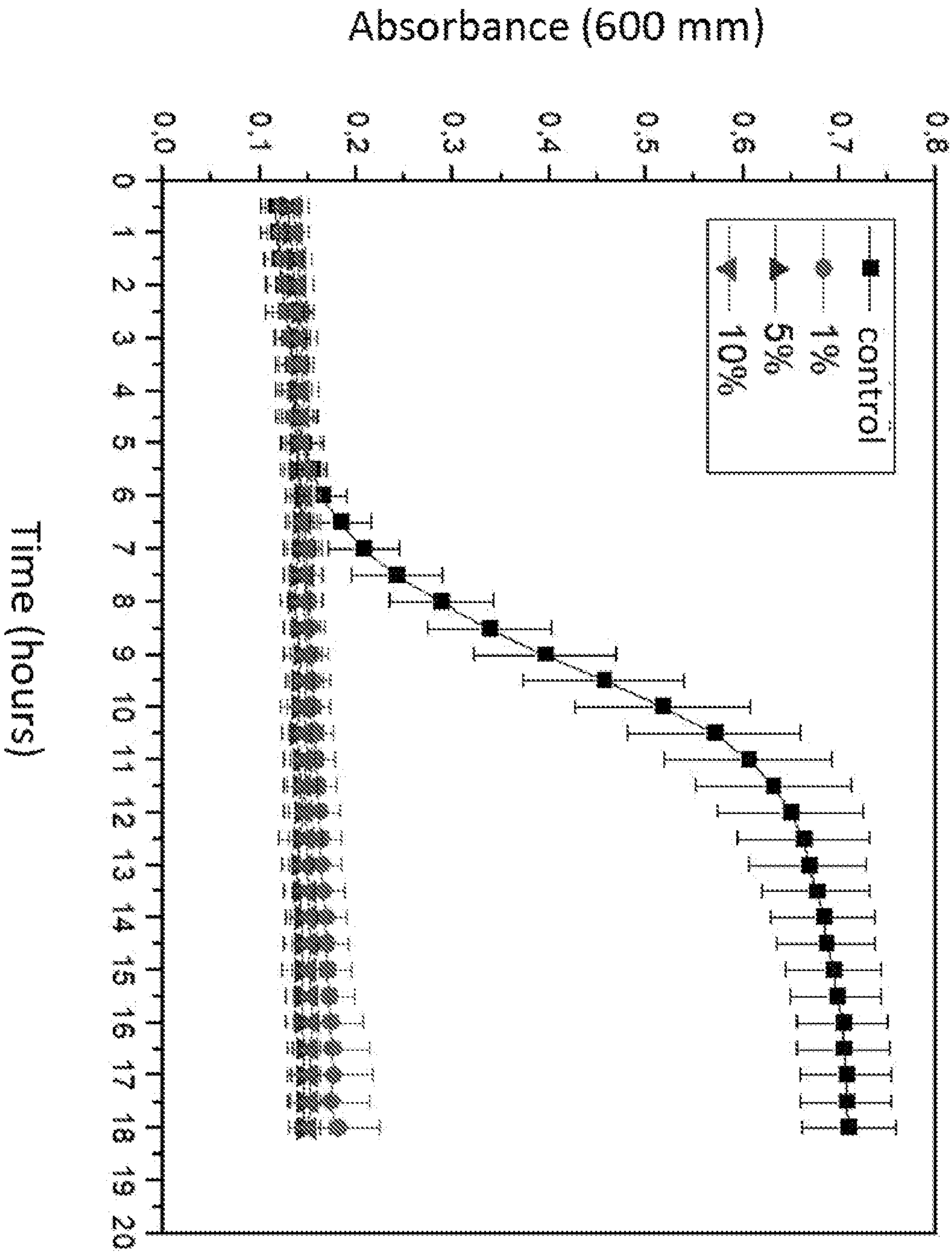
Absorbance (600 mm)
0,0
0,1
0,2
0,3
0,4
0,5
0,6
0,7
0,8
Time (hours)
0
1
2
3
4
5
6
7
8
9
10
11
12
13
14
15
16
17
18
19
20
control
1%
5%
10%

FIG. 39

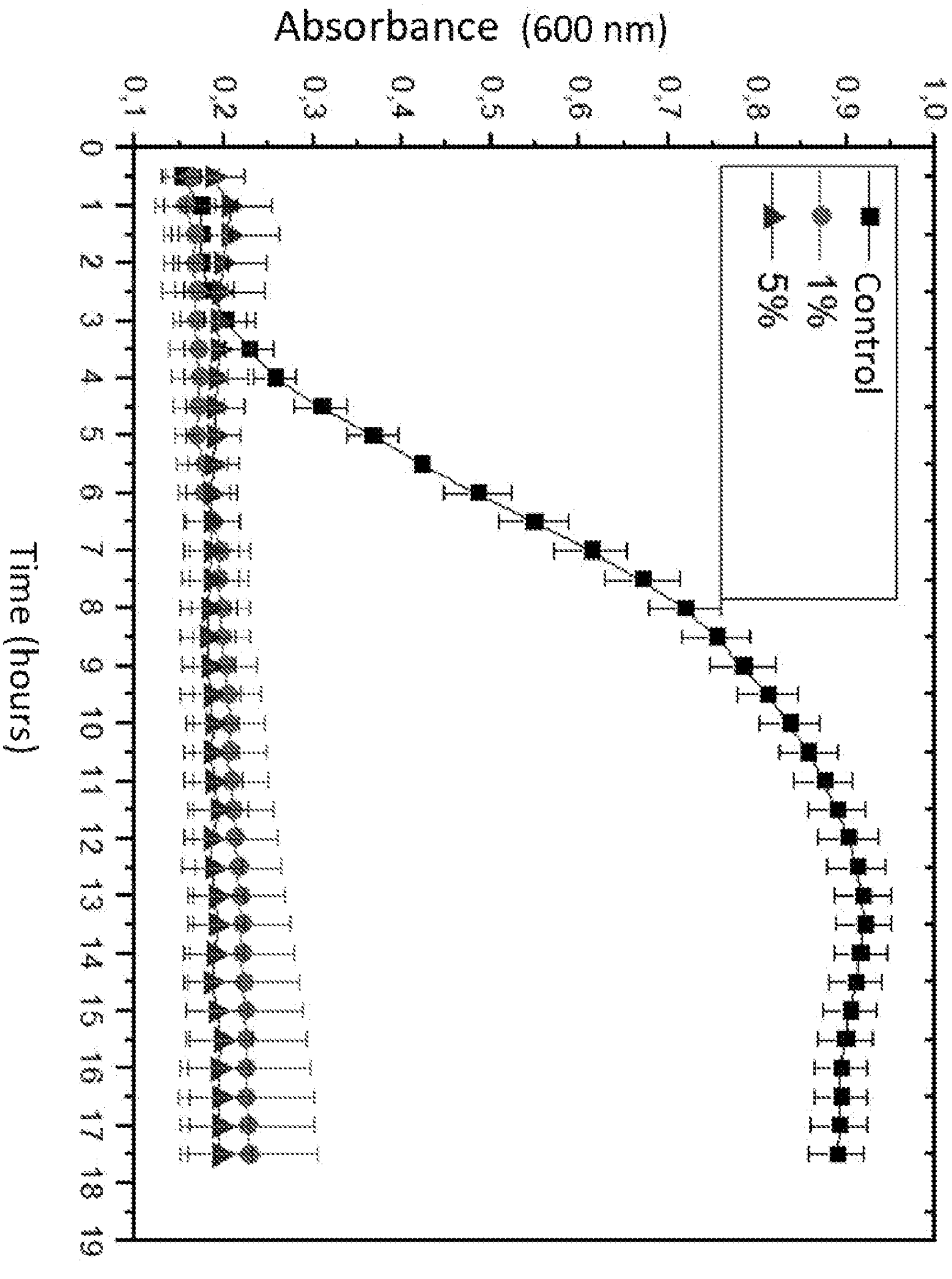
Absorbance (600 nm)
0,1
0,2
0,3
0,4
0,5
0,6
0,7
0,8
0,9
1,0
Time (hours)
0 1 2 3 4 5 6 7 8 9 10 11 12 13 14 15 16 17 18 19
Control
1%
5%

FIG. 40

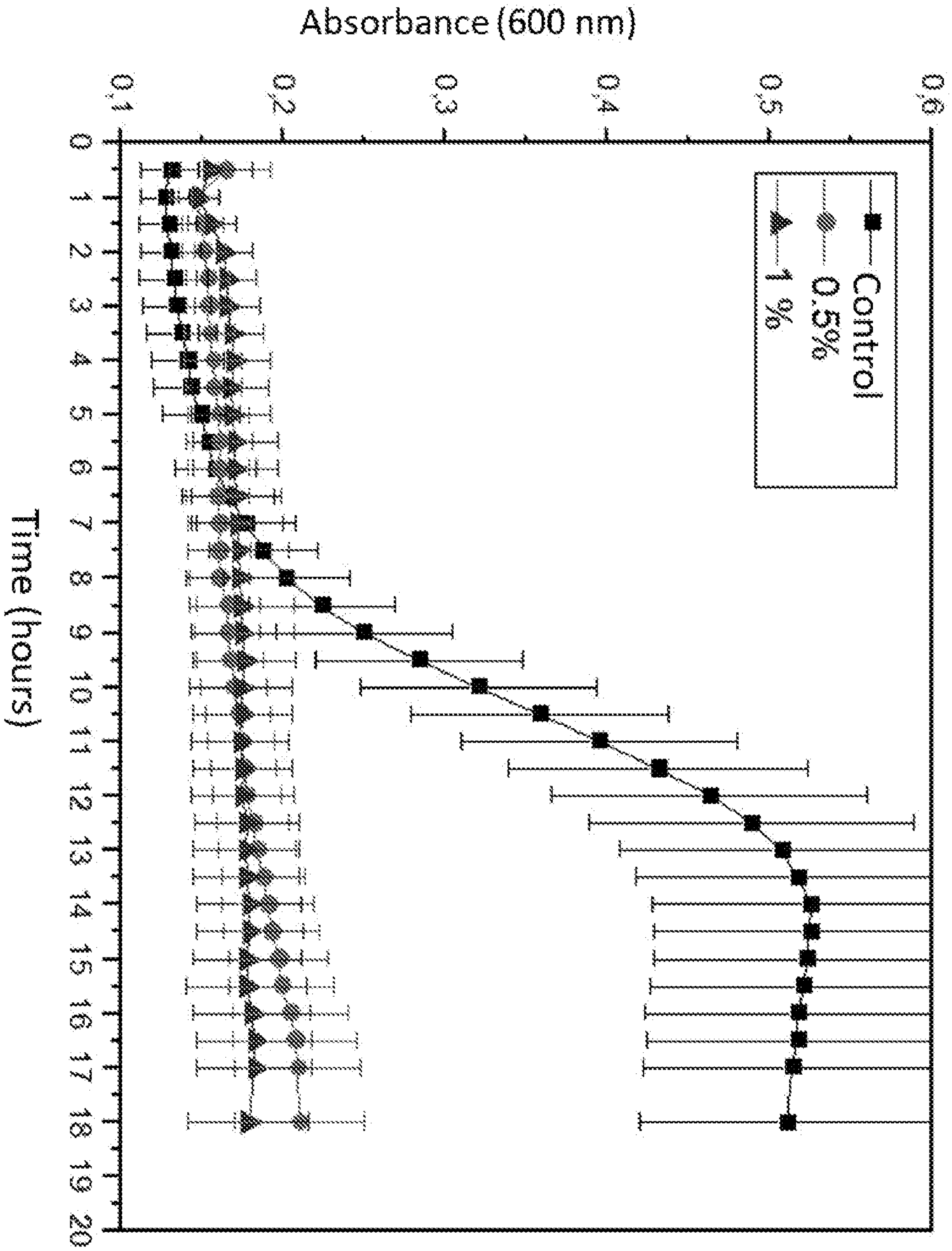
Absorbance (600 nm)
0,1
0,2
0,3
0,4
0,5
0,6
Control
0.5%
1 %
Time (hours)
0
1
2
3
4
5
6
7
8
9
10
11
12
13
14
15
16
17
18
19
20

FIG. 41

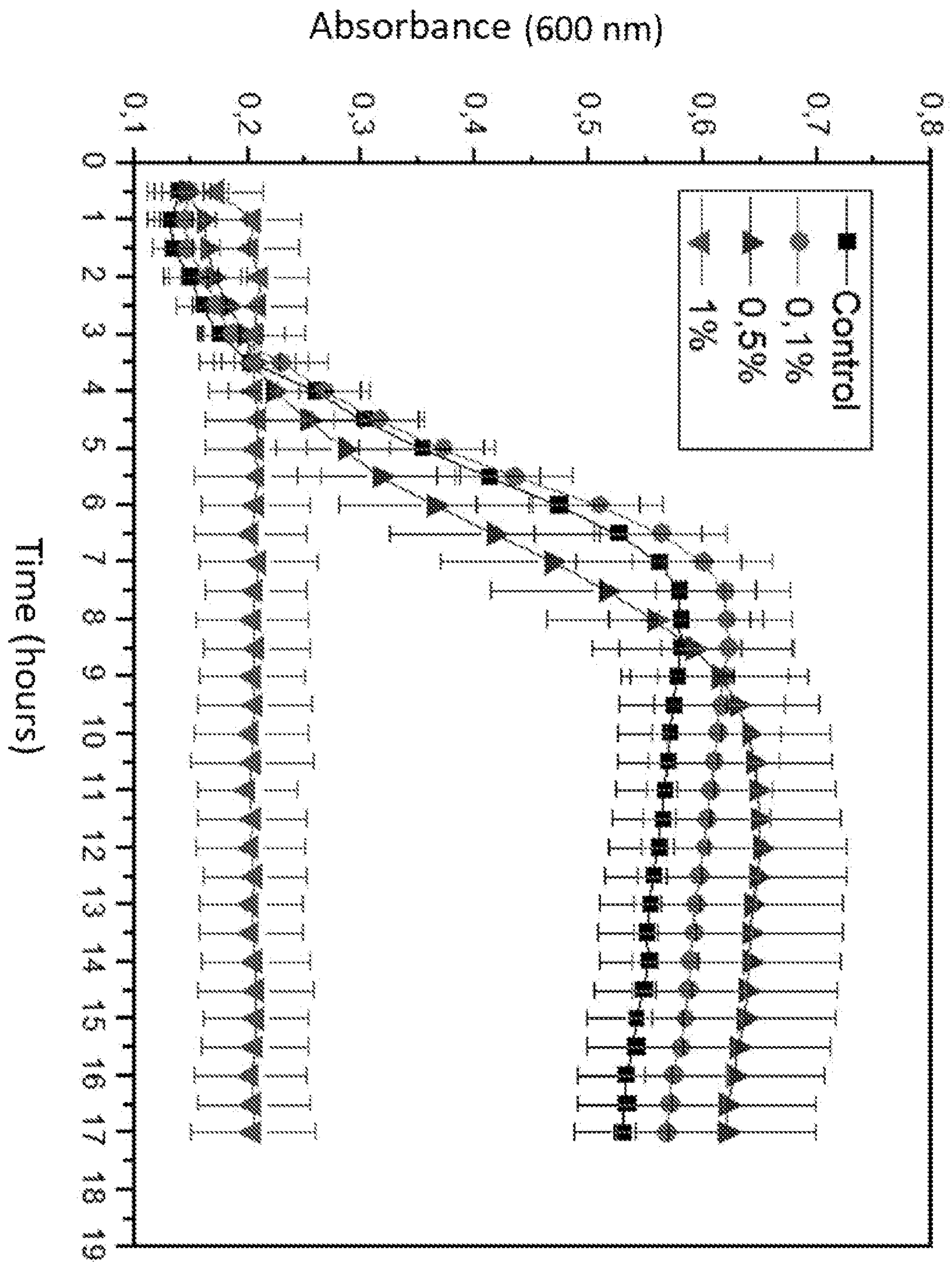


FIG. 42

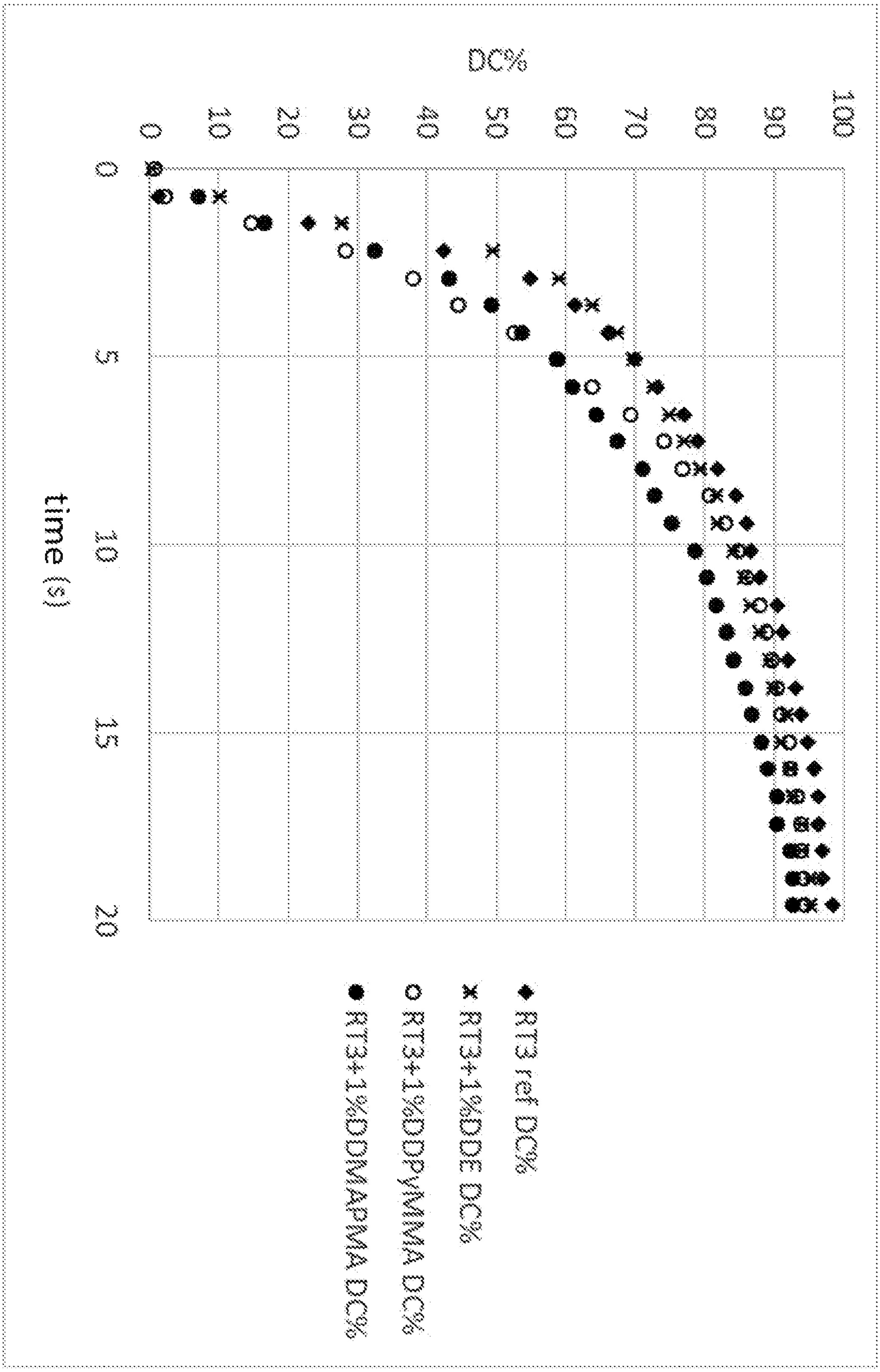
DC%
0
10
20
30
40
50
60
70
80
90
100
0
5
10
15
20
time (s)
RT3 ref DC%
RT3+1%DDE DC%
RT3+1%DDPyMMA DC%
RT3+1%DDMAPMA DC%

FIG. 43

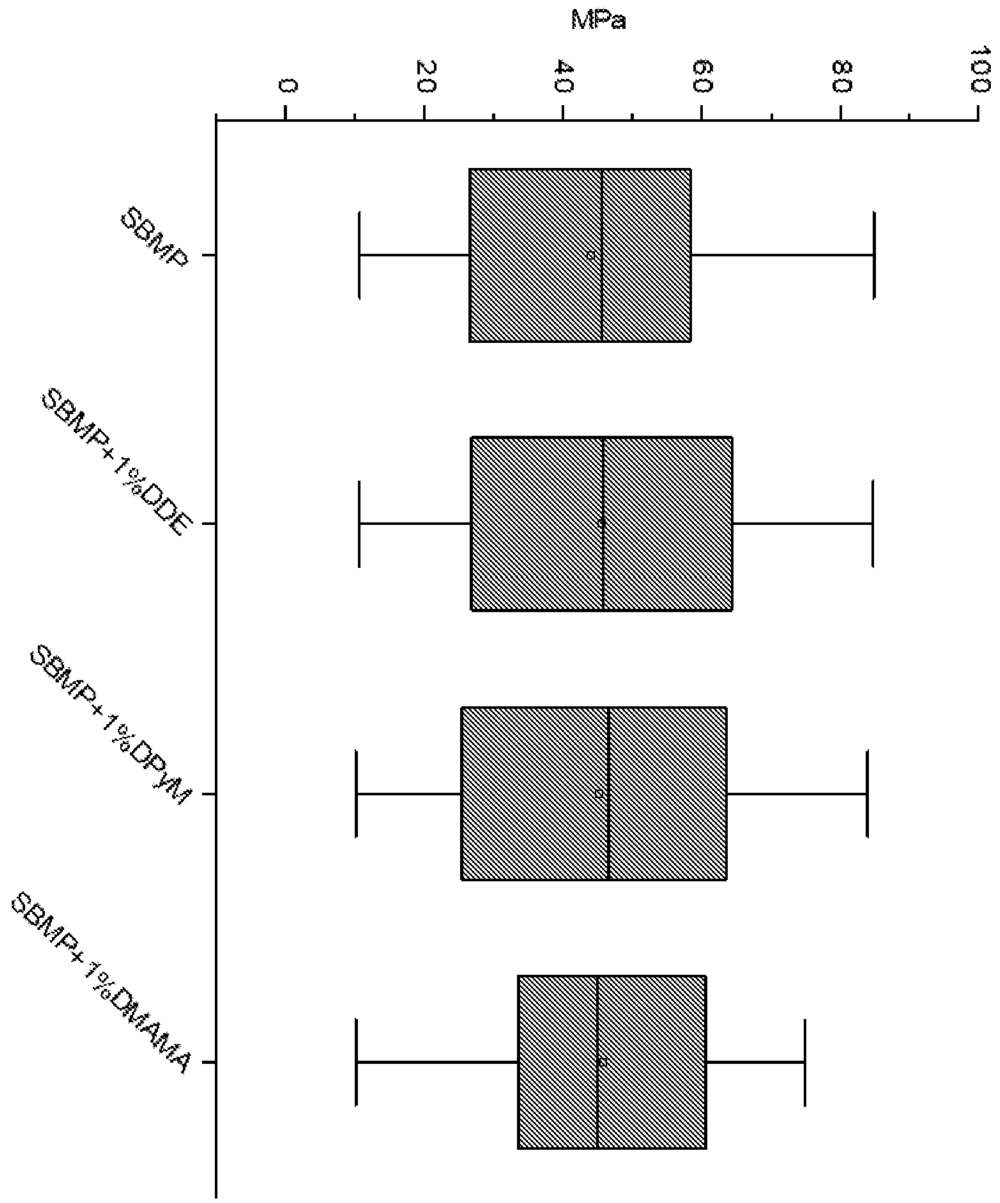


FIG. 44

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0980682 A1
- JP H08157318 A
- US 6339113 B1

### Non-patent literature cited in the description

- **BROWN DF** ; **KOTHARI D**. Comparison of antibiotic discs from different sources. *J. Clin. Pathol.*, 1975, vol. 28 (10), 779-83
- **S. ARMSTRONG** ; **L. BRESCHI** ; **M. OZCAN** ; **F. PFEFFERKORN** ; **M. FERRARI** ; **B. VAN MEERBEEK**. Academy of Dental Materials guidance on in vitro testing of dental composite bonding effectiveness to dentin/enamel using micro-tensile bond strength (μTBS) approach. *Dent. Mater.*, 2017, vol. 33 (2), 133-143
- *CHEMICAL ABSTRACTS*, 1565-94-2
- *CHEMICAL ABSTRACTS*, 868-77-9
- *CHEMICAL ABSTRACTS*, 10373-78-1
- *CHEMICAL ABSTRACTS*, 10287-53-3
- *CHEMICAL ABSTRACTS*, 32435-46-4
- *CHEMICAL ABSTRACTS*, 109-16-0
- *CHEMICAL ABSTRACTS*, 79-41-4
- *CHEMICAL ABSTRACTS*, 75980-60-8
- **S. ARMSTRONG** ; **L. BRESCHI** ; **M . OZCAN** ; **F. PFEFFERKORN** ; **M. FERRARI** ; **B. VAN MEERBEEK**. Academy of Dental Materials guidance on in vitro testing of dental composite bonding effectiveness to dentin/enamel using micro-tensile bond strength (μTBS) approach. *Dent. Mater.*, 2017, vol. 33 (2), 133-143